(19) Europäisches Patentamt
European Patent Office
Office européen des brevets

(11) Veröffentlichungsnummer: **0 228 045 B1**

# (12) EUROPÄISCHE PATENTSCHRIFT

(45) Veröffentlichungstag der Patentschrift: **03.04.91**

(51) Int. Cl.5: **C07C 323/40**, C07D 215/54, C07D 311/24, C07D 311/18, C07D 335/06, A61K 31/215, A61K 31/47, A61K 31/35, //C07C33/03

(21) Anmeldenummer: **86117740.0**

(22) Anmeldetag: **19.12.86**

Die Akte enthält technische Angaben, die nach dem Eingang der Anmeldung eingereicht wurden und die nicht in dieser Patentschrift enthalten sind.

## (54) Aromatische Thioether.

(30) Priorität: **23.12.85 CH 5513/85**
**04.04.86 CH 1321/86**

(43) Veröffentlichungstag der Anmeldung:
**08.07.87 Patentblatt 87/28**

(45) Bekanntmachung des Hinweises auf die Patenterteilung:
**03.04.91 Patentblatt 91/14**

(84) Benannte Vertragsstaaten:
**AT BE CH DE ES FR GB GR IT LI LU NL SE**

(56) Entgegenhaltungen:
**EP-A- 0 123 543**
**EP-A- 0 134 111**

**CHEMICAL ABSTRACTS, Band 97, Nr. 3, 19. Juli 1982, Seite 44, Zusammenfassungsnr. 16807g, Columbus, Ohio, US; P. SHEARD et al.: "Antagonists of SRS-A and leukotrienes"**

(73) Patentinhaber: **CIBA-GEIGY AG**
**Klybeckstrasse 141**
**CH-4002 Basel(CH)**

(72) Erfinder: **von Sprecher, Andreas, Dr.**
**Nelkenweg 5**
**CH-4104 Oberwil(CH)**
Erfinder: **Breitenstein, Werner, Dr.**
**St. Galler-Ring 179**
**CH-4054 Basel(CH)**
Erfinder: **Beck, Andreas, Dr.**
**Reutebachgasse 20A**
**W-7800 Freiburg(DE)**
Erfinder: **Lang, Robert W., Dr.**
**Hagenbachweg 10**
**CH-4133 Pratteln(CH)**
Erfinder: **Oertle, Konrad, Dr.**
**Vogesenstrasse 10**
**CH-4106 Therwil(CH)**

EP 0 228 045 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

CHEMICAL ABSTRACTS, Band 86, Nr. 12, 21. März 1977, Seite 14, Zusammenfassungsnr. 83500n, Columbus, Ohio, US; R.A. APPLETON et al.: "Antagonists of slow reacting substance of anaphylaxis. Synthesis of a series of chromone-2-carboxylic acids"

(74) Vertreter: Zumstein, Fritz, Dr. et al
Bräuhausstrasse 4
W-8000 München 2(DE)

## Beschreibung

Die Erfindung betrifft neuartige unsymmetrische α-Hydroxythioether, deren Schwefelatom durch eine Valenz mit einem aromatischen oder hetero-aromatischen Rest (M), wie einem gegebenenfalls substituierten und/oder mit einem 6-gliedrigen heterocyclischen Ring kondensierten Phenylrest, durch die andere Valenz mit einem mindestens 11 Kohlenstoffatome aufweisenden linearen Rest (L) verbunden ist, welcher in α-Stellung zum Schwefelatom auf einer Seite seiner Kette ein Hydroxyl, das in bezug auf das S-Atom vorzugsweise trans -orientiert ist, trägt, auf der anderen Seite eine oder mehrere Doppelbindungen und/oder einen Phenylenring aufweisen kann.

Insbesondere betrifft die Erfindung Verbindungen der Formel

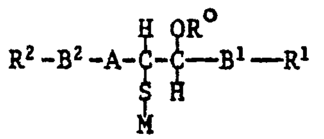

(I)

worin die allgemeinen Symbole die folgenden Bedeutungen haben:

$R^0$ ist Wasserstoff oder $C_{1-7}$-Alkanoyl;

$R^1$ ist $C_{1-3}$Alkyl, welches durch ein oder mehrere Halogene mit Atomzahl von höchstens 17 substituiert sein kann,

$R^2$ ist ein aliphatischer Rest mit 5-15 C-Atomen,

A ist eine Einfachbindung, Ethylen oder Vinylen;

$B^1$ ist $C_{1-7}$-Alkylen oder Phenylen;

$B^2$ ist eine Einfachbindung, Ethylen oder Phenylen;

M ist ein aromatischer Rest der Teilformel

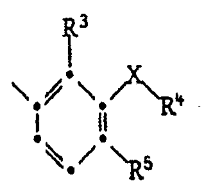

(M)

mit den folgenden Bedeutungen der Symbole:

$R^3$ ist Wasserstoff oder $C_{1-4}$-Alkyl;

X ist NH, O, S oder, falls $R^4$ Wasserstoff ist, die Einfachbindung;

eines der Symbole $R^4$ und $R^5$ bedeutet Wasserstoff und das andere die Gruppe -CO-$R^6$, oder

$R^4$ und $R^5$ zusammen bedeuten den Rest -CO-C($R^6$) = C($R^7$)- oder -CO-C($R^7$) = C($R^6$)- oder

$R^4$ und $R^5$ zusammen mit X bedeuten den Rest -N = C($R^8$)-C($R^6$) = CH-; wobei

$R^6$ für $-(CH_2)_b-COOR^3$, worin b = 0 bis 2 ist;

$R^7$ für Wasserstoff oder $C_{1-4}$-Alkyl und

$R^8$ für Wasserstoff, Methyl, Methoxy oder Halogen steht,

sowie Salze von diesen Verbindungen, sofern sie salzbildende Eigenschaften besitzen.

Die Raumdarstellung in der obigen Formel I ist für die bevorzugten Vebindungen, in welchen das O-Atom der Hydroxylgruppe mit dem S-Atom in der relativen trans -Konfiguration sind, so zu verstehen, dass die Symbole der ersten Zeile oberhalb, die der dritten Zeile dann unterhalb der Darstellungsebene liegen (oder umgekehrt), was für die abgebildete Formel der gegenseitigen Konfiguration (RS)-(SR) beider centralen Kohlenstoffatome gemäss der Kahn-Ingold-Prelog-Konvention entspricht.

Gegenstand der Erfindung bilden auch Verfahren zur Herstellung der oben definierten erfindungsgemässen Verbindungen, sowie pharmazeutische Zusammensetzungen, welche diese Verbindungen als Wirkstoff enthalten, und entsprechende Herstellungsverfahren, mit welchen solche Zusammensetzungen auf nicht-chemischem Wege hergestellt werden. - Ein weiterer Gegenstand der Erfindung ist die therapeutische Anwendung der oben definierten Verbindungen und pharmazeutischen Zusammensetzungen, insbesondere bei Linderung und Behebung solcher krankhafter Zustände, bei welchen die ausgeprägte Leukotrien-antagonisierende und/oder Phospholipase-hemmende Wirksamkeit der erfindungsgemässen Verbindungen zur Geltung kommt, wie bei Allergien verschiedener Art, vor allem bei Asthma, bzw. bei Entzündungen, vor

allem der Haut und Schleimhäute.

Vor einigen Jahren wurde nachgewiesen, vgl. H.R. Morris et al. Nature 285 , 1045-106 (May 1980) und L. Oerning, S. Hammarström und B. Samuelsson: Proc. Natl. Acad. Sci. USA 77 (4), 2014-2017 (1980), dass Leukotriene, insbesondere Leukotrien C und D, mit höchster Wahrscheinlichkeit als primäre Vermittler einer sofort einsetzenden Ueberempfindlichkeits-Reaktion für die Bronchokonstriktion bei Asthma verantwortlich sind.

Das strukturelle Grundgerüst der Leukotriene allgemein wird durch eine mehrfach ungesättigte, lineare Eicosansäure gebildet, die charakteristische Substituenten in 1-, 5- und 6-Stellung trägt, wie es für die erwähnten wichtigsten Vertreter formelmässig abgebildet wird:

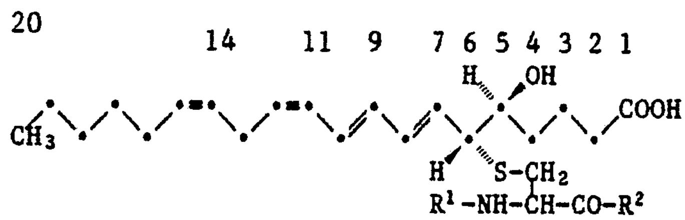

LTC-4: $R^1$ = $HOCOCH(NH_2)CH_2CH_2CO-$ ; $R^2$ = $-NHCH_2COOH$

LTD-4: $R^1$ = H- ; $R^2$ = $-NHCH_2COOH$

LTE-4: $R^1$ = H- ; $R^2$ = -OH

[Hier ist die Raumdarstellung so zu verstehen, dass die ganze olefinische Kette in der Darstellungsebene liegt und die mit Pfeil angegebenen Valenzstriche sich oberhalb der Darstellungsebene, die punktierten dagegen unterhalb der Ebene befinden.]

In ihren physiologischen Eigenschaften zeichnen sich Leukotriene im allgemeinen dadurch aus, dass sie eine markante Kontraktion von glatten Muskeln verschiedenster Art verursachen. Vom Standpunkt der Gesundheit ist ein solcher Effekt meistens unerwünscht, und dementsprechend steht die Suche nach geeigneten Leukotrien-Antagonisten im Vordergrund der Forschung auf diesem Gebiet.

In den erfindungsgemässen Verbindungen der Formel I wird das lineare Grundgerüst von bekannten Leukotrienen im Prinzip beibehalten und über das Schwefelatom mit gewissen Strukturmerkmalen kombiniert, welche üblicherweise bei aromatischen und insbesondere heteroaromatischen Verbindungen mit blutgerinnungshemmenden Eigenschaften vorkommen - überraschenderweise wirken die neuen Verbindungen in verschiedenen Testanordnungen in vitro deutlich Leukotrien-antagonisierend.

Allgemein bekannt war, dass man durch Modifikation der Eicosapolyenkette natürlich vorkommender Leucotriene, bzw. durch Einbau von Phenylengruppen, zu Leucotrienantagonisten gelangen kann. Zusätzlich geht z.B. aus der EP-134 111 hervor, dass die $LTD_4$-antagonistischen Eigenschaften solcher Modifikate erhalten bleiben, wenn man die Dipeptid-Seitenkette durch gegebenenfalls substituiertes Phenyl ersetzt. Noch einen Schritt wieter geht die Lehre der EP-123 543, demzufolge als Substitut für die Dipeptid-Seitenkette neben gegebenenfalls substituiertem Phenyl auch gegebenenfalls benzokondensierte Heteroarylreste, u.a. auch solche vom Typ des erfindungsgemäss vorgeschlagenen Restes M in Betracht kommen.

Die vorliegende Erfindung beruht demgegenüber auf der überraschenden Feststellung, dass nicht nur die $LTD_4$-antagonistische Wirkung erhalten bleibt, sondern zusätzlich eine starke Phospholipasehemmende Wirkungskomponente erzielt wird, wenn das terminale saure Zentrum entfernt wird.

So hemmen die erfindungsgemäss bereitgestellten Verbindungen im getesteten Konzentrationsbereich von etwa 0,1-25 $\mu$Mol/l die durch Leukotrien-$D_4$ ($LTD_4$ - siehe oben) induzierte Kontraktion eines glatten Muskels. Dieser sogenannte $LTD_4$-Antagonismus wird experimentell z.B. folgendermassen ermittelt: In Segmenten, die dem Ileum eines 300-400 g schweren Meerschweinchens entnommen wurden und in einem Organbad in Tyrode-Lösung bei 38° C und unter Begasung mit einem Gemisch von 95 % Sauerstoff und 5 % Kohlenstoffdioxid bei einer Belastung von 1 g inkubiert wurden, werden mit synthetischem Leukotrien $D_4$ (als Kaliumsalz) Kontraktionen ausgelöst und isotonisch registriert. Das Ausmass der Hemmung durch die Prüfsubstanz wird nach einer Vorinkubation von 2 Minuten ermittelt und als $IC_{50}$, d.h. die Konzentration, welche die Testkontraktion um 50 % reduziert, ausgewertet. Der $LTD_4$-Antagonismus kann auch in vivo durch Bronchokonstriktions-Standardtest am Meerschweinchen bei Aerosol-Verabreichung nachgewiesen werden.

Ueberraschenderweise üben Verbindungen der Formel I auch eine ausgeprägte Hemmwirkung auf andere physiologisch wichtige Enzymsysteme aus. So wurde die Hemmung von Phospholipase $A_2$ aus menschlichen Leukocyten im getesteten Konzentrationsbereich von etwa 0,5-50 $\mu$Mol/l beobachtet. Eben-

falls wurde eine Hemmung von Phospholipase C aus menschlichen Thrombocyten im getesteten Konzentrationsbereich von etwa 1-100 µMol/l beobachtet.

Die durch diese Methoden in vitro angedeuteten antiallergischen bzw. antiinflammatorischen Eigenschaften werden auch im Tierversuch in vivo bestätigt. So lässt sich die lokale antiinflammatorische Wirksamkeit beispielsweise nach der von G. Tonelli und L. Thibault [Endocrinology 77 , 625 (1965)] entwickelten Methode durch Hemmung des mit Crotonöl induzierten Rattenohroedems bei der Normalratte im Dosisbereich von etwa 1 bis etwa 100 mg/ml nachweisen.

Dank diesen wertvollen pharmakologischen Eigenschaften können die erfindungsgemässen Verbindungen der Formel I überall dort therapeutische Anwendung finden, wo die allergogene Wirkung der Leukotriene zu krankhaften Zuständen führt und zu mildern oder zu beheben ist. Demzufolge können sie beispielsweise zur Behandlung allergischer Zustände und Erkrankungen, wie insbesondere Asthma, aber auch Heufieber sowie obstruktiver Lungenkrankheiten einschliesslich zystischer Fibrose, verwendet werden. Ebenfalls sind sie, dank ihrer antiinflammatorischen Wirksamkeit, als entzündungshemmende Mittel, insbesondere als externe (topische) Hautphlogistatika für die Behandlung enzündlicher Dermatosen jeglicher Genese, wie bei leichten Hautirritationen, Kontaktdermatitis, Exanthemen und Verbrennungen, sowie als Schleimhautphlogostatika für die Behandlung von Mukosaentzündungen, z.B. der Augen, Nase, Lippen, Mund und Genital-, bzw. Analregion, geeignet. Ferner können sie als Sonnenschutzmittel verwendet werden. Die hohe hemmende Wirksamkeit auf verschiedene Blutfaktoren weist zudem auf die Möglichkeit der therapeutischen Anwendung der Verbindungen der Formel I im Indikationsbereich Thrombose und Blutgerinnung hin.

Wie bereits oben erwähnt wurde, ist im Strukturaufbau der erfindungsgemässen Verbindungen der Formel I und Leukotrienen allgemeine Analogie zu finden, insbesondere in der eingangs definierten bevorzugten trans-Konfiguration des vicinalen S- und O-Atoms und im linearen Aufbau des Rests (L). Von Leukotrienen unterscheidet sich dieses dagegen dadurch, dass ihm die charakteristische terminale Carboxylgruppe fehlt oder durch verschiedene Anzahl Halogene ersetzt wird. Im Unterschied zu Leukotrienen ist bei ihm auch die Anzahl, Charakter und Raumordnung der Mehrfachbindungen nicht wesentlich, da sie sogar fehlen oder durch Phenylenreste ersetzt werden können. Auch die Gesamtlänge des Rests (L) ist für die Wirksamkeit in breiten Grenzen nebensächlich, und nicht einmal die absolute, oder sogar die relative, Konfiguration beider oben diskutierten asymmetrischen C-Atome ist für die Wirksamkeit kritisch, wie man es z.B. an wirksamen 5(R),6(S)-Epimeren zeigen kann, welche die gegenüber natürlichen Leukotrienen umgekehrte absolute Konfiguration der Kohlenstoffatome 5 und 6 der Kohlenwasserstoff-Kette (L) hat.

Unter bevorzugten Bedeutungen von $R^0$ in Formel I ist in erster Linie Wasserstoff, ferner auch $C_{1-4}$-Alkanoyl, wie Acetyl, zu nennen.

In der oben definierten Formel I steht Symbol $R^1$ beispielsweise für ein unsubstituiertes Alkyl, wie Ethyl, Propyl oder insbesondere Methyl, oder ein analoges durch Chlor oder Fluor, insbesondere am endständigen C-Atom, substituiertes Alkyl, wie Chlor- oder Fluormethyl, 2-Fluorethyl, oder 3-Fluorpropyl, oder aber ein Perfluoralkyl, wie vor allem Trifluormethyl.

Der durch Symbol $R^2$ dargestellte aliphatische Rest ist vorzugsweise ein linearer Rest, z.B. ein Alkylrest, bestehend aus 5-15, vorzugsweise 7-12 C-Atomen, wie insbesondere Heptyl, Nonyl, Undecyl und Dodecyl, oder ein entsprechender einfach oder mehrfach ungesättigter Rest, der eine, zwei oder drei mehrfache Bindungen, wie Dreifachbindungen und vor allem Doppelbindungen beliebig in cis - oder trans - Konfiguration, in beliebigen Kombinationen trägt. Diese Mehrfachbindungen befinden sich vorzugsweise möglichst nahe dem Schwefelatom, d.h. in $\alpha,\beta$-Stellung zum schwefeltragenden C-Atom bzw. konjugiert mit dem durch A dargestellten Vinylenrest. Bevorzugte Reste $R^2$ dieser Art sind z.B. 1-Alkenyl-, 1,3-Alkadienyl- und 1,3,6-Alkatrienyl-Reste, wie insbesondere 1-Heptenyl, 1-Octenyl, 1-Nonenyl, 1-Decenyl, 1-Undecenyl und 1-Dodecenyl bzw. 1,3-Octadienyl, 1,3-Decadienyl, 1,3-Dodecadienyl sowie 1,3,6-Dodecatrienyl, in welchen allen die Doppelbindungen je einzeln in cis - oder trans -Konfiguration vorliegen und beliebige Kombinationen bilden können.

Der durch Symbol A in Formel I dargestellte Vinylenrest kann in cis -oder trans -Konfiguration vorliegen.

Falls $B^1$ in Formel I ein $C_{1-7}$-Alkylen darstellt, ist es vorzugsweise ein solches der Teilformel $-(CH_2)_a-$, worin a gleich 1 bis 7, vorzugsweise 2 bis 4, ist. Falls $B^1$ ein Phenylen darstellt, ist es vorzugsweise m-Phenylen; es kann, ungeachtet des Rests $R^1$, zusätzlich einen oder auch mehrere $C_{1-4}$-Alkylreste, vor allem Methylreste, die insgesamt höchstens 6 C-Atome zählen, tragen, ist aber vorzugsweise unsubstituiert.

Symbol $B^2$ in Formel I stellt vorzugsweise die Einfachbindung oder ein Phenylen, wie insbesondere o - oder p -Phenylen, dar; das Phenylen kann, analog wie beim Symbol $B^1$ angegeben wurde, zusätzlich Alkylreste bis zur Gesamtzahl von 6 C-Atomen tragen, vorzugsweise ist es aber unsubstituiert. Falls $B^2$ für Phenylen steht, bedeutet A vorzugsweise die Einfachbindung oder Vinylen.

Das in der eingangs angegebenen Formel I definierte Symbol $R^3$ bedeutet einerseits, als Bestandteil

von Symbol $R^6$, vorzugsweise Methyl und vor allem Wasserstoff; andererseits, als der Substituent des Phenylrings, vorzugsweise Wasserstoff oder Propyl.

Unter aromatischen Resten der Teilformel (M), d.h. unter denjenigen, worin der Phenylrest in m - oder p -Stellung einen aliphatischen Substituenten trägt, sind solche hervorzuheben, worin je $R^3$ und $R^4$ Wasserstoff, X die Einfachbindung und $R^5$ den Rest $-CO-(CH_2)_b-COOR^9$ bedeuten (wobei b vorzugsweise für 2 und $R^9$ für Methyl, Ethyl oder vorzugsweise Wasserstoff steht), und insbesondere solche, worin je $R^3$ und $R^5$ Wasserstoff, X die Gruppe -NH- und $R^4$ den Rest $-CO-(CH_2)_b-COOR^9$ darstellt (wobei b für 0, 2 oder vorzugsweise 1 und $R^9$ für Methyl, Ethyl oder vorzugsweise Wasserstoff steht), d.h. Reste der Teilformel

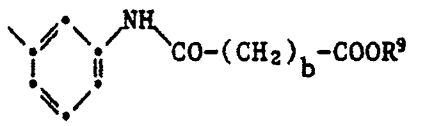

(M²),

worin b und $R^9$ die obige Bedeutung haben.

Unter heteroaromatischen Resten der Teilformel (M) sind in erster Linie sauerstoffhaltige, von Chromen abgeleitete Reste hervorzuheben, insbesondere solche der Teilformel

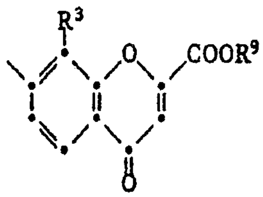

(M¹)

oder auch der Teilformel

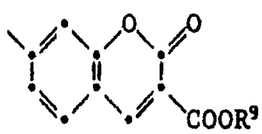

(M⁴),

worin $R^3$ die obgenannten Bedeutungen hat und vorzugsweise für Propyl oder insbesondere Wasserstoff steht und $R^9$ Ethyl oder insbesondere Methyl und vorzugsweise Wasserstoff darstellt.

Unter heteroaromatischen Resten der Teilformel (M) sind auch stickstoffhaltige, vom Chinolin abgeleitete Reste der Teilformel

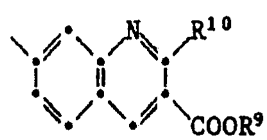

(M³)

zu erwähnen, worin $R^9$ Ethyl oder insbesondere Methyl oder auch Wasserstoff, und $R^{10}$ Halogen, wie insbesondere Chlor, oder vor allem Methoxyl darstellt.

Unter diesen hervorgehobenen Verbindungen sind auch diejenigen als besonders bevorzugt mitinbegriffen, worin die Carboxylgruppe in Form eines Salzes, insbesondere eines Alkalimetallsalzes, vorliegt.

Auch die übrigen Verbindungen der Formel I können, ihrem individuellen Charakter nach , auch in Form von Salzen vorliegen. Diejenigen davon, welche eine genügende Acidität haben, wie insbesondere die mit freien Carboxylgruppen, können Salze mit Basen, wie insbesondere anorganischen Basen, vorzugsweise physiologisch verträgliche Alkalimetall-Salze, vor allem Natrium- und Kalium-Salze, bilden. Diejenigen der Verbindungen der Formel I, welche eine genügende Basizität haben, wie Ester von Chinolinderivaten der oben charakterisierten Teilformel $M^3$, können als Säureadditionssalze, insbesondere als physiologisch verträgliche Salze, mit üblichen pharmazeutisch anwendbaren Säuren vorliegen; von anorganischen Säuren sind die Halogenwasserstoffsäuren, wie die Chlorwasserstoffsäure, sowie Schwefelsäure und Phosphor- bzw. Pyrophosphorsäure besonders zu nennen, von den organischen Säuren sind es in erster Linie die

Sulfonsäuren, z.B. die aromatischen, wie Benzol- oder p-Toluol-sulfonsäure, Embonsäure und Sulfanilsäure, oder Niederalkansulfonsäuren, wie Methansulfon-, Ethansulfon-, Hydroxyethansulfon-sowie Ethylendisulfonsäure, oder aber auch aliphatische, alicyclische, aromatische oder heterocyclische Carbonsäuren, wie Ameisen-, Essig-, Propion-, Bernstein-, Glykol-, Milch-, Aepfel-, Wein-, Zitronen-, Fumar-, Malein-, Hydroxymalein-, Oxal-, Brenztrauben-, Phenylessig-, Benzoe-, p-Aminobenzoe-, Anthranil-, p-Hydroxybenzoe-, Salicyl- und p-Aminosalicylsäure, sowie Ascorbinsäure. Verbindungen der Formel I, die sowohl basische wie saure funktionelle Gruppen, wie Chinolinderivate der Teilformel $M^3$ mit freier Carboxylgruppe, enthalten, können auch als innere Salze vorliegen.

Unter den erfindungsgemässen Verbindungen der Formel I sind solche hervorzuheben, worin die Symbole die folgenden Bedeutungen haben; $R^0$ ist ein $C_{1-4}$-Alkanoyl oder vorzugsweise Wasserstoff; $R^1$ ist Methyl, Chlormethyl oder Trifluormethyl; $R^2$ ist ein lineares Alkyl mit 7-15 C-Atomen oder ein entsprechender Rest mit 2, 3 oder vorzugsweise einer Doppelbindung, wie einer der oben genannten; A ist *cis* -oder *trans* -Vinylen; $B^1$ ist ein lineares Alkylen mit 2-5, vorzugsweise 2 oder 3 C-Atomen; $B^2$ ist die Einfachbindung und M hat eine der unter den Teilformeln $M^2$, $M^3$ und vorzugsweise $M^1$ oben angegebenen Bedeutungen, darunter ganz besonders Verbindungen mit freier Carboxylgruppe und ihre pharmakologisch verträglichen Salze, z.B. Alkalimetallsalze, wie Natrium- und Kaliumsalze.

Hervorzuheben sind auch diejenigen Verbindungen der Formel I, worin die Symbole die folgenden Bedeutungen haben: $R^0$ ist ein $C_{1-4}$-Alkanoyl oder vorzugsweise Wasserstoff; $R^1$ ist Methyl, Chlormethyl oder Trifluormethyl; $R^2$ ist ein lineares Alkyl mit 7-15, vorzugsweise mit 8-12, C-Atomen, wie einer der oben genannten; A ist die Einfachbindung oder Vinylen in *cis* - oder *trans* -Konfiguration, $B^1$ ist ein lineares Alkylen mit 2-5, vorzugsweise 2 oder 3 C-Atomen; $B^2$ ist Phenylen, insbesondere *o* - oder *p* -Phenylen und M hat eine der unter den Teilformeln $M^2$, $M^3$ und vorzugsweise $M^1$ oben angegebenen Bedeutungen, darunter ganz besonders Verbindungen mit freier Carboxylgruppe und ihre pharmakologisch verträglichen Salze, z.B. Alkalimetallsalze, wie Natrium- und Kaliumsalze.

Besonders sind hervorzuheben diejenigen Verbindungen der Formel I, worin die Symbole die folgenden Bedeutungen haben: $R^0$ ist ein $C_{1-4}$-Alkanoyl oder vorzugsweise Wasserstoff; $R^1$ ist Methyl, Chlormethyl oder Trifluormethyl; $R^2$ ist ein lineares Alkyl mit 7-15 C-Atomen oder ein entsprechender Rest mit 2, 3 oder vorzugsweise einer Doppelbindung, wie einer der oben genannten; A ist *cis* -oder *trans* -Vinylen; $B^1$ ist Phenylen, insbesondere *m* -Phenylen; $B^2$ ist die Einfachbindung und M hat eine der unter den Teilformeln $M^2$, $M^3$ und vorzugsweise $M^1$ oben angegebenen Bedeutungen, darunter ganz besonders Verbindungen mit freier Carboxylgruppe und ihre pharmakologisch verträglichen Salze, z.B. Alkalimetallsalze, wie Natrium-und Kaliumsalze.

Besonders hervorzuheben sind auch diejenigen Verbindungen der Formel I, worin die Symbole die folgenden Bedeutungen haben: $R^0$ ist ein $C_{1-4}$-Alkanoyl oder vorzugsweise Wasserstoff; $R^1$ ist Methyl, Chlormethyl oder Trifluormethyl; $R^2$ ist ein lineares Alkyl mit 7-15, vorzugsweise mit 8-12, C-Atomen, wie einer der oben genannten; A ist die Einfachbindung oder insbesondere Vinylen in *cis* - oder *trans*-Konfiguration, $B^1$ ist Phenylen, insbesondere *m* -Phenylen, $B^2$ ist Phenylen, insbesondere *o* - oder *p* -Phenylen und M hat eine der unter den Teilformeln $M^2$, $M^3$ und vorzugsweise $M^1$ oben angegebenen Bedeutungen, darunter ganz besonders Verbindungen mit freier Carboxylgruppe und ihre pharmakologisch verträglichen Salze, z.B. Alkalimetallsalze, wie Natrium- und Kaliumsalze.

In erster Linie sind die in den Beispielen beschriebenen Verbindungen der Formel I hervorzuheben.

Die erfindungsgemässen Thioether können in an sich bekannter Weise hergestellt werden, beispielsweise dadurch, dass man ein dem eingangs definierten Rest (L) entsprechendes aliphatisches *cis* - oder vorzugsweise *trans* -Epoxid von mindestens 11 C-Atomen, insbesondere ein solches der Formel

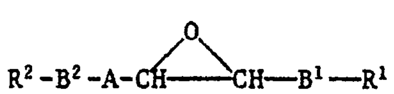

(II)

worin A, $B^1$, $B^2$, $R^1$ und $R^2$ die oben genannten Bedeutungen haben und worin vorzugsweise beide Wasserstoffe am Oxiran-Ring gegeneinander *trans* orientiert sind, mit einer dem oben definierten Rest (M) entsprechenden Mercapto-Verbindung der Formel

$$H\text{-}S\text{-}M \qquad (III)$$

worin M die obgenannte Bedeutung hat, oder mit ihrem Salz umsetzt und, wenn notwendig oder erwünscht,

eine erhaltene Verbindung der Formel I, worin $R^0$ Wasserstoff bedeutet, zu einer entsprechenden Verbindung, worin $R^0$ ein $C_{1-7}$-Alkanoyl ist, acyliert, und/oder eine als Ester vorliegende Verbindung zur freien Säure oder einem Salz davon verseift und, wenn erwünscht, eine erhaltene freie Verbindung mit salzbildenden Eigenschaften in ein Salz davon oder ein erhaltenes Salz in eine freie Verbindung überführt.

Die Umwandlung erfolgt unter an sich bekannten Bedingungen bei Temperaturen von etwa -20 °C bis etwa +50 °C, vorzugsweise bei Raumtemperatur, und vornehmlich in einem basischen Milieu, beispielsweise in Gegenwart eines Amins, insbesondere eines tertiären aliphatischen, arylaliphatischen oder gesättigten heterocyclischen Amins, wie Trialkylamin (z.B. Triethylamin, oder Ethyl-diisopropylamin), Dialkyl-benzylamin (z.B. N,N-Dimethylbenzylamin), N,N-Dialkylanilin (z.B. N,N-Dimethylanilin) bzw. N-Methyl- oder N-Ethylpiperidin oder N,N'-Dimethylpiperazin. Ueblicherwiese wird die Umsetzung in einem indifferenten organischen Lösungsmittel, wie einem Niederalkanol, z.B. Methanol oder Ethanol, durchgeführt.

Die gegebenenfalls durchzuführende nachträgliche Acylierung der im Hauptverfahren gebildeten Hydroxylgruppe, welche zu Verbindungen der Formel I, worin $R^0$ für $C_{1-7}$-Alkanoyl steht, führt, kann in an sich bekannter Weise durchgeführt werden, z.B. durch Behandeln des primären Produkts, worin $R^0$ für Wasserstoff steht, mit der gewünschten Säure, wie beispielsweise der Ameisensäure, oder mit einem geeigneten reaktionsfähigen Säurederivat, insbesondere einem Halogenid (vorzugsweise Chlorid), symmetrischem Anhydrid, gemischtem Anhydrid (insbesondere einem solchen mit Trifluoressigsäure) oder Keten. Als Reaktionsmedium kann man z.B. überschüssiges Acylierungsmittel verwenden, sowie auch neutrale, nicht acylierbare organische Lösungsmittel, wie Kohlenwasserstoffe (z.B. Pentan, Hexan, Cyclohexan), halogenierte Kohlenwasserstoffe (z.B. Methylenchlorid, Chloroform), Ether (z.B. Diethylether, Ethylenglykoldimethylether, Tetrahydrofuran, Dioxan), Säureester (z.B. Ethylacetat) und Säureamide (z.B. Acetamid, Dimethylformamid); und gegebenenfalls auch nicht acylierbare organische Basen verschiedener Basizität, wie heteroaromatische Basen (z.B. Pyridin, Collidin, Chinolin), tertiäre Amine (z.B. Triethylamin, N-Ethylpiperidin, N-Methylmorpholin, N,N'-Dimethylpiperazin) oder 1,5-Diazabicyclo[5,4,0]undec-5-en; oder aber man arbeitet mit zweckmässigen Kombinationen aller dieser Lösungsmittel. Die Reaktionstemperatur kann sich im Bereich von etwa -70° bis zur Siedetemperatur des Gemisches, vorzugsweise zwischen etwa -20° bis etwa +30 °C, bewegen.

In der Hauptreaktion (Kondensation mit Epoxid) wird die Mercapto-Komponente der Formel III vornehmlich in Form ihres $C_{1-4}$-Alkylesters (wie Methyl- oder Ethyl-esters) eingesetzt; falls der erfindungsgemässe Endstoff als freie Säure oder ihr Salz erwünscht ist, dann muss der erhaltene Ester hydrolysiert werden. Die Hydrolyse wird unter den üblichen Bedingungen durchgeführt, z.B. mit Alkalimetallcarbonaten (z.B. Natrium- oder Kaliumcarbonat) oder verdünnten Alkalilaugen (z.B. Natrium- oder Kalium-hydroxid) in Anwesenheit von Wasser in einem mit Wasser mischbaren organischen Lösungsmittel, wie einem Niederalkanol (z.B. Methanol oder Ethanol) oder cyclischen Ether (z.B. Tetrahydrofuran oder Dioxan) bei Temperaturen von etwa 0-80 °C, vorzugsweise bei Raumtemperatur. Man kann bei besonders empfindlichen Verbindungen unter noch milderen Bedingungen, wie insbesondere bei niedriger Temperatur (vorzugsweise unterhalb Raumtemperatur), mit einer äquivalenten stöchiometrischen Menge Alkali und bei verkürzter Reaktionszeit, gegebenenfalls unter analytischer Kontrolle, z.B. durch Dünnschichtchromatographie, die Estergruppe unter Erhaltung von weniger alkali-stabilen Gruppierungen, wie solchen von Ketocarbonsäuren, abspalten, eine acylierte Hydroxylgruppe im Rest $R^0$ wird dabei jedoch meistenfalls gleichzeitig abgespalten.

Ausgangsstoffe für das erfindungsgemässe Kondensationsverfahren sind entweder an sich bekannt oder nach bekannten Analogieverfahren in an sich bekannter Weise erhältlich. - So sind z.B. die wichtigen Mercapto-Verbindungen der Formel III beschrieben (vgl. z.B. EP-OLO123543), andere analoge Säuren sind auf dieselbe Weise ausgehend von entsprechenden bekannten Ausgangsstoffen zugänglich.

Das als Ausgangsstoff verwendete cis - oder vorzugsweise trans -Epoxid, z.B. jenes der oben definierten Formel II, kann vornehmlich mittels denselben Verfahren hergestellt werden, welche auch bei der Synthese von Leukotrienen verwendet werden. Bei einem typischen allgemeinen Syntheseweg wird z.B. von einem $R^1$-substituierten Benzaldehyd oder einem gesättigten aliphatischen Aldehyd (Alkanal) der Formel

$$O{=}CH{-}(CH_2)_a{-}R^1 \qquad (IV)$$

ausgegangen, worin a und $R^1$ die oben angegebenen Bedeutungen haben.

Diese Verbindung wird mit Formylmethylentriphenylphosphoran (oder einem äquivalenten Reagens) kondensiert, womit der entsprechende $\alpha,\beta$-ungesättigte Aldehyd, 2-trans -alkenal, der Formel

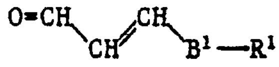

(V)

entsteht, worin $B^1$ die oben angegebenen Bedeutungen hat. Diese Verbindung wird anschliessend in an sich bekannter Weise, vorzugsweise unter schwach alkalischen Bedingungen (z.B. in Gegenwart von Alkalicarbonaten) mit wässrigem Wasserstoffperoxid epoxidiert, wodurch ein trans-Epoxid, 2-(RS),3(SR)-Epoxy-Alkanal der Formel

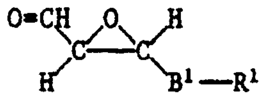

(VI)

resultiert, worin $B^1$ die oben angegebenen Bedeutungen hat. In analoger Weise ergibt ein Aldehyd mit cis - Doppelbindung die entsprechende cis -Epoxy-Konfiguration im 2(RS), 3(RS)-Epoxyalkdehyd. Dieser Epoxyaldehyd kann zum gewünschten trans -ungesättigten Epoxid, z.B. dem der oben definierten Formel II, worin A für den Vinylenrest steht, durch Kondensation mit einem entsprechenden bekannten Benzyliden- bzw. Alkylidentriphenylphosphoran kondensiert werden.

Für mehrfach ungesättigte Epoxide, z.B. diejenigen der Formel II, worin $R^2$ eine oder mehrere Doppelbindungen aufweist, bietet sich eine indirekte Alternative an: anstelle der Wittig-Reaktion mit einem in seiner Kette ungesättigten Yliden-Phoshoran wird der Aldehyd IV zuerst mit $\gamma$-Triphenylphosphoranylidenbutyraldehyd (4-Triphenylphosphoranyliden-butanal) um 4 Kohlenstoffatome verlängert, epoxidiert und erst das erhaltene 4(RS),5(RS)-Epoxy-2-alkenal mit einem einfachen, gesättigten Alkylidentriphenylphosphoran oder einem weniger komplizierten Benzyliden- bzw. Alkenylidentriphenylphosphoran zum gewünschten Epoxid (z.B. einem der Formel II) kondensiert. - Bei Epoxiden der Formel II, worin A eine Einfachbindung und $B^2$ ein Phenylen ist, wird der Aldehyd IV mit einem entsprechenden Benzylidentriphenylphosporan umgesetzt und anschliessend epoxidiert. In diesem Falle entsteht jedoch meistenfalls ein Gemisch von cis - und trans -Styryl-Derivaten, welches entweder in beide individuelle Isomere aufgetrennt werden muss, oder aber zum Gemisch beider isomerer Epoxide führt, aus welchem dann eventuell im Hauptverfahren 4 Stereoisomere entstehen.

Wenn individuelle Diastereomere erwünscht wind, so kann vorteilhaft an beliebiger Stufe ein individuelles Diastereomeres eines Ausgangsstoffes eingesetzt werden oder aus einem razemischen oder optisch inaktiven Ausgangsstoff durch stereoselektive Reaktionsbedingungen oder optisch aktive Reagentien ein Diastereomeres bevorzugt gebildet werden, oder razemische Diastereomerengemische durch physikalische Trennmethoden, gegebenenfalls unter Anwendung optisch aktiver Hilfsstoffe, in optisch individuelle Diastereomere aufgetrennt werden.

In stereochemischer Hinsicht wird jedoch sowohl die erfindungsgemässe Kondensation der Bildungskomponenten II und III, wie auch die Zubereitung der Ausgangsstoffe vornehmlich so durchgeführt, dass man jeweils stereochemisch einheitliche Ausgangsstoffe einsetzt, die Reaktionen nach Möglichkeit stereoselektiv, z.B. durch Einsatz von optisch aktiven Reagentien und/oder Hilfsstoffen, durchführt und stereochemisch einheitliche Produkte aus Reaktionsgemischen unmittelbar nach der Reaktion isoliert. So werden z.B. bei Herstellung der ungesättigten Ausgangsstoffe gegebenenfalls gebildete Isomere mit cis - und trans - Doppelbindungen sofort voneinander getrennt, wozu die üblichen physikalischen Trennungsmethoden, wie insbesondere Chromatographie, geeignet sind. In der Hauptreaktion wird vornehmlich das Epoxid der Formel II als ein individuelles trans -Stereoisomer, jedoch in razemischer Form (wie es normalerweise durch die Epoxydierung eines Olefins gebildet wird) eingesetzt; die Mercapto-Komponente der Formel III, sofern sie optisch aktiv ist, wird vorzugsweise als ein individueller optischer Antipode verwendet -diese Massnahme ermöglicht, die beiden gebildeten optisch aktiven Diastereomeren einfach durch geläufige physikalische Methoden, wie Chromatographie, voneinander zu trennen; falls eine optisch inaktive Mercapto-Komponente verwendet wird, ist es zur Gewinnung individueller optisch aktiver Produkte unumgänglich, die Methoden der Spaltung in Antipode mittels optisch aktiver Hilfsstoffe, wie z.B. die Bildung von Salzen mit optisch aktiven Basen, anzuwenden. Alle geeigneten Trennungsverfahren sind an sich bekannt und können auch wiederholt oder untereinander zweckmässig kombiniert werden.

Infolge der engen Beziehung zwischen den neuen Verbindungen in freier Form und in Form ihrer Salze sind im vorausgegangenen und nachfolgend unter den freien Verbindungen oder ihren Salzen sinngemäss auch die entsprechenden Salze bzw. freien Verbindungen zu verstehen.

Die Erfindung betrifft auch diejenigen Ausführungsformen des Verfahrens, nach denen man von einer

auf irgendeiner Stufe des Verfahrens als Zwischenprodukt erhältlichen Verbindung ausgeht und die fehlenden Schritte durchführt oder einen Ausgangsstoff in Form eines Salzes verwendet oder unter den Reaktionsbedingungen bildet.

Die bei den erfindungsgemässen Verfahren und ihren Vorstufen auftretenden neuen Ausgangsstoffe und Zwischenprodukte bilden ebenfalls Gegenstand der Erfindung .

Vorzugsweise werden solche Ausgangsstoffe verwendet, und die Reaktionsbedingungen so gewählt, dass man zu den vorstehend hervorgehobenen oder als besonders bevorzugt aufgeführten Verbindungen gelangt.

Die vorliegende Erfindung betrifft ebenfalls pharmazeutische Zusammensetzungen und Arzneimittel, welche eine der erfindungsgemässen Verbindungen der Formel I oder ein pharmazeutisch verwendbares Salz davon enthalten. Bei den erfindungsgemässen pharmazeutischen Zusammensetzungen handelt es sich insbesondere um solche, die zur lokalen Verabreichung und vor allem zur Inhalationsverabreichung, z.B. in Form eines Aerosols, mikronisierten Pulvers oder einer feinversprühten Lösung, an Säuger, vor allem Menschen, bestimmt sind und den Wirkstoff allein oder zusammen mit einem pharmazeutisch anwendbaren Trägermaterial enthalten.

Pharmazeutische Präparate für topische und lokale Verwendung sind z.B. für die Behandlung der Haut Lotionen und Cremen, die eine flüssige oder semifeste Oel-in-Wasser- oder Wasser-in-Oel-Emulsion enthalten, und Salben (wobei solche vorzugsweise ein Konservierungsmittel enthalten). Für die Behandlung der Augen eignen sich Augentropfen, welche die aktive Verbindung in wässriger oder öliger Lösung enthalten, und Augensalben, die vorzugsweise in steriler Form hergestellt werden. Für die Behandlung der Nase sind Aerosole und Sprays (ähnlich den weiter unten beschriebenen für die Behandlung der Atemwege), grobe Puder, die durch schnelles Inhalieren durch die Nasenlöcher verabreicht werden, und vor allem Nasentropfen, welche die aktive Verbindung in wässriger oder öliger Lösung enthalten, geeignet; für die lokale Behandlung der Mundhöhle eignen sich auch Lutschbonbons, welche die aktive Verbindung in einer im allgemeinen aus Zucker und Gummiarabikum oder Tragaganth gebildeten Masse enthalten, welcher Geschmacksstoffe beigegeben sein können, sowie Pastillen, die den Aktivstoff in einer inerten Masse, z.B. aus Gelatine und Glycerin oder Zucker und Gummiarabikum, enthalten.

Als pharmazeutische Zusammensetzungen für die Verabreichung in Form von Aerosolen oder Sprays sind geeignet z.B. Lösungen, Suspensionen oder Emulsionen des erfindungsgemässen Wirkstoffs der Formel I mit einem geeigneten pharmazeutisch annehmbaren Lösungsmittel, wie insbesondere Ethanol und Wasser, oder einem Gemisch solcher Lösungsmittel. Sie können nach Bedarf auch andere pharmazeutische Hilfsstoffe, wie nicht-ionische oder anionische oberflächenaktive Mittel, Emulgatoren und Stabilisatoren, sowie Wirkstoffe anderer Art enthalten und vor allem zweckmässig mit einem Treibgas, wie einem inerten Gas unter erhöhtem Druck oder insbesondere mit einer leicht flüchtigen, vorzugsweise unter normalem atmosphärischem Druck unterhalb der üblichen Raumtemperatur (z.B. zwischen etwa -30 und +10° C) siedenden Flüssigkeit, wie einem mindestens teilweise fluorierten polyhalogenierten Niederalkan, oder einem Gemisch solcher Flüssigkeiten, vermischt ist. Derartige pharmazeutische Zusammensetzungen, welche vorwiegend als Zwischenprodukte oder Vorratsgemische für die Herstellung der entsprechendenden Arzneimittel in fertiger Form verwendet werden, enthalten den Wirkstoff üblicherweise in einer Konzentration von etwa 0,1 bis etwa 10, insbesondere von etwa 0,3 bis etwa 3 Gewichts-%. - Zur Herstellung von Arzneimitteln in fertiger Form wird eine solche pharmazeutische Zusammensetzung in geeignete Behälter, wie Flacons und Druckflaschen, abgefüllt, welche mit einer für solche Zwecke geeigneten Versprühungseinrichtung bzw. Ventil versehen sind. Das Ventil ist vorzugsweise als ein Dosierungsventil konstruiert, welches bei der Betätigung eine vorbestimmte Menge der Flüssigkeit, entsprechend einer vorbestimmten Dosis des Wirkstoffs, freigibt. Bei Herstellung der fertigen Arzneimittelform kann man auch so vorgehen, dass entsprechende Mengen der als Vorratslösung vorliegenden pharmazeutischen Zusammensetzung und des Treibmittels separat in die Behälter abgefüllt werden und erst dort zur Vermischung kommen. Die Dosierung des zu verabreichenden Wirkstoffs der Formel I und die Häufigkeit der Verabreichung hängen ab von der jeweiligen Wirksamkeit bzw. der Länge der Wirkung jeder individuellen dieser Verbindungen, von der Stärke der zu behandelnden Krankheit bzw. ihrer Symptome, sowie vom Geschlecht, Alter, Gewicht und individueller Ansprechbarkeit des zu behandelnden Säugers. Im Durchschnitt dürfte die empfohlene tägliche Dosis einer erfindungsgemässen Verbindung der Formel I bei einem 75 kg schweren Säuger (in erster Linie Menschen) im Bereich von etwa 10 bis etwa 500, vorzugsweise zwischen etwa 25 bis etwa 250 mg liegen, wobei die Verabreichung zweckmässig nach Bedarf in mehreren Dosen pro Tag erfolgen kann.

Die Erfindung betrifft auch die Anwendung der erfindungsgemässen Wirkstoffe der Formel I zur Linderung oder Behebung krankhafter Zustände und/oder Symptome des Körpers eines Säugers, insbesondere des Menschen, welche auf die allergogene Einwirkung von Leukotrienen zurückzuführen sind und insbesondere bei Asthma vorkommen. Diese Anwendung bzw. die entsprechende Heilmethode, ist durch

die Behandlung des leidenden Körpers bzw. Körperteils mit einer antiallergisch wirksamen Menge einer Verbindung der Formel I allein oder in Form eines Arzneimittels, insbesondere einer zur Inhalation bestimmten pharmazeutischen Zusammensetzung, charakterisiert. Unter der Bezeichnung "eine antiallergisch wirksame Menge" ist eine solche Menge des Wirkstoffes zu verstehen, die zu einer signifikanten Inhibition der durch Leukotriene verursachten Kontraktionen ausreicht.

Die folgenden Beispiele illustrieren näher die vorliegende Erfindung, ohne sie in ihrem Umfang einzuschränken. Alle Temperaturen sind in Celsiusgraden angegeben.

Beispiel 1: 3-[5S),6(R)-5-Hydroxy-7-cis-pentadecen-6-yl-thio]-malonanilinsäure-methylester und dessen 5-(R),6(S)-Stereoisomeres.

Zu einer Lösung von 1,1 g 5(S),6(S)-5,6-Epoxy-7-cis-pentadecen in 10 ml Methanol gibt man 6,2 ml Triethylamin und 1,2 g 3-Mercaptomalonanilinsäure-methylester (EP-OL 0123543), rührt 16 Stunden bei Raumtemperatur, dampft im Vakuum zur Trockne ein und chromatographiert den Rückstand an Kieselgel mit Hexan/Ethylacetat (3:2). Man erhält die Titelverbindung als hellgelbes Oel,
$[\alpha]_D^{20}$ = +3,3 ± 1.6° (c = 0,62 %, in Chloroform).

In analoger Weise ergibt das entsprechende 5(R),6(R)-Epoxid den stereoisomeren 3-[5(R),6(S)-5-Hydroxy-7-cis-pentadecen-6-yl-thio]-malonanilinsäure-methylester.

Das als Ausgangsstoff verwendete 5(S),6(S)-5,6-Epoxy-7-cis-pentadecen wird beispielsweise folgendermassen hergestellt:

a) 2-trans-Heptenol

Zu einer Lösung von 10 g Lithiumaluminiumhydrid in 400 ml Ether werden bei 0° C 16.9 g 2-Heptinol in 200 ml Ether innerhalb von 30 Minuten unter Rühren zugetropft, und das entstandene Reaktionsgemisch wird über Nacht unter Rückfluss gekocht. Der Ueberschuss von $LiAlH_4$ wird unter Kühlen in einem Eis-Wasser-Bad durch Zugabe von 40 ml Ethylacetat zerstört und das resultierende Reaktionsgemisch zwischen Ether und kalter 1N Schwefelsäure aufgenommen. Die angesäuerte (pH 2) wässrige Schicht wird noch mit Ether nachextrahiert, die vereinigten organischen Extrakte werden über Magnesiumsulfat getrocknet und im Vakuum eingedampft. Destillation des Rückstandes (18 g) unter vermindertem Druck ergibt 13.2 g 2-trans-Heptenol als farbloses Oel, Sdp. 71.5-72° C/13 mbar.

b) 2(R),3(R)-2,3-Epoxyheptanol

Unter wasserfreien Bedingungen wird eine gerührte Lösung von 66.3 ml Tetraisopropylorthotitanat und 38.51 ml D-(-)-Weinsäurediethylester in 1.1 1 Methylenchlorid bei -23° C nacheinander mit 25.7 g 2-trans-Heptenol (siehe oben) und 140 ml einer 3.2M-Lösung von tert-Butylhydroperoxid in Toluol versetzt, 16 Stunden bei -20° C gehalten und bei -23° C mit 56 ml 10%iger wässriger L-Weinsäure-Lösung tropfenweise behandelt. Nach weiteren 30 Minuten wird das Gemisch auf +20° C erwärmen gelassen und noch so lange weiter gerührt, bis sich die organische Schicht klar abtrennen lässt. Diese wird mit 1 l 1%iger wässriger Natriumsulfit-Lösung 1 Stunde gerührt, abgetrennt, mit Wasser gewaschen über Natriumsulfat getrocknet und im Wasserstrahlvakuum eingeengt. Der Rückstand wird in 1.6 l Diethylether gelöst, auf 0° C gekühlt, tropfenweise mit 675 ml N-Natronlauge versetzt und 30 Minuten bei 0° C gerührt. Die abgetrennte organische Phase wird mit gesättigter Natriumchloridlösung gewaschen, getrocknet und eingeengt, womit 2(R),3(R)-2,3-Epoxyheptanol als farblose instabile Flüssigkeit resultiert, welche in der nächsten Stufe sofort verarbeitet wird.

Das 2(S),3(S)-epimere 2,3-Epoxyheptanol kann auch folgendermassen erhalten werden:

Eine Lösung von 2.28 g (20 mMol) 2-trans-Heptenol in 10 ml Methylenchlorid wird bei -20° C zu einer Lösung von 5.94 ml Tetraisopropylorthotitanat und 4.12 g L-(+)-Weinsäure-diethylester in 210 ml Methylenchlorid zugegeben, gefolgt von 9.75 ml einer 4.1 M-Lösung von tert-Butylhydroperoxid in 1,2-Dichlorethan. Das resultierende Reaktionsgemisch wird bei -20° C über Nacht stehen gelassen. Nach Zugabe von 8 ml Dimethylsulfid wird bei -20° bis -23° C 45 Minuten gerührt, danach mit 50 ml einer 10%-igen wässrigen Lösung von L-(+)-Weinsäure versetzt und 30 Min. bei -20° C und 60 Min. ohne Kühlung weitergerührt. Die organische Phase wird abgetrennt, mit 100 ml Wasser nachgewaschen und nach Trocknen über Magnesiumsulfat unter vermindertem Druck eingedampft. Der Rückstand, gelöst in 150 ml Ether, wird bei 0° C mit

60 ml 1N-NaOH 30 Minuten gerührt, die wässrige Phase abgetrennt, mit Ether nachextrahiert, und die vereinigten organischen Extrakte werden mit Kochsalzlösung geschüttelt. Nach Trocknen des organischen Anteils über Magnesiumsulfat und Abdestillieren des Lösungsmittels in Vakuum erhält man 2.3 g 2(S),3(S)-2,3-Epoxyheptanol als farbloses, instabiles Oel. Es wird gleich weiterverarbeitet.

c) 2(S),3(R)-2,3-Epoxyheptanal

Eine Lösung von 13.3 g 2(R),3(R)-2,3-Epoxyheptanol in 100 ml Methylenchlorid wird innert 30 Minuten zu einer gerührten Suspension von 110.1 g Pyridiniumchlorochromat und 41.9 g Natriumacetat in 500 ml Methylenchlorid getropft, wobei die Temperatur durch leichtes Kühlen bei 25° C gehalten wird. Nach 3 Stunden wird das Reaktionsgemisch mit 500 ml Diethylether verdünnt und über Kieselgel filtriert. Das Filtrat wird mit Phosphatpuffer vom pH 8 gewaschen, über Natriumsulfat getrocknet und eingedampft. Chromatographie des Rückstandes an Kieselgel mit einem Gemisch von Petrolether (Sdp. 30-45° ) und Diethylether (3:2) ergibt 2(S),3(R)-2,3-Epoxyheptanal als farblose Flüssigkeit; das Produkt weist analoge spektrale Eigenschaften wie sein 2(R),3(S)-Antipode (siehe nachfolgend).

Das 2(R),3(S)-Epimere kann auch folgendermassen erhalten werden:

Eine Lösung von 1.2 g 2(S),3(S)-2,3-Epoxyheptanol in 28 ml Methylenchlorid wird bei Raumtemperatur zu einer frisch hergestellten Lösung von 5.5 g Chromtrioxid und 8.76 g Pyridin in 70 ml Methylenchlorid zugegeben und das erhaltene Reaktionsgemisch 30 Minuten weitergerührt. Das dunkelfarbige Reaktionsgemisch wird vom ausgeschiedenen Material abdekantiert, das letztere mit 160 ml Methylenchlorid nachgewaschen, und die vereinigten organischen Anteile werden mit 80 ml Phosphatpuffer von pH 8.0 gewaschen. Nach Trocknen über Magnesiumsulfat und Eindampfen unter vermindertem Druck wird das zurückbleibende Rohprodukt auf 90 g Merck Kieselgel 60 mit Toluol/Ethylacetat (4:1) chromatographiert. Man erhält 464 mg 2(R),3(S)-2,3-Epoxyheptanal als farbloses Oel. $[\alpha]_D^{20}$ = $+101^0 \pm 1^0$ (1.225 % in $CHCl_3$); IR($CH_2Cl_2$): 2950, 2925, 2860, 2815, 2730, 1722, 1462, 1432, 1380, 1360, 1230, 1156, 850 $cm^{-1}$.]

d) 5(S),6(S)-5,6-Epoxy-7-cis-pentadecen

Eine Lösung von 370 mg Octyl-triphenylphosphoniumbromid in 4.2 ml Tetrahydrofuran und 1.26 ml Hexamethylphosphorsäuretriamid wird bei -78° C unter Argon mit 0.31 ml einer 20%-Lösung von Butyllithium in Hexan tropfenweise versetzt und die resultierende Lösung bei -78° C weitere 30 Min. gerührt. Zu der erhaltenen Lösung von Triphenylphosphoranyliden-octen wird bei -78° C eine Lösung von 110 mg 2(S),3-(R)-2,3-Epoxyheptanal in 1.0 ml Tetrahydronfuran zugetropft und das entstandene Reaktionsgemisch 30 Min. bei -78° C weitergerührt. Zur Aufarbeitung wird das Reaktonsgemisch zwischen 100 ml Ether und 30 ml Phosphatpuffer von pH 8.0 verteilt, und beide Phasen werden noch mit Ether, bzw. Pufferlösung, nachextrahiert. Die vereinigten etherischen Anteile werden über Magnesiumsulfat getrocknet und im Vakuum eingedampft, wodurch man 421 mg öliges Rohprodukt bekommt. Dieses wird mit 2-3 ml Hexan-Ether-Gemisch 3:1 verrührt, das kristallin ausgeschiedene Triphenylphosphinoxid abgetrennt und das Filtrat eingedampft. Der erhaltene Rückstand (195 mg) wird an einer Aluminiumoxid-Säule (10 g), präpariert in Hexan mit 0.5 % Triethylamin, mit demselben Eluens chromatographiert. Man erhält 90.6 mg der Titelverbindung als dickflüssiges, gelbliches Oel

Das epimere 5(R),6(R)-5,6-Epoxy-7-cis-pentadecen wird in analoger Weise aus dem epimeren 2(R),3-(S)-2,3-Epoxyheptanol erhalten.

Beispiel 1A : 3-[5(R),6(S)-5-Hydroxy-7-cis-eicosen-6-yl-thio]-malonanilinsäure-methylester.

In analoger Weise wie im Beispiel 1, aber ausgehend von 5(R),6(R)-5,6-Epoxy-7-cis-eicosen und 3-Mercapto-malonanilinsäure-methylester erhält man die Titelverbindung in öliger Form.

Das als Ausgangsstoff verwendete 5(R),6(R)-5,6-Epoxy-7-cis-eicosen kann analog der im Beispiel 1d beschriebenen Methode durch die Umsetzung von 2(R),3(S)-2,3-Epoxyheptanal mit Triphenylphosphoranylidentridecen (hergestellt aus Tridecyl-triphenylphosphoniumbromid mit Butyllithium) erhalten werden.

Beispiel 1B: 3-[5(S),6(R)-5-Hydroxy-7-cis-eicosen-6-yl-thio]-malonanilinsäure-methylester

In analoger Weise wie im Beispiel 1, aber ausgehend von 5(S),6(S)-Epoxy-7-cis-eicosen (hergestellt analog Beispiel 1A unter Verwendung von 2(S),3(R)-2,3-Epoxyheptanal) und 3-Mercaptomalonanilinsäure-methylester erhält man die Titelverbindung; IR ($CH_2Cl_2$): 2930, 2850, 1725, 1690, 1590, 1535, 1350 $cm^{-1}$.

Beispiel 2: 7-[5(S),6(R)-5-Hydroxy-7-cis-pentadecen-6-yl-thio]-4-oxo-4H-chromen-2-carbonsäure-methylester und dessen 5(R),6(S)-Stereoisomeres.

In analoger Weise wie im Beispiel 1, ausgehend von 5(S),6(S)- oder 5(R),6(R)-5,6-Epoxy-7-cis-pentadecen und 7-Mercapto-4-oxo-4H-chromen-2-carbonsäure-methylester (EP-OL 0123 543) erhält man die Titelverbindung $[\alpha]_D^{20}$ = -9,5 ± 1,6° (c = 0,63 % in Chloroform), bzw. ihr 5(R),6(S)-Stereoisomeres.

Beispiel 2A : 7-[5(R),6(S)-5-Hydroxy-7-cis-eicosen-6-yl-thio]-4-oxo-4H-chromen-2-carbonsäure-methylester.

In analoger Weise wie im Beispiel 1, aber ausgehend von 5(R),6(R)-5,6-Epoxy-7-cis-eicosen (siehe Beispiel 1A) und 7-Mercapto-4-oxo-4H-chromen-2-carbonsäure-methylester erhält man die Titelverbindung als gelbliches Oel.

Beispiel 2B : 7-[5(S),6(R)-5-Hydroxy-7-cis-eicosen-6-yl-thio]-4-oxo-4H-chromen-2-carbonsäure-methylester

In analoger Weise wie im Beispiel 1, aber ausgehend von 5(S),6(S)-5,6-Epoxy-7-cis-eicosen (hergestellt analog Beispiel 1A unter Verwendung von 2(S),3(R)-2,3-Epoxyheptanal) und 7-Mercapto-4-oxo-4H-chromen-2-carbonsäure-methylester, erhält man die Titelverbindung; IR ($CH_2Cl_2$): 2930, 2860, 1750, 1660, 1600, 1415 $cm^{-1}$.

Beispiel 3: 7-[5(S)6(R)-5-Hydroxy-7-trans,9-cis-eicosadien-6-yl-thio]-4-oxo-4H-chromen-2-carbonsäure Methylester

In analoger Weise wie im Beispiel 1, ausgehend von 5(S),6(S)-5,6-Epoxy-7-trans,9-cis-eicosadien und 7-Mercapto-4-oxo-4H-chromen-2-carbonsäure-methylester erhält man die Titelverbindung
$[\alpha]_D^{20}$ = -97,4 ± 0.9° (c = 1,1 % in Chloroform).

Das als Ausgangsstoff verwendete 5(S),6(S)-5,6-Epoxy-7-trans,9-cis-eicosadien wird beispielsweise folgendermassen hergestellt:

a) 4(S),5(S)-4,5-Epoxy-2-trans-nonenal

Die Lösung von 10 g 2(R),3(S)-Epoxyheptanal (siehe Beispiel 1c) und 23.7 g Formylmethylentriphenylphosphoran in 350 ml Chloroform wird unter Argon 1,5 Std. rückfliessend erhitzt. Die erkaltete Lösung wird im Vakuum bei Raumtemperatur vom Lösungsmittel befreit und der Rückstand mit Ether/Hexan (4:1) verrührt. Die Suspension wird über wenig Kieselgel filtriert und mit Ether/Hexan (4:1) gewaschen. Das Filtrat wird im Vakuum eingeengt und der Rückstand mit Hexan/Ethylacetat (5:1, mit 1 % Triethylamin) an Kieselgel chromatographiert. Man erhält die Titelverbindung als farbloses Oel.

b) 5(S),6(S)-5,6-Epoxy-7-trans,9-cis-eicosadien

In analoger Weise wie im Beispiel 1d wird die Titelverbindung durch Umsetzen von 4(S),5(S)-4,5-Epoxy-2-trans-nonenal mit Undecyl-triphenylphosponiumbromid erhalten.

Beispiel 3A : 3-[5(S)6(R)-5-Hydroxy-7-trans,9-cis-eicosadien-6-yl-thio]-malonanilinsäuremethylester.

In analoger Weise wie im Beispiel 1, aber ausgehend von 5(S),6(S)-5,6-Epoxy-7-trans,9-cis-eicosadien (siehe Beispiel 3b) und 3-Mercapto-malonanilinsäure-methylester erhält man die Titelverbindung.

Beispiel 4: 7-[5(R),6(S)-5-Hydroxy-7,9-trans-11-cis-eicosatrien-6-yl-thio]-4-oxo-4H-chromen-2-carbonsäure-methylester.

In analoger Weise wie im Beispiel 1, ausgehend von 5(R),6(R)-5,6-Epoxy-7,9-trans-11-cis-eicosatrien und 7-Mercapto-4-oxo-4H-chromen-2-carbonsäure-methylester erhält man die Titelverbindung; $[\alpha]_D^{20}$ = + 198,9 ± 2,7° (c = 0,38 % in $CHCl_3$), UV ($CHCl_3$): $\lambda_{max}$ ($\epsilon$) = 268 (34100); 322 (11900) nm.

Das als Ausgangsstoff verwendete 5(R),6(R)-5,6-Epoxy-7,9-trans-11-cis-eicosatrien wird beispielsweise folgendermassen hergestellt:

a) 6(R),7(R)-6,7-Epoxy-2,4-trans-undecadienal

Eine Lösung von 6,7 g 2(S),3(R)-2,3-Epoxyheptanal (siehe Beispiel 1c) in 250 ml Methylenchlorid wird bei 20° C während 1 Stunde mit einer Lösung von 20.85 g λ-Triphenylphosphoranylidencrotonaldehyd in 200 ml Methylenchlorid tropfenweise versetzt und eine weitere Stunde bei 20° C gerührt. Das Reaktionsgemisch wird mit 240 ml Hexan und 120 ml Ethylacetat verdünnt, über Kieselgel filtriert und eingeengt. Der Rückstand wird in gleiche Volummengen Hexan und Ethylacetat aufgenommen, 15 Minuten gerührt, erneut durch Kieselgel filtriert und eingeengt. Das resultierende ölige Gemisch von cis,trans- und trans,trans-Isomeren wird zwecks Isomerisierung in 200 ml Methanol gelöst, mit 220 mg Jod versetzt und bei 20° C 3 Stunden stehen gelassen. Nach Waschen mit einer wässrigen Natriumthiosulfatlösung und Wasser und Trocknen über Natriumsulfat wird die Lösung eingeengt und der Rückstand an Kieselgel chromatographiert. Eluieren mit Hexan-Ethylacetat (4:1) ergibt das gewünschte 6(R),7(R)-6,7-Epoxy-2,4-trans-undecadienal als gelbliches Oel,
$[\alpha]_D^{20}$ = -21.1 ± 1.3° (0.75 G/V-% in Chloroform)
$\lambda_{max}$=276 nm; $\epsilon$=29900; IR($CH_2Cl_2$): 2950, 2920, 2850, 2800, 2720, 1678, 1640, 1600, 1460, 1163, 1120, 1007, 985 $cm^{-1}$.

b) 5(R),6(R)-5,6-Epoxy-7,9-trans-11-cis-eicosatrien

Zu einer auf -78° C abgekühlten, gerührten Lösung von 5.15 g Nonyltriphenylphosphoniumbromid in 50 ml Tetrahydrofuran wird unter Argon 6.85 ml einer 1.6M-Lösung von Butyllithium in Toluol zugesetzt. Nach 30 Minuten bei -78° C wird das Gemisch nacheinander durch tropfenweise Zugabe von 15.1 g Hexamethylphosphorsäuretriamid und einer Lösung von 1.52 g (6(R),7(R)-6,7-Epoxy-2,4-trans-undecadienal in 10 ml Tetrahydrofuran behandelt, weitere 15 Minuten bei -78° C gehalten und auf 0° C erwärmen gelassen. Das Reaktionsgemisch wird mit Phosphatpuffer (pH 8) versetzt und mit Ether extrahiert. Die vereinigten Ether-Auszüge werden mit einigen Tropfen Triethylamin stabilisiert, über Natriumsulfat getrocknet und bei 20° C im Vakuum von leicht-flüchtigen Anteilen befreit. Der Rückstand wird mit kleinen Mengen Ether verrührt und vom ausgeschiedenen festen Triphenylphosphinoxid durch Filtration befreit. Aus dem Filtrat entfernt man die letzten Anteile Triphenylphosphinoxids durch Filtration über eine Kieselgelsäule, die durch Auswaschen mit einem Gemisch (4:1) von Ether-Hexan mit einer 2%igen Beimischung von Triethylamin vorpräpariert wurde. Nach Abdestillieren der Lösungsmittel aus dem Filtrat resultiert das gewünschte Produkt in Form von leicht gelben Kristallen, Smp. 31-32° C.

Beispiel 5: 3-[5(S),6(R)-5-Hydroxy-7-cis-pentadecen-6-yl-thio]-malonanilinsäure

1,2 g des Methylesters der Titelverbindung (siehe Beispiel 1) werden in 30 ml Methanol gelöst und mit 27 ml 0,1-N Natronlauge vesetzt. Das Reaktionsgemisch wird 40 Stunden bei Raumtemperatur gerührt und im Vakuum eingedampft. Der Rückstand wird mit Dichlormethan/Methanol (9:1) über Kieselgel filtriert, das Eluat wird im Vakuum vom Laufmittel befreit, der Rückstand in Wasser gelöst und mit 1-N Salzsäure angesäuert. Die wässrige Phase wird mit Ether extrahiert, der Extrakt über Magnesiumsulfat getrocknet, filtriert und eingedampft. Man erhält die Titelverbindung als weisses Harz.
Drehwert des Natriumsalzes:
$[\alpha]_D^{20}$ = +17,2 ± 1,6° (c = 0,65 %; Methanol)

Beispiel 5A : 3-[5(S)6(R)-5-Hydroxy-7-trans,9-cis-eicosadien-6-yl-thio]-malonanilinsäure.

In analoger Weise wie im Beispiel 5 erhält man die Titelverbindung aus dem entsprechenden Methyleter (siehe Beispiel 3A);
$[\alpha]_D^{20}$ = -24.2 ± 0.9° (c = 1.16 %; Methanol);
UV (Methanol): $\lambda_{max}$ ($\epsilon$) = 210 (sh); 240 (37240) nm.

Beispiel 6: 7-[5(S),6(R)-5-Hydroxy-7-cis-pentadecen-6-yl-thio]-4-oxo-4H-chromen-2-carbonsäure

In analoger Weise wie im Beispiel 5 erhält man die Titelverbindung ausgehend vom entsprechenden Methylester $[\alpha]_D^{20}$ = +14,1 ± 1,5° (c = 0,68 %, Methanol).

Beispiel 7: 7-[5(R),6(S)-5-Hydroxy-7,9-trans-11-cis-eicosatrien-6-yl-thio]-4-oxo-4H-chromen-2-carbonsäure-natriumsalz

1,1 g des Methylesters der Titelverbindung werden in 30 ml Methanol gelöst und mit 21 ml 0,1-N NaOH versetzt. Man rührt 10 Stunden bei Raumtemperatur, engt die Lösung im Vakuum ein und chromatographiert mit Methanol/Wasser (3:1) über eine Reversed-Phase-Säule (Merck Fertigsäule RP-8). Man erhält die Titelverbindung als weisses Harz.
$[\alpha]_D^{20}$ = +145,8 ± 2,6° (c = 0,38 %; Methanol). UV (Methanol): $\lambda_{max}$ ($\epsilon$) = 206 (23500); 225 (23360); 267 (34340); 285 (sh); 324 (10040) nm.

Beispiel 7A : 3-[5(R),6(S)-5-Hydroxy-7-cis-pentadecen-6-yl-thio]-malonanilinsäure-natriumsalz

In analoger Weise wie im Beispiel 7 erhält man die Titelverbindung aus dem entsprechenden Methylester (siehe Beispiel 1);
$[\alpha]_D^{20}$ = +13.8 ± 2.8° (c = 0.36 %; Methanol). UV (Methanol): $\lambda_{max}$ ($\epsilon$) = 223 (sh); 246 (18200) nm.

Beispiel 7B : 7-[5(R),6(S)-5-Hydroxy-7-cis-pentadecen-6-yl-thio]-4-oxo-4H-chromen-2-carbonsäure-natriumsalz

In analoger Weise wie im Beispiel 7 erhält man die Titelverbindung aus dem entsprechenden Methylester (siehe Beispiel 2);
Smp. 246-249°; $[\alpha]_D^{20}$ = 1.1 ± 3.7° (c = 0,27 %; Methanol). UV (Methanol) $\lambda_{max}$ ($\epsilon$) = 206 (22200); 223 (19800); 260 (sh); 267 (14100); 324 (11000) nm.

Beispiel 7C : 7-[5(R),6(S)-5-Hydroxy-7-cis-eicosen-6-yl-thio]-4-oxo-4H-chromen-2-carbonsäure-natriumsalz

In analoger Weise wie im Beispiel 7 erhält man die Titelverbindung aus dem entsprechenden Methylester (siehe Beispiel 2A);
$[\alpha]_D^{20}$ = 11.3 ± 3.8° (c = 0,27 %; Methanol).

Beispiel 7D : 3-[5(R),6(S)-5-Hydroxy-7-cis-eicosen-6-yl-thio]-malonanilinsäure-natriumsalz

In analoger Weise wie im Beispiel 7 erhält man die Titelverbindung aus dem entsprechenden Methylester (siehe Beispiel 1A);
$[\alpha]_D^{20}$ = -15.5 ± 2.3° (c = 0.43 %; Methanol); UV (Methanol): $\lambda_{max}$ ($\epsilon$) = 223 (sh); 246 (18100) nm.

Beispiel 7E : 7-[5(S),6(R)-5-Hydroxy-7-cis-eicosen-6-yl-thio]-4-oxo-4H-chromen-2-carbonsäure-natriumsalz

In analoger Weise wie im Beispiel 7 erhält man die Titelverbindung aus dem entsprechenden Methylester (siehe Beispiel 2B);
UV (Methanol): $\lambda_{max}$ ($\epsilon$) = 324 (10560), 266 (13320), 223 (18640).

Beispiel 7F : 3-[5(S),6(R)-5-Hydroxy-7-cis- eicosen-6-yl-thio]-malonanilinsäure-natriumsalz

In analoger Weise wie im Beispiel 7 erhält man die Titelverbindung aus dem entsprechenden Methylester (siehe Beispiel (1B); $[\alpha]_D^{20}$ = +23.9 ± 2.8° (0.36 % in Methanol).

Beispiel 8: 7-[5(S),6(R)-5-Hydroxy-7-trans,9-cis-eicosadien-6-yl-thio]-4-oxo-4H-chromen-2-carbonsäure-natriumsalz.

In analoger Weise wie im Beispiel 7 erhält man die Titelverbindung aus dem entsprechenden Methylester. $[\alpha]_D^{20}$ = -91,6 ± 1° (0,8 % in Methanol)

Beispiel 9: 7-[4(R),5(S)-1,1,1-Trifluor-4-hydroxy-6,10-cis,89-transnonadecatrien-5-yl-thio]-4-oxo-4H-chromen-2-carbonsäure-methylester.

Eine Lösung von 0,67 g 4(R),5(R)-4,4-Epoxy-1,1,1-trifluor-6,10-cis,8-trans-nonadecatrien in 10 ml Methanol wird unter Rühren und unter Argon mit 0,74 g Triethylamin und dann mit 0,58 g 7-Mercapto-4-oxo-4H-chromen-2-carbonsäure-methylester versetzt. Die braunrote Lösung wird 16 Std. bei 20° gerührt und eingedampft. Der Rückstand wird an Kieselgel mit Hexan/Ethylacetat (7:3) chromatographiert und ergibt die Titelverbindung in Form von gelben Kristallen.
IR ($CH_2Cl_2$): 3020, 2970, 2940, 2860, 1750, 1670, 1610, 1420, 1150 $cm^{-1}$.

Das als Ausgangsstoff verwendete 4(R),5(R) 4,5-Epoxy-1,1,1-trifluor-6,10-cis,8-trans-nonadecatrien wird beispielsweise folgendermassen hergestellt:

a) 6,6,6-Trifluor-2-trans-hexensäureethylester

In Analogie zu J. Am. Chem. Soc. 104 , 3527-29 (1982) werden in einem 0,3 lt Autoklaven 3,42 g (10 mMol) $Co_2(CO)_8$ in 50 ml Mesitylen bei 0° vorgelegt und 0,5 Mol 3,3,3-Trifluorpropen eingefüllt, gefolgt bei Raumtemperatur nacheinander mit 65 bar Kohlenstoffmonoxid und 65 bar Wasserstoff. Das Reaktionsgemisch wird auf 110° C aufgeheizt und durch Zugabe eines (1:1)-Gemisches (V/V) von CO und $H_2$ wird der Druck bei 130 bar konstant gehalten. Nach Aufnahme der theor. Menge des $CO/H_2$-Gemisches (1-5 Stdn.) wird auf 0° abgekühlt und der Autoklav entlastet. Das mit wenig Mesitylen ausgespülte Rohgemisch wird bei Raumtemperatur langsam mit einer lösung von 156,8 g (0,45 Mol) Ethoxycarbonylmethylentriphenylphosphoran in 600 ml Methylenchlorid versetzt. Nach Abklingen der leicht exothermen Reaktion wird über 2 1/2 Stunden bei Raumtemperatur gerührt und anschliessend eingedampft. Der Rückstand wird mit Pentan (ca. 100 ml) aufgeschlämmt, vom ausfallenden Triphenylphosphinoxid getrennt und bei vermindertem Druck destilliert. Der gewünschte Ester resultiert als farbloses Oel (Sdp. 78-82° /26 mm Hg) in 50 % Ausbeute.

b) 6,6,6-Trifluor-2-trans-hexenol

Eine Lösung von 10 g 6,6,6-Trifluor-2-trans-hexensäureethylester (siehe oben) in 70 ml Diethylether wird auf 0-5° gekühlt und bei dieser Temperatur mit 102 ml 1-M Diisobutylaluminumhydrid-Lösung in Hexan versetzt. Das Reaktionsgemisch wird noch 5 Min. bei 0-5° gerührt und vorsichtig mit ca. 200 ml 6-N Salzsäure hydrolysiert. Die organische Phase wird abgetrennt und die wässrige Phase noch 3mal mit Ether extrahiert. Die vereinigten organischen Phasen werden über $MgSO_4$ getrocknet und eingedampft. Chromatrographie des Rückstandes an Kieselgel mit Hexan/Ethylacetat (7:3) liefert die Titelverbindung als farblose Flüssigkeit.

c) 2(R),3(R)-2,3-Epoxy-6,6,6-trifluorhexanol

Unter absolut wasserfreien Bedingungen wird die Lösung von 6,38 ml Tetraisopropyl-orthotitanat und 4,5 ml D-(-)-Weinsäurediethylester in 80 ml $CH_2Cl_2$ auf -70° gekühlt. Bei dieser Temperatur gibt man 6,3 g

6,6,6-Trifluor-2-trans-hexenol (siehe oben) und 30,15 ml 2,74-M tert-Butylhydro-peroxid-Lösung in Toluol zu. Die Temperatur lässt man innert 2 Stunden auf 0° steigen und gibt eine Lösung von 27 g Eisen(II)sulfat und 11 g Weinsäure in 110 ml Wasser zu. Nach 30 Minuten Rühren bei 10° wird die organische Phase abgetrennt, die wässrige zweimal mit Ether extrahiert, und die vereinigten organischen Phasen werden über $Na_2SO_4$ getrocknet und eingedampft. Der Rückstand wird in 120 ml Ether aufgenommen, auf 0-5° gekühlt und mit einer Suspension von 4,2 g NaOH in 110 ml gesättigter NaCl-Lösung versetzt. Das Gemisch wird 1 Stunde bei 0-5° gerührt, die organische Phase abgetrennt und die wässrige dreimal mit Ether extrahiert. Die vereinigten organischen Phasen werden über $Na_2SO_4$ getrocknet und eingedampft. Die erhaltene Titelverbindung kann ohne weitere Reinigung weiterverarbeitet werden.

d) 2(S),3(R)-2,3-Epoxy-6,6,6-trifluorhexanal

Eine Lösung von 3,8 ml Oxalylchlorid in 40 ml $CH_2Cl_2$ werden auf -70° gekühlt und mit 7 ml Dimethylsulfoxid in 15 ml $CH_2Cl_2$ tropfenweise versetzt, wobei die Temperatur nicht über -60° steigt. Nach 10 Minuten Rühren bei -70° werden 6,95 g 2(R),3(R)-2,3-Epoxy-6,6,6-trifluorhexanol (hergestellt nach c) in 40 ml $CH_2Cl_2$ innert 15 Minuten zugetropft. Nach weiteren 30 Minuten bei -70° werden 28,7 ml Triethylamin tropfenweise zugegeben und die Temperatur auf 0° steigen gelassen. Das Reaktionsgemisch wird auf Phosphatpuffer (pH 8) gegossen, die wässrige Phase zweimal mit $CH_2Cl_2$ extrahiert, und die vereinigten organischen Phasen werden mit Wasser gewaschen, über $Na_2SO_4$ getrocknet und eingedampft. Die erhaltene Titelverbindung wird direkt weiter verarbeitet.

e) 6(R),7(R)-6,7-Epoxy-10,10,10-trifluor-2-trans,4-trans-decadienal und 6(R),7(R)-6,7-Epoxy-10,10,10-trifluor-2-trans,4-cis-decadienal

Eine Lösung von 6,87 g 2(S),3(R)-2,3-Epoxy-6,6,6-trifluorhexanal in 100 ml $CH_2Cl_2$ wird innert 60 Minuten mit einer Lösung von 14,87 g 4-Triphenylphosphoranylidencrotonaldehyd in 150 ml $CH_2Cl_2$ tropfenweise versetzt. Das Reaktionsgemisch wird noch 1 Stunde bei 20° gerührt und eingedampft. Der Rückstand wird in Hexan/Ethylacetat (1:1) aufgenommen und über Kieselgel filtriert. Das Filtrat wird eingedampft und der Rückstand an Kieselgel mit Hexan7Ethylacetat (4:1) chromatographiert. Eindampfen der ersteren Fraktionen ergibt das 2-trans,4-cis-Isomere der Titelverbindung. Die späteren Fraktionen enthalten das 2-trans,4-trans-Isomere der Titelverbindung. Beide Produkte sind gelbbraune Oele.

f) 4(R),5(R)-4,5-Epoxy-1,1,1-trifluor-6-cis,8-trans,10-cis-nonadecatrien

Eine gerührte Lösung von 1,25 g Nonyltriphenylphosphoniumbromid in 20 ml Tetrahydrofuran wird auf -78° gekühlt und unter Argon mit 1,66 ml einer 1,6-M Butyllithiumlösung in Hexan versetzt. Nach 30 Min. bei -78° tropft man 4 g Hexamethylphosphorsäuretriamid und anschliessend eine Lösung von 0,45 g 6(R)-,7(R) 6,7-Epoxy-10,10,10-trifluor-2-trans,4-cis-decadienal in einer kleinen Menge Tetrahydrofuran zu. Man rührt noch 15 Mintuen bei -78°, lässt die Temperatur auf 0° steigen, gibt Phosphatpuffer (pH 8) zu und extrahiert mit Ether. Die vereinigten organischen Phasen werden 3mal mit Phosphatpuffer (pH 8) gewaschen, über $Na_2SO_4$ getrocknet und eingedampft. Der Rückstand wird in einer minimalen Menge Ether aufgechwämmt, vom ausgefallenen Triphenylphosphinoxid durch Filtration befreit und erneut eingedampft. Die erhaltene Titelverbindung wird direkt im Haupverfahren weiterverarbeitet.

Beispiel 10: 7-[4(R),5(S)-1,1,1-Trifluor-4-hydroxy-6,8-trans,10-cis-nonadecatrien-5-yl-thio]-4-oxo-4H-chromen-2-carbonsäure-methylester

In analoger Weise wie im Beispiel 9 erhält man die Titelverbindung aus 4(R),5(R)-4,5-Epoxy-1,1,1-trifluor-6,8-trans,10-cis-nonadecatrien und 7-Mercapto-4-oxo-4H-chromen-2-carbonsäure-methylester IR ($CH_2Cl_2$): 3020, 2970, 2940, 2860, 1750, 1665, 1610, 1420, 1390, 1150 $cm^{-1}$.

Das als Ausgangsstoff verwendete 4(R),5(R)-4,5-Epoxy-1,1,1-trifluor-6,8-trans,10-cis-nonadecatrien wird beispielsweise folgendermassen hergestellt:

a) Das im Beispiel 9e erhaltene 6(R),7(R)-6,7-Epoxy-10,10,10-trifluor-2-trans,4-cis-decadienal wird in identischer Weise wie im Beispiel 9f umgesetzt und zum 4(R),5(R)-4,5-Epoxy-1,1,1-trifluor-6,8-trans,10-cis-nonadecatrien verarbeitet.

Beispiel 11: 7-[4(S),5(R)-1,1,1-Trifluor-4-hydroxy-6,10-cis,8-trans-nonadecatrien-5-yl-thio]-4-oxo-4H-chromen-2-carbonsäure-methylester.

In analoger Weise wie im Beispiel 9 erhält man die Titelverbindung aus 4(S),5(S)-4,5-Epoxy-1,1,1-trifluor-6,10-cis,8-trans-nonadecatrien und 7-Mercapto-4-oxo-4H-chromen-2-carbonsäure-methylester.

Das als Ausgangsstoff verwendete 4(S),5(S)-4,5-Epoxy-1,1,1-trifluor-6,10-cis,8-trans-nonadecatrien wird beispielsweise folgender massen hergestellt:

a) 2(S),3(S)-2,3-Epoxy-6,6,6-trifluorhexanol

In analoger Weise wie im Beispiel 9c, aber unter Einwirkung von D-(+)-Weinsäurediethylester erhält man die Titelverbindung als farbloses Oel.

b) 2(R),3(S)-2,3-Epoxy-6,6,6-trifluorhexanal

In analoger Weise wie im Beispiel 9d wird das 2(S),3(S)-2,3-Epoxy-6,6,6-trifluorhexanol aus der vorangehenden Stufe zur Titelverbindung verarbeitet.

c) 6(S),7(S)-6,7-Epoxy-10,10,10-trifluor-2-trans,4-trans-decadienal und 6(S),7(S)-6,7-Epoxy-10,10,10-trifluor-2-trans,4-cis-decadienal

In analoger Weise wie im Beispiel 9e, wird das 2(R),3(S)-2,3-Epoxy-6,6,6-trifluorhexanal aus der Stufe 11b zum Isomerengemisch verarbeitet, welches durch Chromatographie in das 2-trans,4-cis- und das 2-trans,4-trans-Isomere aufgetrennt werden.

d) 4(S),5(S)-4,5-Epoxy-1,1,1-trifluor-6,10-cis,8-trans-nonadecatrien

In analoger Weise wie im Beispiel 9f erhält man die Titelverbindung durch Umsetzen von 6(S),7(S)-6,7-Epoxy-10,10,10-trifluor-2-trans,4-cis-decadienal.

Beispiel 12: 7-[4(S),5(R)-1,1,1-Trifluor-4-hydroxy-6,8-trans,10-cis-nonadecatrien-5-yl-thio]-4-oxo-4H-chromen-2-carbonsäure-methylester

In analoger Weise wie im Beispiel 9 erhält man die Titelverbindung aus 7-Mercapto-4-oxo-4H-chromen-2-carbonsäure-methylester und 4(S),5(S)-4,5-Epoxy-1,1,1-trifluor-6,8-trans,10-cis-nonadecatrien, welches durch die Umsetzung analog Beispiel 9f aus 6(S),7(S)-6,7-Epoxy-10,10,10-trifluor-2-trans,4-trans-decadienal (siehe Beispiel 11c) hergestellt werden kann.

Beispiel 12A : 3-[4(R),5(S)-1,1,1-Trifluor-4-hydroxy-6-cis-eicosen-5-yl-thio]-malonanilinsäure-methylester

In analoger Weise wie im Beispiel 9 erhält man die Titelverbindung aus 3-Mercapto-malonanilinsäure-methylester und 4(R),5(R)-4,5-Epoxy-1,1,1-trifluor-6-cis-eicosen, welches durch Umsetzung von 2(S),3(R)-2,3-Epoxy-6,6,6-trifluorhexanol mit Tetradecylphosphoniumbromid analog Beispiel 9f erhalten wird.

Beispiel 12B : [7-4(R),5(S)-1,1,1-Trifluor-4-hydroxy-6-cis-eicosen-5-yl-thio]-4-oxo-4H-chromen-2-carbonsäure-methylester

In analoger Weise wie im Beispiel 9 erhält man die Titelverbindung aus 7-Mercapto-4-oxo-4H-chromen-2-carbonsäure-methylester und 2(S),3(R)-2,3-Epoxy-6,6,6-trifluor-hexanol.

Beispiel 12C : 7-[4(R),5(S)-1,1,1-Trifluor-4-hydroxy-6-cis-eicosen-5-yl-thio]-2-methoxychinolin-3-carbonsäure-methylester

In analoger Weise wie im Beispiel 9 erhält man die Titelverbindung aus 7-Mercapto-2-methoxychinolin-3-carbonsäure-methylester und 2(S),3(R)-2,3-Epoxy-6,6,6-trifluor-hexanol.

Beispiel 13: 7-[4(R),5(S)-1,1,1-Trifluor-4-hydroxy-6,10-cis,8-trans-nonadecatrien-5-yl-thio]-4-oxo-4H-chromen-2-carbonsäure

0,35 g des entsprechenden Methylesters (siehe Beispiel 9) werden in 40 ml Methanol gelöst, auf 0° gekühlt und mit 8 ml 0,4 N NaOH langsam versetzt. Nach beendeter Zugabe lässt man auf Raumtemperatur erwärmen und rührt noch 45 Minuten. Das Methanol wird bei Raumtemperatur abgedampft, der Rückstand mit Wasser und $CH_2Cl_2$ versetzt und mit kalter 2-N Salzsäure angesäuert, die abgetrennte wässrige Phase zweimal mit $CH_2Cl_2$ nachextrahiert und die vereinigten organischen Phasen werden über $MgSO_4$ getrocknet und eingedampft. Die Titelverbindung resultiert als braungelbe zähe Masse.
IR ($CH_2Cl_2$); 3040, 2950, 2870, 1750, 1670, 1640, 1610, 1425, 1150 $cm^{-1}$.

Beispiel 14: 7-[4(R),5(S)-1,1,1-Trifluor-4-hydroxy-6,8-trans,10-cis-nonadecatrien-5-yl-thio]-4-oxo-4H-chromen-2-carbonsäure

In analoger Weise wie im Beispiel 13 erhält man die Titelverbindung aus dem entsprechenden Methylester (siehe Beispiel 10).

Beispiel 15: 7-[4(S),5(R)-1,1,1-Trifluor-4-hydroxy-6,10-cis,8-transnonadecatrien-5-yl-thio]-4-oxo-4H-chromen-2-carbonsäure

In analoger Weise wie im Beispiel 13 erhält man die Titelverbindung aus dem entsprechenden Methylester (siehe Beispiel 11).

Beispiel 16: 7-[4(S),5(R)-1,1,1-Trifluor-4-hydroxy-6,8-trans,10-cis-nonadecatrien-5-yl-thio]-4-oxo-4H-chromen-2-carbonsäure

In analoger Weise wie im Beispiel 13 erhält man die Titelverbindung aus dem entsprechenden Methylester (siehe Beispiel 12).

Beispiel 16A : 3-[4(R),5(S)-1,1,1-Trifluor-4-hydroxy-6-cis-eicosen-5-yl-thio]-malonanilinsäure-natriumsalz

In analoger Weise wie im Beispiel 7 erhält man die Titelverbindung aus dem entsprechenden Methylester (siehe Beispiel 12A);
$[\alpha]_D^{20}$ = -10.0 ±2.0° (0.5 %; Methanol);

Beispiel 16B : 7-[4(R),5(S)-1,1,1-Trifluor-4-hydroxy-6-cis-eicosen-5-yl-thio]-4-oxo-4H-chromen-2-carbonsäure-natriumsalz

In analoger Weise wie im Beispiel 7 erhält man die Titelverbindung aus dem entsprechenden Methylester (siehe Beispiel 12B);
Smp. 243-245°.

Beispiel 16C : 7-[4(R),5(S)-1,1,1-Trifluor-4-hydroxy-6-cis-eicosen-5-yl-thio]-2-methoxy-chinolin-3-carbonsäure-natriumsalz

In analoger Weise wie im Beispiel 7 erhält man die Titelverbindung aus dem entsprechenden Methylester (siehe Beispiel 12C);
UV (Methanol); $\lambda_{max}$ ($\epsilon$) = 332 (9300), 293 (7380), 226 (37960);
$[\alpha]_D^{20}$ = -21.0 ± 10.0° (0.1 % in Methanol);

Beispiel 17: 7-[1(RS),2(SR)-2-Hydroxy-1-(2-nonylphenyl)-hexyl-thio]-4-oxo-4H-chromen-2-carbonsäure-methylester.

Ein Gemisch von 2,42 g 1(RS),2(RS)-1,2-Epoxy-1-(2-nonylphenyl)-hexan, 1,77 g 7-Mercapto-4-oxo-4H-chromen-2-carbonsäure-methylester, 16 ml Triethylamin und 30 ml Methanol wird während 22 Stunden bei Raumtemperatur unter Argon gerührt. Anschliessend wird das Reaktionsgemisch unter vermindertem Druck bei Raumtemperatur eingedampft und der Rückstand durch Chromatographie an Kieselgel mit $CH_2Cl_2$/Aceton (98,5 : 1,5) gereinigt. Man erhält 7-[1(RS),2(SR)-2-Hydroxy--1-(2-nonylphenyl)-hexylthio]-4-oxo-4H-chromen-2-carbonsäure-methylester als blassgelben Festkörper vom Smp. 65-68° C. IR ($CH_2Cl_2$): 3580, 2930, 2860, 1745, 1655, 1600, 1415, 1240, 1140 $cm^{-1}$

Das als Ausgangsmaterial verwendete 1(RS),2(RS)-1,2-Epoxy-1-(2-nonylphenyl)-hexan kann folgendermassen erhalten werden:

a) 1-(2-Nonylphenyl)-1-hexanol

Ein unter Argonatmosphäre gerührtes Gemisch von 1,1 g Magnesiumspänen, 8 ml Tetrahydrofuran und 3 Tropfen Tetrachlorkohlenstoff wird mit einem Drittel einer Lösung von 11 g 2-Nonylbrombenzol [vgl. EP-OL 0 123 543] in 15 ml Tetrahydrofuran versetzt und während 30 Minuten unter Rückfluss zum Sieden erhitzt. Anschliessend wird die restliche Lösung von 2-Nonylbrombenzol während 35 Minuten zugetropft und das Reaktionsgemisch 2 Stunden am Rückfluss gehalten. Nach Verdünnen mit 15 ml Tetrahydrofuran wird die Suspension auf -10° C abgekühlt und portionenweise zu einer auf -70° C gekühlten Lösung von 4,6 g Hexanal in 12 ml Tetrahydrofuran gegeben. Nach 1-stündigem Rühren bei -70° C wird das Reaktionsgemisch mit 200 ml gesättigter wässriger Ammoniumchloridlösung versetzt, die organische Schicht abgetrennt und die wässrige dreimal mit Ether ausgezogen. Der nach dem Trocknen und Eindampfen der vereinigten Etherextrakte verbliebene Rückstand wird durch Chromatographie an Kieselgel mit Gemischen von Petrolether mit steigendem Anteil an Methylenchlorid gereinigt, womit das gewünschte 1-(2-Nonylphenyl)-1-hexanol als farbloses Oel erhalten wird. IR ($CH_2Cl_2$): 3600, 2960, 2925, 2855, 1465 $cm^{-1}$.

b) 1-(2-Nonylphenyl)-1-trans-hexen

Ein Gemisch aus 14,4 g 1-(2-Nonylphenyl)-1-hexanol, 2 g Toluol-4-sulfonsäure-monohydrat und 250 ml Toluol wird während 3 Stunden am Wasserabscheider unter Rückfluss erhitzt. Nach dem Abkühlen wird das Reaktionsgemisch zweimal mit 10%iger (G/V) Natriumbicarbonatlösung und zweimal mit Wasser gewaschen. Die organische Phase wird über Natriumsulfat getrocknet und im Vakuum eingedampft, und der Rückstand durch Chromatographie an Kieselgel und Elution mit Hexan gereinigt. Das gewünschte 1-(2-Nonylphenyl)-trans-hexen wird als blassgelbes Oel erhalten. IR ($CH_2Cl_2$): 2960, 2930, 2855, 1465, 970 $cm^{-1}$.

c) 1(RS),2(RS)-1,2-Epoxy-1-(2-nonylphenyl)-hexan

Eine Lösung von 13,6 g 1-(2-Nonylphenyl)-1-trans-hexen in 350 ml Methylenchlorid wird mit 15,2 g 85%iger 3-Chlorperbenzoesäure versetzt und während 3 Stunden bei Raumtemperatur gerührt. Das Reaktionsgemisch wird mit Methylenchlorid verdünnt und je zweimal mit gesättigter Natriumbicarbonatlösung und Wasser gewaschen. Der nach dem Trocknen und Eindampfen der organischen Phase verbleibende halbfeste Rückstand wird in Hexan aufgeschlämmt und abfiltriert, und das Filtrat unter vermindertem Druck eingedampft. Chromatographische Reinigung des Rohprodukts an Kieselgel mit Hexan/Ether (97:3)

ergibt das gewünschte 1(RS),2(RS)-1,2-Epoxy-1-(2-nonylphenyl)-hexan als farboses Oel. IR ($CH_2Cl_2$): 2960, 2930, 2860, 1470 $cm^{-1}$.

Beispiel 17A : 7-[1(RS),2(SR)-1-(2-Dodecylphenyl)-2-hydroxypentyl-thio]-4-oxo-4H-chromen-2-carbonsäure-methylester

In analoger Weise wie im Beispiel 17 aber ausgehend von 1(RS),2(RS)-1-(2-Dodecylphenyl)-1,2-epoxypentan und 7-Mercapto-4-oxo-4H-chromen-2-carbonsäure-methylester erhält man die Titelverbindung; Smp. 77-78°; IR ($CH_2Cl_2$): 3580, 2960, 2925, 2855, 1745, 1655, 1600, 1415 $cm^{-1}$.

Das als Ausgangsstoff verwendete 1(RS),2(RS)-1-(2-Dodecylphenyl)-1,2-epoxypentan kann gemäss der im Beispiel 17a - c beschriebenen Methode folgendermassen hergestellt werden: 2-Dodecylphenylmagnesiumbromid und Pentanal ergeben 1-(2-Dodecylphenyl)-pentanol, welches zum 1-(2-Dodecylphenyl)-penten dehydratisiert wird, und dieses mit 3-Chlorperbenzoesäure zum gewünschten Epoxid (farbloses Oel, IR in $CH_2Cl_2$, 2960, 2920, 2850, 1460 $cm^{-1}$) umgewandelt wird.

Beispiel 17B : 7-[1(RS),2(SR)-2-Hydroxy-1-(2-pentadecyl-phenyl)-pentyl-thio]-4-oxo-4H-chromen-2-carbonsäure-methylester

In analoger Weise wie im Beispiel 17 aber ausgehend von 1(RS),2(RS)-1,2-Epoxy-1-(2-pentadecylphenyl)-pentan und 7-Mercapto-4-oxo-4H-chromen-2-carbonsäure-methylester erhält man die Titelverbindung als Festkörper (aus Petrolether), Smp. 68-69°C; IR ($CH_2Cl_2$): 3580, 2960, 2930, 2860, 1745, 1655, 1600, 1415 $cm^{-1}$.

Das als Ausgangsstoff verwendete 1(RS),2(RS)-1,2-Epoxy-1-(2-pentadecylphenyl)-pentan kann gemäss der im Beispiel 17a - c beschriebenen Methode folgendermassen hergestellt werden: 2-Pentadecylphenylmagnesiumbromid und Pentanal ergeben 1-(2-Pentadecylphenyl)-pentanol, welches zum 1-(2-Pentadecylphenyl)-penten dehydratisiert wird, und dieses mit 3-Chlorperbenzoesäure zum gewünschten Epoxid umgewandelt wird.

Beispiel 17C : 7-[1(RS), 2(SR)-1-(2-Dodecylphenyl)-2-hydroxypentyl-thio]-4-oxo-8-propyl-4H-chromen-2-carbonsäure-methylester

In analoger Weise wie im Beispiel 17 aber ausgehend von 1(RS),2(RS)-1-(2-Dodecylphenyl)-1,2-epoxypentan (siehe Beispiel 17A) und 7-Mercapto-4-oxo-8-propyl-4H-chromen-2-carbonsäure-methylester erhält man die Titelverbindung; dickflüssiges Oel; IR ($CH_2Cl_2$): 3580, 2960, 2930, 2860, 1745, 1655, 1590, 1410, 1250 $cm^{-1}$.

Beispiel 17D : 7-[1(RS),2(SR)-2-Hydroxy-1-(2-nonylphenyl)-hexyl-thio]-2-methoxychinolin-3-carbonsäure-methylester

In analoger Weise wie im Beispiel 17 aber ausgehend von 1(RS),2(RS)-1,2-Epoxy-1-(2-nonylphenyl)-hexan (siehe Beispiel 17c) und 7-Mercapto-2-methoxychinolin-3-carbonsäure-methylester, erhält man die Titelverbindung; blassgelber Honig; IR ($CH_2Cl_2$): 3580, 2950, 2920, 2850, 1725, 1610, 1480, 1450, 1400, 1340, 1190, 1080 $cm^{-1}$.

Beispiel 17E : 7-[1(RS),2(SR)-1-(2-Dodecylphenyl)-2-hydroxypentyl-thio]-2-methoxychinolin-3-carbonsäure-methylester

In analoger Weise wie im Beispiel 17 aber ausgehend von 1(RS),2(RS)-1-(2-Dodecylphenyl)-1,2-epoxypentan und 7-Mercapto-2-methoxychinolin-3-carbonsäure-methylester erhält man die Titelverbindung; dickflüssiges Oel, IR ($CH_2Cl_2$): 3580, 2960, 2925, 2860, 1730, 1610, 1480, 1455, 1400, 1345, 1190, 1080 $cm^{-1}$.

Beispiel 17F : 7-[1(RS),2(SR)-2-Hydroxy-1-(2-pentadecyl-phenyl)-pentyl-thio]-2-methoxychinolin-3-carbonsäure-methylester

In analoger Weise wie im Beispiel 17 aber ausgehend von 1(RS),2(RS)-1,2-Epoxy-1-(2-pentadecylphenyl)-pentan und 7-Mercapto-2-methoxychinolin-3-carbonsäure-methylester erhält man die Titelverbindung als dickflüssiges Oel; IR ($CH_2Cl_2$): 3580, 2960, 2925, 2850, 1730, 1610, 1480, 1455, 1400, 1340, 1080 $cm^{-1}$

Beispiel 17G : 7-[1(RS),2(SR)-2-Hydroxy-1-(2-nonylphenyl)-pentyl-thio]-4-oxo-4H-chromen-2-carbonsäure-methylester

In analoger Weise wie im Beispiel 17, aber ausgehend von 1(RS),2(RS)-1,2-Epoxy-1-(2-nonylphenyl)-pentan und 7-Mercapto-4-oxo-4H-chromen-2-carbonsäure-methylester, erhält man die Titelverbindung; IR ($CH_2Cl_2$): 2930, 2860, 1745, 1660, 1605, 1240 $cm^{-1}$.

Das als Ausgangsstoff verwendete 1(RS),2(RS)-1,2-Epoxy-1-(2-nonylphenyl)-pentan kann analog Beispiel 17a-c ausgehend von 2-Nonylbrombenzol und Pentanol hergestellt werden.

Beispiel 18: 7-[1(RS),2(SR)-2-Hydroxy-1-(2-nonylphenyl)-hexyl-thio]-4-oxo-4H-chromen-2-carbonsäure

Ein Gemisch von 2,5 g 7-[1(RS),2(SR)-2-Hydroxy-1-(2-nonylphenyl)-hexylthio]-4-oxo-4H-chromen-2-carbonsäure-methylester vom Beispiel 17, 100 ml Methanol und 55 ml 0,1-N wässriger Natronlauge wird während 18 Stunden bei Raumtemperatur gerührt. Das Reaktionsgemisch wird bei Raumtemperatur unter vermindertem Druck eingedampft, und der Rückstand zwischen Methylenchlorid und 0,2-N Salzsäure verteilt. Die organische Phase wird mit Wasser gewaschen, über Natriumsulfat getrocknet, und das Lösungsmittel bei Raumtemperatur unter vermindertem Druck entfernt. Umkristallisieren des festen Rückstands aus Ether-Hexan ergibt die Titelverbindung als blassgelben kristallinen Feststoff, vom Smp. 72-75°. IR (KBr): 3420 (breit), 2955, 2925, 2855, 1735, 1635, 1595, 1420, 1235, 1150, 960, 905, 760 $cm^{-1}$.

Beispiel 18A : 7-[1(RS),2(SR)-1-(2-Dodecylphenyl)-2-hydroxypentyl-thio]-4-oxo-4H-chromen-2-carbonsäure

In analoger Weise wie im Beispiel 18 erhält man die Titelverbindung aus dem entsprechenden Methylester (siehe Beispiel 17A);
Smp. 111-116°; IR ($CH_2Cl_2$): 3580, 3450, 2960, 2930, 2860, 1740, 1655, 1600, 1420 $cm^{-1}$.

Beispiel 18B : 7-[1(RS),2(SR)-2-Hydroxy-1-(2-pentadecyl-phenyl)-pentylthio]-4-oxo-4H-chromen-2-carbonsäure-natriumsalz

In analoger Weise wie im Beispiel 18F unten beschrieben ist, erhält man die Titelverbindung aus dem entsprechenden Methylester (siehe Beispiel 17B) als Feststoff, Smp. 238-240° (Zersetzung); IR (KBr): 2960, 2925, 2855, 1635, 1610, 1420 $cm^{-1}$

Beispiel 18C : 7-[1(RS),2(SR)-1-(2-Dodecylphenyl)-2-hydroxypentyl-thio]-4-oxo-8-propyl-4H-chromen-2-carbonsäure-natriumsalz

In analoger Weise wie im Beispiel 18F unten beschrieben ist, erhält man die Titelverbindung aus dem entsprechenden Methylester (siehe Beispiel 17C) in fester Form, Smp. 247-249° (Zersetzung); IR ($CH_2Cl_2$): 2960, 2930, 2860, 1635, 1415, 1365 $cm^{-1}$.

Beispiel 18D : 7-[1(RS),2(SR)-2-Hydroxy-1-(2-nonylphenyl)-hexyl-thio]-2-methoxychinolin-3-carbonsäure

Ein Gemisch von 1.1 g 7-[1(RS),2(SR)-2-Hydroxy-1-(2-nonylphenyl)-hexyl-thio]-2-methoxychinolin-3-carbonsäuremethylester (siehe Beispiel 17D), 35 ml Methanol und 2.5 ml einer wässrigen 2N-Lösung von Natriumhydroxid wird unter Argon 14 Stunden bei Raumtemperatur gerührt und anschliessend bei 45°

unter vermindertem Druck eingeengt. Der Rückstand wird mit 0.1N-Salzsäure angesäuert und in Methylenchlorid aufgenommen. Der organische Extrakt wird über Natriumsulfat getrocknet und im Vakuum eingeengt. Der Rückstand wird am Kieselgel chromatographiert und mit Ether mit steigendem Anteil an Methanol eluiert. Durch Abdestillieren der Lösungsmittel resulriert die Titelverbindung als blassgelber Honig: IR ($CH_2Cl_2$): 3580, 3320, 2960, 2930, 2860, 1745, 1610, 1485, 1390, 1340 $cm^{-1}$.

Beispiel 18E : 7-[1(RS),2(SR)-1-(2-Dodecylphenyl)-2-hydroxypentyl-thio]-2-methoxychinolin-3-carbonsäure

In analoger Weise wie im Beispiel 18D erhält man die Titelverbindung aus dem entsprechenden Methylester (siehe Beispiel 17E); gelbliches Harz, IR ($CH_2Cl_2$): 3580, 3320, 2960, 2930, 2860, 1745, 1610, 1485, 1390, 1340 $cm^{-1}$.

Beispiel 18F : 7-[1(RS),2(SR)-2-Hydroxy-1-(2-pentadecyl-phenyl)-pentyl-thio]-2-methoxychinolin-3-carbonsäure-natriumsalz

Ein Gemisch von 7-[1(RS),2(SR)-2-Hydroxy-1-(2-pentadecyl-phenyl)-pentyl-thio]-2-methoxychinolin-3-carbonsäure-methylester (0.81 g), 15 ml Methanol und 5 ml Tetrahydrofuran wird mit 1.3 ml einer wässrigen 1N-Lösung von Natriumhydroxid versetzt und bei Raumtemperatur 12 Stunden unter Argon gerührt. Die leichtflüchtigen Anteile werden bei 45° unter vermindertem Druck entfernt und der Rückstand mit Tetrachlorkohlenstoff verrieben. Durch Abdampfen der flüchtigen Anteile im Vakuum resultiert die Titelverbindung als farbloser Feststoff mit Schmelzpunkt oberhalb 250° ; IR (KBr): 2960, 2925, 2855, 1635, 1610, 1585, 1390, 1240 $cm^{-1}$.

Beispiel 18G : 7-[1(RS),2(SR)-2-Hydroxy-1-(2-nonylphenyl)-pentyl-thio]-4-oxo-4H-chromen-2-carbonsäure

In analoger Weise wie im Beispiel 18 erhält man die Titelverbindung aus dem entsprechenden Methylester (siehe Beispiel 17G);
Smp. 124-126° .

Beispiel 19: 7-[1(RS),2(SR)-2-Hydroxy-1-(4-nonylphenyl)-hexylthio]-4-oxo-4H-chromen-2-carbonsäuremethylester.

Unter Argon werden 0,85 g 1(RS),2(RS)-1,2-Epoxy-1-(4-nonylphenyl)-hexan in 20 ml Methanol gelöst, mit 0,86 g Triethylamin und dann mit 0,85 g 7-Mercapto-4-oxo-4H-chromen-2-carbonsäuremethylester versetzt, und 22 Std. bei Raumtemperatur gerührt.

Der ausgeschiedene Niederschlag wird abgesaugt und mit wenig Methanol und Hexan gewaschen. Die erhaltene Titelverbindung schmilzt bei 135-136° .

Das als Ausgangsmaterial verwendete 1(RS),2(RS)-1,2-Epoxy-1-(4-nonylphenyl)-hexan kann z.B. wie folgt hergestellt werden:

a) 1-(4-Nonylphenyl)-hex-1-en (Gemisch vom cis- und trans-Isomeren)

Eine Suspension von 13,9 g Pentyltriphenylphosphoniumbromid in 150 ml Tetrahydrofuran wird unter Argon auf -20° gekühlt, innert 5 Minuten mit 21,2 ml 1,6-M Butyllithiumlösung in Hexan versetzt und weitere 30 Min. bei 0-10° gerührt. In das auf -60° bis -70° abgekühlte Gemisch wird 6 g 4-Nonylbenzaldehyd in 40 ml Tetrahydrofuran während 30 Minuten zugetropft. Das Reaktionsgemisch wird auf 0-10° spontan erwärmen gelassen, bei dieser Temperatur noch 45 Min. gerührt und eingedampft. Der Rückstand wird in Hexan/Ethylacetat (1:1) aufgenommen und über Kieselgel filtriert. Das Filtrat wird eingedampft und an Kieselgel mit Hexan chromatographiert. Die Titelverbindung (Gemisch vom cis - und trans -Isomeren) resultiert als farbloses Oel, welches in nächster Stufe direkt eingesetzt wird.

b) 1,2-Epoxy-1-(4-nonylphenyl)-hexan und Trennung in das individuelle cis- [1(RS),2(SR)-] und trans- [1-

(RS),2(RS)-] -Isomere

Eine Lösung von 6,32 g 1-(4-Nonylphenyl)-hex-1-en (Gemisch vom cis -und trans -Isomeren) aus vorangehender Stufe in 150 ml Dichlormethan wird unter Kühlung auf 0-5° mit 6,76 g m-Chlorperbenzoesäure (90 % Gehalt) in 100 ml Dichlormethan versetzt und 20 Stunden bei 20° gerührt. Das Reaktionsgemisch wird nacheinander mit 10%iger (G/V) Natriumsulfit-Lösung, 5%iger (G/V) Natriumcarbonat-Lösung und 3 Portionen Wasser gewaschen, über Natriumsulfat getrocknet und eingedampft. Chromatographie des Rückstandes an Kieselgel mit Hexan/Ethylacetat (19:1) ergibt nacheinander das trans [1(RS),2(RS)-] und das cis - [1(RS),2(SR)-] -Isomere als farblose Oele.

Beispiel 19A : 3-[1(RS),2(SR)-2-Hydroxy-1-(4-nonylphenyl)-hexylthio]-malonanilinsäuremethylester

In anloger Weise wie im Beispiel 19 aber ausgehend von 1(RS),2(RS)-1,2-Epoxy-1-(4-nonylphenyl)-hexan und 3-Mercapto-malonanilinsäuremethylester als Mercapto-Komponente erhält man die Titelverbindung.

Beispiel 19B : 7-[1(RS),2(SR)-2-Hydroxy-1-(4-nonylphenyl)-hexylthio]-2-methoxychinolin-3-carbonsäuremethylester

In analoger Weise wie im Beispiel 19 aber ausgehend von 1(RS),2(RS)-1,2-Epoxy-1-(4-nonylphenyl)-hexan und 7-Mercapto-2-methoxychinolin-3-carbonsäuremethylester als Mercapto-Komponente erhält man die Titelverbindung.

Beispiel 20: 7-[1(RS),2(RS)-2-Hydroxy-1-(4-nonylphenyl)-hexylthio]-4-oxo-4H-chromen-2-carbonsäuremethylester.

In analoger Weise wie im Beispiel 19, aber ausgehend vom entsprechenden 1(RS),2(SR)-1,2-Epoxy-1-(4-nonylphenyl)-hexan (siehe Beispiel 19b) erhält man die Titelverbindung nach Chromatographie an Silicagel und Elution mit Hexan/Ethylacetat (3:2) als Kristalle vom Smp. 85-86°.

Beispiel 20A : 7-[1(RS),2(SR)-2-Hydroxy-1-(4-nonylphenyl)-pentyl-thio]-4-oxo-4H-chromen-2-carbonsäuremethylester

In analoger Weise wie im Beispiel 19, aber ausgehend von 1(RS),2(RS)-1,2-Epoxy-1-(4-nonylphenyl)-pentan und 7-Mercapto-4-oxo-4H-chromen-2-carbonsäuremethylester, erhält man die Titelverbindung als gelbe Kristalle; Smp. 136-138°.

Das als Ausgangsmaterial verwendete 1(RS),2(RS)-1,2-Epoxy-1-(4-nonylphenyl)-pentan kann analog Beispiel 17a-c ausgehend von 4-Nonylbrombenzol und Pentanal hergestellt werden; IR ($CH_2Cl_2$): 2890, 2820, 1500, 1445 $cm^{-1}$.

Beispiel 21 : 7-[1(RS),2(SR)-2-Hydroxy-1-(4-nonylphenyl)-hexylthio]-4-oxo-4H-chromen-2-carbonsäure-natriumsalz

In analoger Weise wie im Beispiel 7 erhält man die Titelverbindung aus dem entsprechenden Methylester (siehe Beispiel 19) als Feststoff vom Smp. 267° (Zersetzung).

Beispiel 22 : 7-[1(RS),2(SR)-2-Hydroxy-1-(4-nonylphenyl)-hexylthio]-malonanilinsäure-natriumsalz

In analoger Weise wie im Beispiel 7 erhält man die Titelverbindung aus dem entsprechenden Methylester (siehe Beispiel 19A) als festes Monohydrat, Smp. 168-169°.

Beispiel 23 : 7-[1(RS),2(SR)-2-Hydroxy-1-(4-nonylphenyl)-hexylthio]-2-methoxychinolin-3-carbonsäurenatriumsalz

In analoger Weise wie im Beispiel 18F erhält man die Titelverbindung aus dem entsprechenden Methylester (siehe Beispiel 19B) in fester Form, Smp. 268-270°.

Beispiel 24 : 7-[1(RS),2(RS)-2-Hydroxy-1-(4-nonylphenyl)-hexylthio]-4-oxo-4H-chromen-2-carbonsäure-natriumsalz

In analoger Weise wie im Beispiel 7 erhält man die Titelverbindung aus dem entsprechenden Methylester (siehe Beispiel 20).

Beispiel 24A : 7-[1(RS),2(SR)-2-Hydroxy-1-(4-nonylphenyl)-pentyl-thio]-4-oxo-4H-chromen-2-carbonsäure

In analoger Weise wie im Beispiel 18 erhält man die Titelverbindung aus dem entsprechenden Methylester (siehe Beispiel 20A); Smp. 98-100°.

Beispiel 25 : 7-[1(RS),2(SR)-1-Hydroxy-1-(m -tolyl)-3-trans-5-cis-pentadecadien-2-yl-thio]-4-oxo-4H-chromen-2-carbonsäuremethylester

Ein Gemisch von 2.2 g 1(RS),2(RS)-1,2-Epoxy-1-(m -tolyl)-3-trans-5-cis-pentadecadien, 2,0 g 7-Mercapto-4-oxo-4H-chromen-2-carbonsäure-methylester und 3.4 ml Triethylamin in 40 ml Tetrahydrofuran wird 20 Stunden bei Raumtempertaur gerührt und durch Destillation unter vermindertem Druck von flüchtigen Anteilen befreit. Der Rückstand ergibt durch Chromatographie am Silicagel und Elution mit einem 3:2-Gemisch (V/V) von Hexan-Ethylacetat die Titelverbindung als rotgelbes Harz.

Das als Ausgangsstoff verwendete 1(RS),2(RS)-1,2-Epoxy-1-(m -tolyl)-3-trans-5-cis-pentadecadien kann folgendermassen hergestellt werden:

a) 3-(m-Tolyl)-2-trans-propenal (m-Methylzimtaldehyd)

In einer siedenden Lösung von 56.64 g m-Tolylaldehyd in 670 ml Toluol wird in mehreren Portionen insgesamt 152.1 g Formylmethylentriphenylphoshoran unter Rückfluss zugegeben und anschliessend 16 Stunden zum Sieden erhitzt. Flüchtige Anteile werden abdestilliert, der Rückstand in einem 4:1-Gemisch (V/V) von Ether-Hexan gelöst und durch Kieselgel filtriert. Das Filtrat wird von den Lösungsmitteln durch Destillation befreit und in höherem Vakuum destilliert. Die Titelverbindung wird als farbloses Oel, Sdp. 87-92°/3 mbar, erhalten.

b) 2(RS),3(SR)-2,3-Epoxy-3-(m-tolyl)-propionaldehyd

Eine Lösung von 25.32 g m-Methylzimtaldehyd (siehe oben) in 400 ml Methanol wird zu einem kräftig gerührten Gemisch von 136 ml 30%igem Wasserstoffperoxid und 54 g Natriumhydrogencarbonat in 1200 ml Wasser bei 20-25° während 45 Minuten zugetropft und weitere 2 Stunden gerührt. Das Reaktionsgemisch wird mehrmals mit Methylenchlorid extrahiert. Die organischen Anteile werden einmal mit einer Natriumsulfitlösung gewaschen, über Natriumsulfat getrocknet und durch Abdampfen vom Lösungsmittel befreit. Chromatographie des Rückstands am Kieselgel und Elution mit einem 3:1-Gemisch (V/V) von Hexan-Ethylacetat ergibt die Titelverbindung als farbloses Oel.

c) 4(RS),5(RS)-4,5-Epoxy-5-(m-tolyl)-2-trans-penenal

Zu einer Lösung von 9.56 g 2(RS),3(SR)-2,3-Epoxy-3-(m-tolyl)-propionaldehyd (siehe unter b) in 50 ml Methylenchlorid wird unter Argonatmosphäre bei Raumtemperatur eine Lösung von 18.63 g Formylmethylen-triphenylphosphoran in 70 ml Methylenchlorid zugetropft und 20 Stunden bei Raumtemperatur gerührt. Das Reaktionsgemisch wird eingeengt, mit einem Gemisch von Hexan und Ethylacetat im Volumenverhältnis 1:1 verdünnt und über Kieselgel filtriert. Nach Abdampfen der Lösungsmittel wird der Rückstand am Kieselgel chromatographiert. Elution mit Hexan-Ether (1:1) und Abdestillieren der Lösungsmittel ergibt die Titelverbindung als farbloses Oel.

d) 1(RS),2(RS)-1,2-Epoxy-1-(m-tolyl)-3-trans-5-cis-pentadecadien

Unter Argonatmosphäre wird eine gerührte, auf -78° gekühlte Lösung von 13.39 g Decyl-triphenylphosphoniumbromid in 120 ml Tetrahydrofuran mit 17.3 ml einer 1.6M-Lösung von Butyllithium in Hexan versetzt. Die Temperatur des Gemisches lässt man vorübergehend auf 0° spontan steigen und kühlt die Lösung erneut auf -78° ab. Unter Rühren und Kühlen wird eine Lösung von 7.02 g 4(RS),5(RS)-4,5-Epoxy-4-(m-tolyl)-2-trans-pentenal (siehe unter c) in 30 ml Tetrahydrofuran so zugetropft, dass die Temperatur nicht -70° übersteigt. Das Reaktionsgemisch wird auf +10° spontan erwärmen gelassen und im Vakuum eingeengt. Der Rückstand wird in ein 4:1-Gemisch (V/V) von Ether-Hexan aufgenommen und durch eine Kieselgelsäule, welche vorher mit demselben Lösungsmittelgemisch mit 1%igem Zusatz von Triethylamin gewaschen wurde, filtriert. Durch Einengen des Filtrats in Vakuum erhält man die Titelverbindung als farbloses zähflüssiges Oel.

Beispiel 25A : 7-[1(RS),2(RS)-1-Hydroxy-1-(4-methylphenyl)-3-cis-hexadecen-2-yl-thio]-4-oxo-4H-chromen-2-carbonsäure-methylester

In analoger Weise wie im Beispiel 25, aber ausgehend von 1(RS),2(RS)-1,2-Epoxy-1-(4-methylphenyl)-3-cis-hexadecen und 7-Mercapto-4-oxo-4H-chromen-2-carbonsäure-methylester, erhält man die Titelverbindung als gelbe Kristalle; Smp. 133-135°.

Das als Ausgangsmaterial verwendete 1(RS),2(RS)-1,2-Epoxy-1-(4-methylphenyl)-3-cis-hexadecen kann gemäss der im Beispiel 25a, b und d beschriebenen Methode wie folgt hergestellt werden: p-Tolylaldehyd und Formylmethylentriphenylphosphoran ergeben p-Methylzimtaldehyd, der zu 2(RS),3(SR)-2,3-Epoxy-3-(p-tolyl)-propionaldehyd umgesetzt wird, und dieser mit Tridecyl-triphenyl-phosphoniumbromid zum gewünschten Produkt umgewandelt wird; IR ($CH_2Cl_2$): 2880, 2810, 1490, 1445 $cm^{-1}$.

Beispiel 25B : 7-[1(S),2(R)-1-Hydroxy-1-(3-trifluormethylphenyl)-4-(3-nonylphenyl)-3-cis-buten-2-yl-thio]-4-oxo-4H-chromen-2-carbonsäure-methylester und dessen trans-Isomeres

In analoger Weise wie im Beispiel 1, aber ausgehend von 1(S),2(S)-1-(3-Trifluormethylphenyl)-4-(3-nonylphenyl)-1,2-epoxy-3-buten (cis-/trans-Isomerengemisch) und 7-Mercapto-4-oxo-4H-chromen-2-carbonsäure-methylester, erhält man die Titelverbindungen als cis-/trans-Isomerengemisch. Chromatographie an Kieselgel mit Hexan-Ethylacetat (7:3) führt zur Auftrennung der Isomeren, wobei das cis-Isomere, $[\alpha]_D^{20}$ = +33.6 ± 8.0° (0.125 % in Chloroform), vor dem trans-Isomeren, $[\alpha]_D^{20}$ = -183.0 ± 7.4° (0.135 % in Chloroform), eluiert wird.

Das als Ausgangsstoff verwendete 1(S),2(S)-1-(3-Trifluormethylphenyl)-4-(3-nonylphenyl)-1,2-epoxy-3-buten lässt sich z.B. wie folgt herstellen:

a) 3-(3-Trifluormethylphenyl)-2-trans-propensäure-ethylester

Zu einer Lösung von 34.0 g 3-Trifluormethylbenzaldehyd in 400 ml Dichlormethan tropft man unter Kühlung 88.9 g Ethoxycarbonylmethylen-triphenylphosphoran. Das Reaktionsgemisch wird eine Stunde bei Raumtemperatur gerührt, das Lösungsmittel im Vakuum abgedampft und der Rückstand mit Ether-Hexan (4:1) über Kieselgel filtriert. Nach Eindampfen des Eluates erhält man die Titelverbindung als weisse Kristalle; Smp. = 40-41° C.

b) 3-(3-Trifluormethylphenyl)-2-trans-propenol

Reduktion von 3-(3-Trifluormethylphenyl)-2-trans-propensäure-ethylester wie im Beispiel 9b beschrieben

ergibt die Titelverbindung als farbloses Oel; IR ($CH_2Cl_2$): 3550, 2820, 1310, 1140, 1100 $cm^{-1}$.

c) 2(S),3(S)-2,3-Epoxy-3-(3-trifluormethylphenyl)-propanol

Epoxidierung von 3-(3-Trifluormethylphenyl)-2-trans-propenol wie im Beispiel 9c beschrieben, aber unter Verwendung von L-(+)-Weinsäurediethylester, ergibt die Titelverbindung als farbloses Oel; IR ($CH_2Cl_2$): 3500, 2820, 1330, 1170, 1125, 1070 $cm^{-1}$.

d) 2(R),3(S)-2,3-Epoxy-3-(3-trifluormethylphenyl)-propanol

Oxidation von 2(S),3(S)-2,3-Epoxy-3-(3-trifluormethylphenyl)-propanol wie im Beispiel 9d beschrieben ergibt die Titelverbindung als farbloses Oel; IR ($CH_2Cl_2$): 2820, 1730, 1330, 1170, 1125, 1070 $cm^{-1}$.

e) 1(S),2(S)-1-(3-Trifluormethylphenyl)-4-(3-nonylphenyl)-1,2-epoxy-3-buten (cis-/trans-Isomerengemisch)

In analoger Weise wie im Beispiel 1d, aber ausgehend von 2(R),3(S)-2,3-Epoxy-3-(3-trifluormethylphenyl)-propanol und 3-Nonylbenzyl-triphenylphosphoniumbromid, erhält man das cis-/trans-Isomerengemisch der Titelverbindung als hellgelbes Oel.

Beispiel 25C : 7-[1(S),2(R)-1-Hydroxy-1-(3-trifluormethylphenyl)-4-(3-nonylphenyl)-3-buten-2-yl-thio]-2-methoxy-chinolin-3-carbonsäure-methylester (cis-/trans-Isomerengemisch)

In analoger Weise wie im Beispiel 1, aber ausgehend von 1(S),2(S)-1-(3-Trifluormethylphenyl)-4-(3-nonylphenyl)-1,2-epoxy-3-buten (cis-/trans-Isomerengemisch) und 7-Mercapto-2-methoxychinolin-3-carbonsäure-methylester, erhält man die Titelverbindungen als farbloses Oel; UV (Chloroform): $\lambda_{max}$ ($\epsilon$) = 340 (11 000) nm.

Beispiel 25D : 7-[1(S),2(R)-1-Hydroxy-1-(3-trifluormethylphenyl)-4-(4-nonylphenyl)-3-cis-buten-2-yl-thio]-4-oxo-4H-chromen-2-carbonsäure-methylester und dessen trans-Isomeres

In analoger Weise wie im Beispiel 1, aber ausgehend von 1(S),2(S)-1-(3-Trifluormethylphenyl)-4-(4-nonylphenyl)-1,2-epoxy-3-buten (cis-/trans-Isomerengemisch) und 7-Mercapto-4-oxo-4H-chromen-2-carbonsäure-methylester, erhält man die Titelverbindungen als cis-/trans-Isomerengemisch. Chromatographie mit Hexan-Ethylacetat (7:3) an Kieselgel führt zur Auftrennung der Isomeren, wobei das cis-Isomere, $[\alpha]_D^{20}$ = +91.3 ± 8.7° (0.115 % in Chloroform), vor dem trans-Isomeren, $[\alpha]_D^{20}$ = -187.4 ± 7.4° (0.135 % in Chloroform), eluiert wird.

Das als Ausgangsstoff verwendete cis-/trans-Isomerengemisch von 1(S),2(S)-1-(3-Trifluormethylphenyl)-4-(4-nonylphenyl)-1,2-epoxy-3-buten wird in analoger Weise wie im Beispiel 1d, aber ausgehend von 2(R),3-(S)-2,3-Epoxy-3-(3-trifluormethylphenyl)-propanol und 4-Nonylbenzyl-triphenylphosphoniumbromid als hellgelbes Oel erhalten.

Beispiel 25E : 7-[1(S),2(R)-1-Hydroxy-1-(3-trifluormethylphenyl)-4-(4-nonylphenyl)-3-buten-2-yl-thio]-2-methoxy-chinolin-3-carbonsäure-methylester (cis-/trans-Isomerengemisch)

In analoger Weise wie im Beispiel 1, aber ausgehend von 1(S),2(S)-1-(3-Trifluormethylphenyl)-4-(4-nonylphenyl)-1,2-epoxy-3-buten (cis-/trans-Isomerengemisch) und 7-Mercapto-2-methoxychinolin-3-carbonsäure-methylester, erhält man die Titelverbindungen als farbloses Oel; UV (Chloroform): $\lambda_{max}$ ($\epsilon$) = 340 (12 400), 300 (10 000) nm.

Beispiel 25F : 7-[1(S),2(R)-1-Hydroxy-1-(3-trifluormethylphenyl)-3-trans-5-cis-hexadecadien-2-yl-thio]-4-oxo-4H-chromen-2-carbonsäure-methylester

In analoger Weise wie im Beispiel 1, aber ausgehend von 1(S),2(S)-1,2-Epoxy-1-(3-trifluormethylphenyl)-3-trans-5-cis-hexadecadien und 7-Mercapto-4-oxo-4H-chromen-2-carbonsäure-methylester, erhält man die Titelverbindung als farbloses Oel; $[\alpha]_D^{20}$ = +49,4 ±10° (0,099 % in Chloroform).

Das als Ausgangsmaterial verwendete 1(S),2(S)-1,2-Epoxy-1-(3-trifluormethylphenyl)-3-trans-5-cis-hexadecadien erhält man ausgehend von 2(R),3(S)-2,3-Epoxy-3-(3-trifluormethylphenyl)-propanol (siehe Beispiel 25B). Dieses wird analog Beispiel 25c vinylogisiert und analog Beispiel 25d, aber unter Verwendung von Undecyl-triphenylphosphoniumbromid, zum benötigten Epoxid umgesetzt.

Beispiel 26 : 7-[1(RS),2(SR)-1-Hydroxy-1-(m -tolyl)-3-trans-5-cis-pentadecadien-2-yl-thio]-4-oxo-4H-chromen-2-carbonsäure

Eine Lösung von 3.02 g Methylester der Titelverbindung (siehe Beispiel 25) in 50 ml Tetrahydrofuran wird mit 6.6 ml einer wässrigen 0.1N-Lösung von Natriumhydroxid versetzt, 2 Stunden bei 20° gerührt und unter vermindertem Druck eingeengt. Der Rückstand wird in Wasser gelöst, mit 1N-Salzsäure auf pH=1 gebracht und mit Ether extrahiert. Die organischen Auszüge werden zweimal mit einer gesättigten Natriumchloridlösung gewaschen, über Magnesiumsulfat getrocknet und im Vakuum vom Lösungsmittel befreit. Der Rückstand wird am Kieselgel chromatographiert, die mit Methylenchlorid-Methanol (5:1) eluierten Fraktionen ergeben nach Abdestillieren des Lösungsmittels die Titelverbindung in Form eines orangefarbenen Pulvers, Smp. 66-67°.

Beispiel 26A : 7-[1(RS),2(SR)-1-Hydroxy-1-(4-methylphenyl)-3-cis-hexadecen-2-yl-thio]-4-oxo-4H-chromen-2-carbonsäure-natriumsalz

In analoger Weise wie im Beispiel 18F erhält man die Titelverbindung aus dem entsprechenden Methylester (siehe Beispiel 25A); Smp. 255-257°.

Beispiel 26B : 7-[1(S),2(R)-1-Hydroxy-1-(3-trifluormethylphenyl)-4-(3-nonylphenyl)-3-cis-buten-2-yl-thio]-4-oxo-4H-chromen-2-carbonsäure

In analoger Weise wie im Beispiel 5 erhält man die Titelverbindung ausgehend vom entsprechenden Methylester als beigen Feststoff; Smp. 253° (Zers.).

Beispiel 26C : 7-[1(S),2(R)-1-Hydroxy-1-(3-trifluormethylphenyl)-4-(3-nonylphenyl)-3-trans-buten-2-yl-thio]-4-oxo-4H-chromen-2-carbonsäure

In analoger Weise wie im Beispiel 5 erhält man die Titelverbindung ausgehend vom entsprechenden Methylester als hellgelben Feststoff; Smp. 253° (Zers.).

Beispiel 26D : 7-[1(S),2(R)-1-Hydroxy-1-(3-trifluormethylphenyl)-4-(3-nonylphenyl)-3-buten-2-yl-thio]-2-methoxy-chinolin-3-carbonsäure (cis-/trans-Isomerengemisch)

In analoger Weise wie im Beispiel 5 erhält man die Titelverbindung ausgehend vom entsprechenden cis-/trans-Isomerengemisch der Methylester als weissen Feststoff; Smp. 81-82°.

Beispiel 26E : 7-[1(S),2(R)-1-Hydroxy-1-(3-trifluormethylphenyl)-4-(4-nonylphenyl)-3-cis-buten-2-yl-thio]-4-oxo-4H-chromen-3-carbonsäure

In analoger Weise wie im Beispiel 5 erhält man die Titelverbindung ausgehend vom entsprechenden Methylester als Feststoff; Smp. 69-70°.

Beispiel 26F : 7-[1(S),2(R)-1-Hydroxy-1-(3-trifluormethylphenyl)-4-(4-nonylphenyl)-3-trans-buten-2-yl-thio]-4-oxo-4H-chromen-2-carbonsäure

In analoger Weise wie im Beispiel 5 erhält man die Titelverbindung ausgehend vom entsprechenden Methylester als Feststoff; Smp. 70-71°.

Beispiel 26G : 7-[1(S),2(R)-1-Hydroxy-1-(3-trifluormethylphenyl)-4-(4-nonylphenyl)-3-buten-2-yl-thio]-2-methoxy-chinolin-3-carbonsäure (cis-/trans-Isomerengemisch)

In analoger Weise wie im Beispiel 5 erhält man die Titelverbindungen ausgehend vom entsprechenden cis-/trans-Isomerengemisch der Methylester als weissen Feststoff; Smp. 91-92°.

Beispiel 26H : 7-[1(S),2(R)-1-Hydroxy-1-(3-trifluormethylphenyl)-3-trans-5-cis-hexadecadien-2-yl-thio]-4-oxo-4H-chromen-2-carbonsäure

In analoger Weise wie im Beispiel 18 erhält man die Titelverbindung aus dem entsprechenden Methylester (siehe Beispiel 25F); IR ($CH_2Cl_2$): 3620, 2930, 1650, 1605, 1330, 1130 $cm^{-1}$.

Beispiel 27 : 7-[1(RS),2(SR)-2-Hydroxy-1-(3-nonylphenyl)-hexyl-thio]-4-oxo-4H-chromen-2-carbonsäure-methylester

In analoger Weise wie im Beispiel 17 aber ausgehend von 1(RS),2(RS)-1,2-Epoxy-1-(3-nonylphenyl)-hexan und 7-Mercapto-4-oxo-4H-chromen-2-carbonsäure-methylester erhält man die Titelverbindung als gelbliches Pulver, Smp. 92-93°.

Das als Ausgangsstoff verwendte 1(RS),2(RS)-1,2-Epoxy-1-(3-nonylphenyl)-hexan kann analog Beispiel 17a-c ausgehend von 3-Nonylbrombenzol hergestellt werden.

Beispiel 27A : 7-[1(RS),2(SR)-2-Hydroxy-1-(3-nonylphenyl)-pentyl-thio]-4-oxo-4H-chromen-2-carbonsäure-methylester

In analoger Weise wie im Beispiel 17, aber ausgehend von 1(RS),2(RS)-1,2-Epoxy-1-(3-nonylphenyl)-pentan und 7-Mercapto-4-oxo-4H-chromen-2-carbonsäure-methylester, erhält man die Titelverbindung als hellgelbe Kristalle; Smp. 84-86°.

Das als Ausgangsmaterial verwendete 1(RS),2(RS)-1,2-Epoxy-1-(3-nonylphenyl)-pentan kann analog Beispiel 17a-c ausgehend von 3-Nonylbrombenzol und Pentanal hergestellt werden; IR ($CH_2Cl_2$): 2930, 2850, 1610, 1460 $cm^{-1}$.

Beispiel 28 : 3-[1(RS),2(SR)-2-Hydroxy-1-(3-nonylphenyl)-hexyl-thio]-malonanilinsäure-methylester

In analoger Weise wie im Beispiel 1, aber ausgehend von 1(RS),2(RS)-1,2-Epoxy-1-(3-nonylphenyl)-hexan (siehe Beispiel 27) und 3-Mercapto-malonanilinsäure-methylester erhält man die Titelverbindung als farbloses Oel.

Beispiel 29 : 7-[1(RS),2(SR)-2-Hydroxy-1-(3-nonylphenyl)-hexyl-thio]-4-oxo-4H-chromen-2-carbonsäure-natriumsalz

In analoger Weise wie im Beispiel 7 erhält man die Titelverbindung aus dem entsprechenden Methylester (siehe Beispiel 27) in Form eines Feststoffes vom Smp. 185° (Zersetzung).

Beispiel 29A : 7-[1(RS),2(SR)-2-Hydroxy-1-(3-nonylphenyl)-pentyl-thio]-4-oxo-4H-chromen-2-carbonsäure

In analoger Weise wie im Beispiel 18 erhält man die Titelverbindung aus dem entsprechenden

Methylester (siehe Beispiel 27A); Smp. 134-136°.

Beispiel 30 : 3-[1(RS),2(SR)-2-Hydroxy-1-(3-nonylphenyl)-hexyl-thio]-malonanilinsäure-natriumsalz

In analoger Weise wie im Beispiel 7 erhält man die Titelverbindung aus dem entsprechenden Methylester (siehe Beispiel 28) in Form eines Feststoffes vom Smp. 150-152°.

Beispiel 31 : 7-[3(RS),4(SR)-4-Hydroxy-1-(2-nonylphenyl)-1-trans-octen-3-yl-thio]-4-oxo-4H-chromen-2-carbonsäure-methylester

In analoger Weise wie im Beispiel 17 aber ausgehend von 3(RS),4(RS)-3,4-Epoxy-1-(2-nonylphenyl)-1-trans-octen und 7-Mercapto-4-oxo-4H-chromen-2-carbonsäure-methylester erhält man die Titelverbindung nach 60 stündiger Reaktionszeit bei Raumtemperatur und üblicher Aufarbeitung als dickflüssiges Oel; IR ($CH_2Cl_2$): 3590, 2960, 2930, 2860, 1750, 1655, 1600, 1420 $cm^{-1}$. Gemäss $^1$H-NMR-Analyse enthält das Produkt etwa 10 % des entsprechenden cis -Isomeren.

Das als Ausgangsstoff verwendete 3(RS),4(RS)-3,4-Epoxy-1-(2-nonylphenyl)-1-trans-octen kann folgendermassen hergestellt werden:

a) 2-Nonyl-benzaldehyd

Ein unter Argonatmosphäre gerührtes Gemisch von 3.4 g Magnesiumspänen, 25 ml Tetrahydrofuran und 3 Tropfen Tetrachlorkohlenstoff wird mit einem Drittel einer Lösung von 22 g 2-Nonylbrombenzol in 35 ml Tetrahydrofuran versetzt und während 30 Minuten unter Rückfluss zum Sieden erhitzt. Anschliessend wird die restliche Lösung von 2-Nonylbrombenzol während einer Stunde zugetropft und das Reaktionsgemisch 2 Stunden am Rückfluss gehalten. Nach Verdünnen mit 40 ml Tetrahydrofuran wird im Eisbad auf ca. 5° abgekühlt und während 15 Minuten eine Lösung von 11,6 ml Dimethylformamid in 20 ml Tetrahydrofuran eingetropft. Nach 1-stündigem Rühren bei Raumtemperatur wird das Reaktionsgemisch mit 250 ml gesättigter Ammoniumchloridlösung versetzt, die organische Schicht abgetrennt und die wässrige dreimal mit Ether ausgezogen. Der nach dem Trocknen und Eindampfen der vereinigten Etherextrakte verbliebene Rückstand wird durch Chromatographie an Kieselgel mit Gemischen vo Petrolether mit steigendem Anteil an Methylenchlorid gereinigt, womit der gewünschte 2-Nonylbenzaldehyd als blassgelbe Flüssigkeit erhalten wird. IR ($CH_2Cl_2$): 2920, 2850, 1695, 1600 $cm^{-1}$.

b) 2-Nonyl-benzylalkohol

Eine gerührte Lösung von 9.3 g 2-Nonyl-benzaldehyd in 150 ml Methanol wird während 15 Minuten portionenweise mit 0.57 g Natriumborhydrid versetzt. Nach weiterem Rühren während 30 Minuten wird das Reaktionsgemisch unter vermindertem Druck eingedampft und der Rückstand in Ether aufgenommen. Die organische Phase wird mit eisgekühlter 0.2N-Salzsäure und mit Wasser gewaschen, über Natriumsulfat getrocknet und im Vakuum eingedampft. Chromatographische Reinigung des Rückstands an Kieselgel mit Gemischen von Petrolether mit steigendem Anteil von Ether liefert 2-Nonyl-benzylalkohol als blassgelbes Oel, IR ($CH_2Cl_2$): 3600, 2925, 2855, 1465, 1000 $cm^{-1}$.

c) 2-Nonylbenzylbromid

Zu einem gerührten Gemisch von 6.6 g 2-Nonyl-benzylalkohol und 50 ml Benzol wird während 15 Minuten eine Lösung von 10 g Phosphortribromid in 50 ml Benzol getropft. Das Reaktionsgemisch wird 30 Minuten unter Rückfluss erhitzt und nach dem Abkühlen mit Eiswasser und Ether versetzt. Die organische Phase wird abgetrennt, mit Wasser gewaschen, über Natriumsulfat getrocknet und im Vakuum eingedampft. Chromatographische Reinigung des Rückstands an Kieselgel mit Petrolether liefert 2-Nonylbenzylbromid als farbloses Oel, IR ($CH_2Cl_2$): 2920, 2850, 1470, 1210 $cm^{-1}$.

d) 2-Nonylbenzyl-triphenylphosphoniumbromid

Ein Gemisch von 7.2 g 2-Nonylbenzylbromid, 5.77 g Triphenylphosphin und 60 ml Toluol wird während 4 Stunden unter Rückfluss erhitzt, abgekühlt und mit 80 ml Ether verdünnt. Der ausgeschiedene 2-Nonylbenzyltriphenylphosphoniumbromid wird durch Filtration abgetrennt, mit Ether gewaschen und im Vakuum getrocknet; Smp. 174-176°.

e) 3(RS),4(RS)-3,4-Epoxy-1-(2-nonylphenyl)-1-trans-octen

Zu einem unter Argonatmosphäre gerührten und auf 5° gekühlten Gemisch von 5.6 g 2-Nonylbenzyl-triphenylphosphoniumbromid und 50 ml absolutem Tetrahydrofuran werden 6.4 ml einer 1.6M-Lösung von Butyllithium in Hexan gegeben. Nach weiteren 10 Minuten wird innerhalb von 3 Minuten eine Lösung von 2-(RS),3(RS)-2,3-Epoxyheptanal in 15 ml Tetrahydrofuran eingetropft. Das Gemisch wird noch eine Stunde bei 5° und 15 Minuten bei Raumtemperatur gerührt, mit Wasser versetzt und dreimal mit Ether extrahiert. Die organische Phase wird über Natriumsulfat getrocknet und eingeengt. Der verbleibende Rückstand wird in Hexan aufgeschlämmt, abfiltriert und das Filtrat unter vermindertem Druck eingedampft. Chromatographische Reinigung des Rückstands an Kieselgel und Eluieren mit einem 97:3-Gemisch (V/V) von Petrolether-Ether ergibt 3(RS),4(RS)-3,4-Epoxy-1-(2-nonylphenyl)-1-trans-octen vom IR ($CH_2Cl_2$): 2960, 2930, 2860, 1470, 870 $cm^{-1}$.

Gemäss $^1$H-NMR-Analyse enthält das Produkt etwa 10 % des entsprechenden cis -Isomeren.

Beispiel 32 : 7-[3(RS),4(SR)-4-Hydroxy-1-(2-nonylphenyl)-1-trans-octen-3-yl-thio]-2-methoxychinolin-3-carbonsäure-methylester

In analoger Weise wie im Beispiel 17 aber ausgehend von 3(RS),4(RS)-3,4-Epoxy-1-(2-nonylphenyl)-1-trans-octen (siehe Beispiel 31e) und 7-Mercapto-2-methoxychinolin-3-carbonsäure-methylester erhält man die Titelverbindung in Form eines blassgelben Oels; IR ($CH_2Cl_2$): 3580, 2960, 2925, 2860, 1730, 1615, 1485, 1460, 1400, 1345 $cm^{1-}$. Gemäss $^1$H-NMR-Analyse enthält das Produkt etwa 10 % des entsprechenden cis -Isomeren.

Beispiel 33 : 7-[3(RS),4(SR)-4-Hydroxy-1-(2-nonylphenyl)-1-trans-octen-3-yl-thio]-4-oxo-4H-chromen-2-carbonsäure

In analoger Weise wie im Beispiel 18 erhält man die Titelverbindung aus dem entsprechenden Methylester (siehe Beispiel 31) in Form eines gelben Honigs; IR ($CH_2Cl_2$): 3580, 3450, 2960, 2925, 2860, 1740, 1655, 1600, 1415, 1240 $cm^{-1}$. Gemäss $^1$H-NMR-Analyse enthält das Produkt etwa 10 % des entsprechenden cis -Isomeren.

Beispiel 34 : 7-[3(RS),4(SR)-4-Hydroxy-1-(2-nonylphenyl)-1-trans-octen-3-yl-thio]-2-methoxychinolin-3-carbonsäure

In analoger Weise wie im Beispiel 18D erhält man die Titelverbindung aus dem entsprechenden Methylester (siehe Beispiel 32) in kristalliner Form, Smp. 65-69°; IR ($CH_2Cl_2$): 3580, 3320, 2960, 2930, 2860, 1745, 1615, 1485, 1390, 1340, 1245 $cm^{-1}$. Gemäss $^1$H-NMR-Analyse enthält das Produkt etwa 10 % des entsprechenden cis -Isomeren.

Beispiel 35 : 3-[3(RS),4(SR)-4-Hydroxy-1-(4-octylphenyl)-1-octen-3-yl-thio]-malonanilinsäure-methylester (Gemisch von cis/trans-Isomeren)

In analoger Weise wie im Beispiel 19A aber ausgehend von 3(RS),4(RS)-3,4-Epoxy-1-(4-octylphenyl)-1-octen (Gemisch von cis/trans-Isomeren) und 3-Mercapto-malonanilinsäure-methylester als Mercapto-Komponente erhält man die Titelverbindung (Gemisch von cis/trans-Isomeren).

a) Das als Ausgansstoff verwendete 3(RS),4(RS)-3,4-Epoxy-1-(4-octylphenyl)-1-octen kann gemäss dem

im Beispiel 31e beschriebenen Verfahren unter Verwendung von 4-Octylbenzyl-triphenylphosphoniumbromid erhalten werden.

Beispiel 36 : 3-[3(RS),4(SR)-4-Hydroxy-1-(4-octylphenyl)-1-octen-3-yl-thio]-malonanilinsäure-natriumsalz (Gemisch von cis/trans-Isomeren). In analoger Weise wie im Beispiel 7 erhält man die Titelverbindung aus dem entsprechenden Methylester (Gemisch von cis/trans-Isomeren) (siehe Beispiel 35) in halbfester Form.

Beispiel 37 : 7-[3(RS),4(SR)-4-Hydroxy-1-(4-octylphenyl)-1-octen-3-yl-thio]-4-oxo-4H-chromen-2-carbonsäure-methylester (individuelles cis - und trans -Isomeres)

In analoger Weise wie im Beispiel 19 aber ausgehend von 3(RS),4(RS)-3,4-Epoxy-1-(4-octylphenyl)-1-octen (Gemisch von cis/trans-Isomeren) (siehe Beispiel 35a) und 7-Mercapto-4-oxo-4H-chromen-2-carbonsäure-methylester erhält man die Titelverbindung als Isomerengemisch.

Dieses kann durch Chromatographie am Kieselgel in individuelle Komponenten aufgetrennt werden, wobei durch Eluieren mit einem 7:3-Gemisch (V/V) von Hexan-Ethylacetat zuerst das cis-Isomere, nachher das trans -Isomere erhalten wird.

Beispiel 38 : 7-[3(RS),4(SR)-4-Hydroxy-1-(4-octylphenyl)-1-trans-octen-3-yl-thio]-4-oxo-4H-chromen-2-carbonsäure-natriumsalz

In analoger Weise wie im Beispiel 7 erhält man die Titelverbindung aus dem entsprechenden Methylester (trans-Isomeren, siehe Beispiel 37).

Beispiel 39 : 7-[3(RS),4(SR)-4-Hydroxy-1-(4-octylphenyl)-1-cis-octen-3-yl-thio]-4-oxo-4H-chromen-2-carbonsäure-natriumsalz

In analoger Weise wie im Beispiel 7 erhält man die Titelverbindung aus dem entsprechenden Methylester (cis-Isomeren, siehe Beispiel 37).

Beispiel 40 : 7-[1(RS),2(SR)-4-Chlor-2-hydroxy-1-(2-nonylphenyl)-butylthio]-4-oxo-4H-chromen-2-carbonsäure-methylester

In analoger Weise wie im Beispiel 17 aber ausgehend von 1(RS),2(RS)-4-Chlor-1,2-epoxy-1-(2-nonylphenyl)-butan und 7-Mercapto-4-oxo-4H-chromen-2-carbonsäure-methylester erhält man die Titelverbindung als blassgelbe Kristalle aus Ether-Petrolether, Smp. 69-73°; IR ($CH_2Cl_2$): 3580, 2960, 2930, 2860, 1745, 1655, 1605, 1420 $cm^{-1}$.

Das als Ausgangsstoff verwendete 1(RS),2(RS)-4-Chlor-1,2-epoxy-1-(2-nonylphenyl)-butan kann folgendermassen hergestellt werden:

a) 4-Chlor-1-(2-nonylphenyl)-butanol

In analoger Weise wie im Beispiel 17a aber ausgehend von 4-Chlorbutyraldehyd ergibt 2-Nonylbrombenzol die Titelverbindung als farbloses Oel, IR ($CH_2Cl_2$): 3600, 2960, 2930, 2860, 1470, 1055 $cm^{-1}$.

b) 4-Chlor-1-(2-nonylphenyl)-1-trans-buten

In analoger Weise wie im Beispiel 17b ergibt 4-Chlor-1-(2-nonylphenyl)-butanol (siehe oben) die Titelverbindung als farbloses Oel, IR ($CH_2Cl_2$): 2960, 2930, 2860, 1465, 970 $cm^{-1}$.

c) 1(RS),2(RS)-4-Chlor-1,2-epoxy-1-(2-nonylphenyl)-butan

In analoger Weise wie im Beispiel 17c ergibt 4-Chlor-1-(2-nonylphenyl)-1-trans-buten (siehe oben) die Titelverbindung als farbloses Oel, IR ($CH_2Cl_2$): 2930, 2860, 1465, 1450 $cm^{-1}$.

Beispiel 40A : 7-[1(RS),2(SR)-4-Chlor-2-hydroxy-1-(2-nonylphenyl)-butylthio]-2-methoxy-chinolin-3-carbonsäure-methylester

In analoger Weise wie im Beispiel 40, aber ausgehend von 1(RS),2(RS)-4-Chlor-1,2-epoxy-1-(2-nonylphenyl)-butan und 7-Mercapto-2-methoxychinolin-3-carbonsäure-methylester, erhält man die Titelverbindung; IR ($CH_2Cl_2$): 3580, 2960, 2935, 2860, 1730, 1615, 1485, 1080 $cm^{-1}$.

Beispiel 40B : 7-[1(RS),2(SR)-6-Chlor-2-hydroxy-1-(2-nonylphenyl)-hexylthio]-4-oxo-4H-chromen-2-carbonsäure-methylester

In analoger Weise wie im Beispiel 40, aber ausgehend von 1(RS),2(RS)-6-Chlor-1,2-epoxy-1-(2-nonylphenyl)-hexan und 7-Mercapto-4-oxo-4H-chromen-2-carbonsäure-methylester, erhält man die Titelverbindung; Smp. 60-62°.

Das als Ausgangsmaterial verwendete 1(RS),2(RS)-6-Chlor-1,2-epoxy-1-(2-nonylphenyl)-hexan kann gemäss der im Beispiel 40a-c beschriebenen Methode wie folgt hergestellt werden: 2-Nonylphenylmagnesiumbromid und 6-Chlorhexanol ergeben 1-(2-Nonylphenyl)-6-chlorhexanol, welches zum 1-(2-Nonylphenyl)-6-chlorhexen dehydratisiert wird, und dieses mit 3-Chlorperbenzoesäure zum gewünschten Epoxid umgewandelt wird; IR ($CH_2Cl_2$): 2930, 2860, 1460, 1220, 900 $cm^{-1}$.

Beispiel 40C : 7-[1(RS),2(SR)-6-Chlor-2-hydroxy-1-(2-nonylphenyl)-hexylthio]-2-methoxy-chinolin-3-carbonsäure-methylester

In analoger Weise wie im Beispiel 40, aber ausgehend von 1(RS),2(RS)-6-Chlor-1,2-epoxy-1-(2-nonylphenyl)-hexan und 7-Mercapto-2-methoxychinolin-3-carbonsäure-methylester, erhält man die Titelverbindung; IR ($CH_2Cl_2$): 3580, 2960, 2930, 2860, 1730, 1615, 1485, 1195, 1080 $cm^{-1}$.

Beispiel 40D : 7-[1(RS),2(SR)-6-Fluor-2-hydroxy-1-(2-nonylphenyl)-hexylthio]-4-oxo-4H-chromen-2-carbonsäure-methylester

In analoger Weise wie im Beispiel 40, aber ausgehend von 1(RS),2(RS)-6-Fluor-1,2-epoxy-1-(2-nonylphenyl)-hexan und 7-Mercapto-4-oxo-4H-chromen-2-carbonsäure-methylester, erhält man die Titelverbindung; Smp. 60-62°.

Das als Ausgangsmaterial verwendete 1(RS),2(RS)-1,2-Epoxy-6-fluor-1-(2-nonylphenyl)-hexan kann beispielsweise wie folgt hergestellt werden.

a) 6-(2-Nonylphenyl)-5-trans-hexenol

Eine unter Stickstoffatmosphäre gerührte Lösung von 34,4 g 6-(2-Nonylphenyl)-5-hexensäure-methylester [EP-OL 0 123 543] in 300 ml Tetrahydrofuran wird während 1 Stunde portionsweise mit 2,8 g Lithiumaluminiumhydrid versetzt. Nach weiteren 30 Minuten werden 30 ml Ethylacetat und dann 30 ml Wasser zugetropft. Das Reaktionsgemisch wird mit 1N Salzsäure angesäuert und mehrmals mit Ethylacetat extrahiert. Die vereinigten Extrakte werden über Natriumsulfat getrocknet, eingedampft und mit Dichlormethan an Kieselgel chromatographiert. Man erhält die Titelverbindung als hellgelbes Oel; IR ($CH_2Cl_2$): 3620, 2930, 2850, 1470, 970 $cm^{-1}$.

b) 6-Fluor-1-(2-nonylphenyl)-1-trans-hexen

Zu einem unter Argonatmosphäre gerührten Gemisch von 4,68 g Diethylamino-schwefeltrifluorid in 20 ml Dichlormethan tropft man während 20 Minuten unter Eiskühlung die Lösung von 8,17 g 6-(2-Nonylphenyl)-5-trans-hexenol in 20 ml Dichlormethan. Man rührt 14 Stunden bei Raumtemperatur, versetzt mit Wasser, trennt die organische Schicht ab und wäscht mit gesättigter Natriumbicarbonatlösung und Wasser. Nach Trocknen und Eindampfen wird das Rohprodukt durch Flash-Chromatographie an Kieselgel mit Petrolether gereinigt. Man erhält die Titelverbindung als farbloses Oel; IR ($CH_2Cl_2$) 2930, 2850, 1465, 970 $cm^{-1}$.

c) 1(RS),2(RS)-1,2-Epoxy-6-fluor-1-(2-nonylphenyl)-hexan

Umsetzen von 6-Fluor-1-(2-nonylphenyl)-1-trans-hexen mit 3-Chlorperbenzoesäure wie im Beispiel 17c beschrieben ergibt die Titelverbindung als Farbloses Oel; IR ($CH_2Cl_2$): 2920, 2850, 1455 $cm^{-1}$.

Beispiel 40E : 7-[1(RS),2(SR)-6-Fluor-2-hydroxy-1-(2-nonylphenyl)-hexylthio]-2-methoxy-chinolin-3-carbonsäure-methylester

In analoger Weise wie im Beispiel 40, aber ausgehend von 1(RS),2(RS)-6-Fluor-1,2-epoxy-1-(2-nonylphenyl)-hexan und 7-Mercapto-2-methoxychinolin-3-carbonsäure-methylester, erhält man die Titelverbindung; IR ($CH_2Cl_2$): 3580, 2925, 2860, 1730, 1615, 1485, 1195, 1080 $cm^{-1}$.

Beispiel 41 : 7-[1(RS),2(SR)-4-Chlor-2-hydroxy-1-(2-nonylphenyl)-butylthio]-4-oxo-4H-chromen-2-carbonsäure

In analoger Weise wie im Beispiel 18 erhält man die Titelverbindung aus dem entsprechenden Methylester (siehe Beispiel 40) in Form von blassgelben Kristallen, Smp. 136-138°; IR ($CH_2Cl_2$): 3580, 3430, 2925, 2860, 1735, 1645, 1600, 1420 $cm^{-1}$.

Beispiel 42: 7-[1(RS),2(SR)-4-Chlor-2-hydroxy-1-(2-nonylphenyl)-butylthio]-2-methoxychinolin-3-carbonsäure-natriumsalz

In analoger Weise wie im Beispiel 18F erhält man die Titelverbindung aus dem entsprechenden Methylester (siehe Beispiel 40A); Smp. 187-192°.

Beispiel 43: 7-[1(RS),2(SR)-6-Chlor-2-hydroxy-1-(2-nonylphenyl)-hexylthio]-4-oxo-4H-chromen-2-carbonsäure

In analoger Weise wie im Beispiel 18 erhält man die Titelverbindung aus dem entsprechenden Methylester (siehe Beispiel 40B); Smp. 135-139°.

Beispiel 44: 7-[1(RS),2(SR)-6-Chlor-2-hydroxy-1-(2-nonylphenyl)-hexylthio]-2-methoxychinolin-3-carbonsäure

In analoger Weise wie im Beispiel 18 erhält man die Titelverbindung aus dem entsprechenden Methylester (siehe Beispiel 40C); IR ($CH_2Cl_2$): 3570, 3300, 2960, 2920, 2850, 1740, 1610, 1480, 1240 $cm^{-1}$.

Beispiel 45: 7-[1(RS),2(SR)-6-Fluor-2-hydroxy-1-(2-nonylphenyl)-hexylthio]-4-oxo-4H-chromen-2-carbonsäure

In analoger Weise wie im Beispiel 18 erhält man die Titelverbindung aus dem entsprechenden Methylester (siehe Beispiel 40D); IR ($CH_2Cl_2$): 3580, 2900 (breit), 2960, 2930, 1740, 1660, 1630, 1600, 1420 $cm^{-1}$.

Beispiel 46: 7-[1(RS),2(SR)-6-Fluor-2-hydroxy-1-(2-nonylphenyl)-hexylthio]-2-methoxy-chinolin-3-carbonsäure

In analoger Weise wie im Beispiel 18 erhält man die Titelverbindung aus dem entsprechenden Methylester (siehe Beispiel 40E); IR ($CH_2Cl_2$): 3580, 2960, 2930, 2820, 1745, 1615, 1490, 1390, 1340, 1245 $cm^{-1}$.

Beispiel 47: 7-[1(S),2(R)-1-Hydroxy-1-(m -tolyl)-3-trans-5-cis-pentadecadien-2-yl-thio]-4-oxo-4H-chromen-2-carbonsäure-methylester und dessen 1(R),2(S)-Diastereoisomeres.

In analoger Weise wie im Beispiel 25, aber ausgehend von den diastereomeren reinen Epoxiden erhält man die Titelverbindungen; 1(S),2(R)-Diastereoisomer: $[\alpha]_D^{20}$ = -50.9 ±9° (0,11 % in Chloroform), und 1(R)-,2(S)-Diastereoisomer: $[\alpha]_D^{20}$ = +55.1 ±8.9° (0,1125 % in Methanol).

Das als Ausgangsmaterial verwendete 1(S),2(S)-1,2-Epoxy-1-(m-tolyl)-3-trans-5-cis-pentadecadien und dessen 1(R),2(R)-Diastereoisomeres erhält man analog Beispiel 25c und d, aber unter Verwendung der entsprechenden diastereomerenreinen Aldehyde, d.h. 2(R),3(S)-2,3-Epoxy-3-(m-tolyl)-propanal und dessen 2(S),3(R)-Stereoisomeres. Diese erhält man analog Beispiel 25B (a-d), aber ausgehend von m-Tolualdehyd unter Verwendung von L-(+)-Weinsäure-diethylester bzw. D-(-)-Weinsäurediethylester bei der Epoxidierung.

Beispiel 48: 7-[1(S),2(R)-1-Hydroxy-1-(m -tolyl)-3-trans-5-cis-pentadecadien-2-yl-thio]-4-oxo-4H-chromen-2-carbonsäure und dessen 1(R),2(S)-Diastereoisomeres.

In analoger Weise wie im Beispiel 18 erhält man die Titelverbindungen aus den entsprechenden Methylestern; 1(S),2(R)-Diastereoisomer: $[\alpha]_D^{20}$ = -54.8 ±8.7° (0,115 % in Methanol), sowie 1(R),2(S)-Diastereoisomer: IR ($CH_2Cl_2$): 3340, 2880, 1615, 1590, 1400, 1340 $cm^{-1}$.

Beispiele der pharmazeutischen Zusammensetzungen und entsprechender fertiger Arzneimittelformen.

Unter dem Ausdruck "Wirkstoff" ist nachstehend eine erfindungsgemässe Verbindung der Formel I zu verstehen, insbesondere eine solche, die als Produkt in Beispielen 1-41 beschrieben ist.

Beispiel A:

Eine treibmittelhaltige, feststoffaerosolbildende Inhalationssuspension enthaltend 0,1 Gew.-% Wirkstoff.

| Zusammensetzung: | Gew.-% |
|---|---|
| Wirkstoff, mikronisiert | 0,1 |
| Sorbitantrioleat | 0,5 |
| Treibmittel A | |
| (Trichlortrifluorethan) | 4,4 |
| Treibmittel B | |
| (Dichlordifluormethan und | 15,0 |
| 1,2-Dichlortetrafluorethan) | 80,0 |

Herstellung:

Der Wirkstoff wird unter Feuchtigkeitsausschluss mit Hilfe eines üblichen Homogenisators unter Zusatz des Sorbitantrioleats im Trichlortrifluorethan suspendiert, die Suspension in einen mit Dosierventil versehenen Aerosolbehälter abgefüllt; dieser wird verschlossen und unter Druck mit dem Treibmittel B aufgefüllt.

Beispiel B:

Eine zur Inhalation geeignete, etwa 2%-ige wässrige Lösung eines Wirkstoffes in Form dessen Natrium- oder Kalium-salzes.

| Zusammensetzung | | |
|---|---|---|
| Wirkstoff (K- oder Na-Salz) | | 2000 mg |
| Ethylendiamintetraessigsäure-Dinatriumsalz | | 10 mg |
| Benzalkoniumchlorid | | 10 mg |
| Wasser, frisch destilliert | ad | 100 ml |

Herstellung:

Der Wirkstoff wird in etwa 60 ml frisch destilliertem Wasser gelöst und der Stabilisator (Ethylendiamintetraessigsäure-Dinatriumsalz) und das Konservierungsmittel (Benzalkoniumchlorid) hinzugegeben. Nach vollständiger Auflösung aller Komponenten wird die erhaltene Lösung auf 100 ml aufgefüllt, in Druckfläschchen abgefüllt und diese gasdicht verschlossen. Das Treibmittel wird nach Bedarf gasförmig unter Druck oder in flüssiger Form zugegeben.

**Ansprüche**

1. Eine Verbindung der Formel

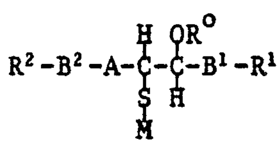

(I)

worin die allgemeinen Symbole die folgenden Bedeutungen haben:

$R^0$ ist Wasserstoff oder $C_{1-7}$-Alkanoyl;

$R^1$ ist $C_{1-3}$-Alkyl, welches durch ein oder mehrere Halogene mit Atomzahl von höchstens 17 substituiert sein kann,

$R^2$ ist ein aliphatischer Rest mit 5-15 C-Atomen,

A ist eine Einfachbindung, Ethylen oder Vinylen;

$B^1$ ist $C_{1-7}$-Alkylen oder Phenylen;

$B^2$ ist eine Einfachbindung, Ethylen oder Phenylen;

M ist ein aromatischer Rest der Teilformel

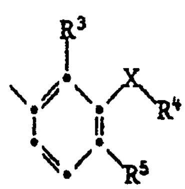

(M)

mit den folgenden Bedeutungen der Symbole:

$R^3$ ist Wasserstoff oder $C_{1-4}$-Alkyl;

X ist NH, O, S oder, falls $R^4$ Wasserstoff ist, die Einfachbindung;

eines der Symbole $R^4$ und $R^5$ bedeutet Wasserstoff und das andere die Gruppe -CO-$R^6$, oder $R^4$ und $R^5$ zusammen bedeuten den Rest -CO-C($R^6$) = C($R^7$)- oder -CO-C($R^7$) = C($R^6$)- oder $R^4$ und $R^5$ zusammen mit X bedeuten den Rest -N = C($R^8$)-C($R^6$ = CH-; wobei
$R^6$ für $-(CH_2)_b-COOR^3$ (worin b = 0 bis 2 ist),
$R^7$ für Wasserstoff oder $C_{1-4}$-Alkyl und
$R^8$ für Wasserstoff, Methyl, Methoxy oder Halogen steht, sowie Salze solcher Verbindungen mit salzbildenden Eigenschaften.

2. Eine Verbindung der Formel I gemäss Anspruch 1, worin die Symbole $R^1$, $R^2$, A, $B^1$ und $B^2$ die im Anspruch 1 angegebene Bedeutung haben, $R^0$ für Wasserstoff steht und M einen Phenylrest, in welchem je $R^3$ und $R^5$ für Wasserstoff, X für die Gruppe -NH- und $R^4$ für den Rest $-CO-CH_2-COOH$ steht, einen Rest der Teilformel

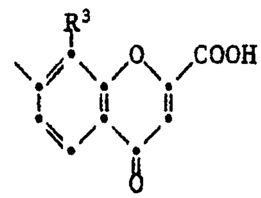

($M^1$),

in welchem $R^3$ für Propyl oder Wasserstoff steht, oder einen Rest der Teilformel

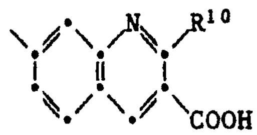

($M^3$)

darstellt, in welchem $R^{10}$ Halogen oder Methoxyl ist, oder ein physiologisch verträgliches Salz davon.

3. Eine Verbindung gemäss Anspruch 2, worin in Formel I die Symbole die folgende Bedeutung haben: $R^0$ ist Wasserstoff; $R^1$ ist Methyl, Chlormethyl oder Trifluormethyl; $R^2$ ist ein lineares Alkyl mit 7-15 C-Atomen oder ein entsprechender Rest mit 1-3 Doppelbindungen; A ist cis - oder trans -Vinylen; $B^1$ ist ein lineares Alkylen mit 2-3 C-Atomen; $B^2$ ist die Einfachbindung und M hat eine der im Anspruch 2 angegebenen Bedeutungen, oder ein Alkalimetallsalz davon.

4. Eine Verbindung gemäss Anspruch 2, worin in Formel I die Symbole die folgende Bedeutung haben: $R^0$ ist Wasserstoff; $R^1$ ist Methyl, Chlormethyl oder Trifluormethyl; $R^2$ ist ein lineares Alkyl mit 8-12 C-Atomen; A ist die Einfachbindung oder Vinylen in cis - oder trans -Konfiguration, $B^1$ ist ein unsubstituiertes Phenylen oder ein lineares Alkylen mit 2-3 C-Atomen, $B^2$ ist ein unsubstituiertes Phenylen und M hat eine der im Anspruch 2 angegebenen Bedeutungen, oder ein Alkalimetallsalz davon.

5. Eine Verbindung gemäss Anspruch 1, worin in Formel I die Symbole die folgende Bedeutung haben: $R^0$ ist Wasserstoff; $R^1$ ist Methyl, Chlormethyl oder Trifluormethyl, $R^2$ ist ein lineares Alkyl mit 7-15 C-Atomen oder ein entsprechender Rest mit 1-3 Doppelbindungen; A ist cis - oder trans -Vinylen; $B^1$ ist ein unsubstituiertes Phenylen; $B^2$ ist die Einfachbindung und M hat eine der in Anspruch 2 angegebenen Bedeutungen, oder ein Alkalimetallsalz davon.

6. Eine Verbindung gemäss Anspruch 2, worin in Formel I die Symbole die folgende Bedeutung haben: $R^0$ ist Wasserstoff; $R^1$ ist Methyl, Chlormethyl oder Trifluormethyl; $R^2$ ist ein lineares $C_{8-12}$-Alkyl; A ist eine trans-Doppelbindung, $B^1$ ist unsubstituiertes m-Phenylen; $B^2$ ist ein unsubstituiertes Phenylen und M hat eine der im Anspruch 2 unter Teilformel $M^1$ angegebenen Bedeutungen, oder ein Alkalimetallsalz davon.

7. Eine Verbindung gemäss einem der Ansprüche 1-6 zur Verwendung als Leukotrien-Antagonist und/oder zur Hemmung von Phospholipasen.

8. Verfahren zur Herstellung von Verbindungen der in Anspruch 1 definierten Formel I, dadurch gekennzeichnet, dass man ein Epoxid der Formel

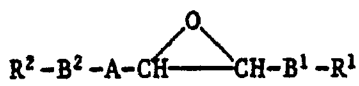

(II)

worin A, $B^1$, $B^2$, $R^1$ und $R^2$ die im Anspruch 1 angegebenen Bedeutungen haben, mit einem Mercapto-Derivat der Formel

$$H\text{-}S\text{-}M \qquad (III)$$

worin M die im Anspruch 1 angegebenen Bedeutungen hat, umsetzt und, wenn notwendig oder erwünscht, eine erhaltene Verbindung der Formel I, worin $R^0$ Wasserstoff bedeutet, zu einer entsprechenden Verbindung, worin $R^0$ ein $C_{1-7}$-Alkanoyl ist, acyliert, und/oder eine als Ester vorliegende Verbindung zur freien Säure oder einem Salz verseift und/oder eine erhaltene freie Verbindung mit salzbildenden Eigenschaften in ein Salz umwandelt und/oder eine Verbindung aus einer entsprechenden Salzform freisetzt.

9. Pharmazeutische Zusammensetzungen enthaltend als Wirkstoff mindestens eine der Verbindungen gemäss einem der Ansprüche 1-7 zusammen mit mindestens einem pharmazeutisch annehmbaren Trägermaterial.

10. Verwendung der Verbindungen gemäss einem der Ansprüche 1-7 zur Herstellung einer pharmazeutischen Zusammensetzung, welche zur Linderung oder Behebung der krankhaften Zustände oder Symptome in einem Säuger, welche auf die allergogene Einwirkung von Leukotrienen oder eine Entzündung zurückzuführen sind, anwendbar ist.

Patentansprüche für folgende Vertragsstaaten : AT, ES, GR

1. Verfahren zur Herstellung einer Verbindung der Formel

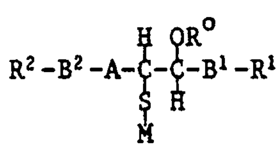

(I)

worin die allgemeinen Symbole die folgenden Bedeutungen haben:

$R^0$ ist Wasserstoff oder $C_{1-7}$-Alkanoyl;
$R^1$ ist $C_{1-3}$-Alkyl, welches durch ein oder mehrere Halogene mit Atomzahl von höchstens 17 substituiert sein kann,
$R^2$ ist ein aliphatischer Rest mit 5-15 C-Atomen,
A ist eine Einfachbindung, Ethylen oder Vinylen;
$B^1$ ist $C_{1-7}$-Alkylen oder Phenylen;
$B^2$ ist eine Einfachbindung, Ethylen oder Phenylen;
M ist ein aromatischer Rest der Teilformel

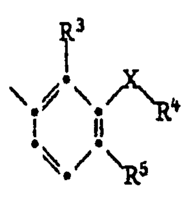

(M)

mit den folgenden Bedeutungen der Symbole:

$R^3$ ist Wasserstoff oder $C_{1-4}$-Alkyl;
X ist NH, O, S oder, falls $R^4$ Wasserstoff ist, die Einfachbindung;
eines der Symbole $R^4$ und $R^5$ bedeutet Wasserstoff und das andere die Gruppe $-CO-R^6$, oder
$R^4$ und $R^5$ zusammen bedeuten den Rest $-CO-C(R^6)=C(R^7)-$ oder $-CO-C(R^7)=C(R^6)-$ oder
$R^4$ und $R^5$ zusammen mit X bedeuten den Rest $-N=C(R^8)-C(R^6)=CH-$; wobei
$R^6$ für $-(CH_2)_b-COOR^3$ (worin b = 0 bis 2 ist),
$R^7$ für Wasserstoff oder $C_{1-4}$-Alkyl und
$R^8$ für Wasserstoff, Methyl, Methoxy oder Halogen steht, sowie von Salzen solcher Verbindungen mit salzbildenden Eigenschaften, dadurch gekennzeichnet, dass man ein Epoxid der Formel

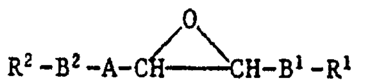
(II)

worin A, $B^1$, $B^2$, $R^1$ und $R^2$ die oben angegebenen Bedeutungen haben, mit einem Mercapto-Derivat der Formel

$$H-S-M \qquad (III)$$

worin M die oben angegebenen Bedeutungen hat, umsetzt und, wenn notwendig oder erwünscht, eine erhaltene Verbindung der Formel I, worin $R^0$ Wasserstoff bedeutet, zu einer entsprechenden Verbindung, worin $R^0$ ein $C_{1-7}$-Alkanoyl ist, acyliert, und/oder eine als Ester vorliegende Verbindung zur freien Säure oder einem Salz verseift und/oder eine erhaltene freie Verbindung mit salzbildenden Eigenschaften in ein Salz umwandelt und/oder eine Verbindung aus einer entsprechenden Salzform freisetzt.

2. Verfahren gemäss Anspruch 1, dadurch gekennzeichnet, dass man eine Verbindung der Formel I herstellt, worin die Symbole $R^1$, $R^2$, A, $B^1$ und $B^2$ die im Anspruch 1 angegebene Bedeutung haben, $R^0$ für Wasserstoff steht und M einen Phenylrest, in welchem je $R^3$ und $R^5$ für Wasserstoff, X für die Gruppe -NH- und $R^4$ für den Rest $-CO-CH_2-COOH$ steht, einen Rest der Teilformel

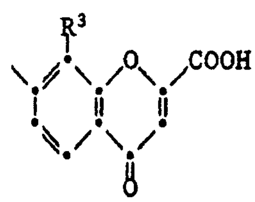
($M^1$),

in welchem $R^3$ für Propyl oder Wasserstoff steht, oder einen Rest der Teilformel

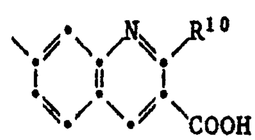
($M^3$)

darstellt, in welchem $R^{10}$ Halogen oder Methoxyl ist, oder ein physiologisch verträgliches Salz davon.

3. Verfahren gemäss Anspruch 2, dadurch gekennzeichnet, dass man eine Verbindung der Formel I, worin die Symbole die folgende Bedeutung haben: $R^0$ ist Wasserstoff; $R^1$ ist Chlormethyl oder Trifluormethyl; $R^2$ ist ein lineares Alkyl mit 7-15 C-Atomen oder ein entsprechender Rest mit 1-3 Doppelbindungen; A ist cis - oder trans-Vinylen; $B^1$ ist ein lineares Alkylen mit 2-3 C-Atomen; $B^2$ ist die Einfachbindung und M hat eine der im Anspruch 2 angegebenen Bedeutungen, oder ein Alkalimetallsalz davon herstellt.

4. Verfahren gemäss Anspruch 2, dadurch gekennzeichnet, dass man eine Verbindung der Formel I, worin die Symbole die folgende Bedeutung haben: $R^0$ ist Wasserstoff; $R^1$ ist Methyl, Chlormethyl oder Trifluormethyl; $R^2$ ist ein lineares Alkyl mit 8-12 C-Atomen; A ist die Einfachbindung oder Vinylen in cis -oder trans -Konfiguration, $B^1$ ist ein unsubstituiertes Phenylen ein lineares Alkylen mit 2-3 C-Atomen, $B^2$ ist ein unsubstituiertes Phenylen und M hat eine der im Anspruch 2 angegebenen Bedeutungen, oder ein Alkalimetallsalz davon herstellt.

5. Verfahren gemäss Anspruch 1, dadurch gekennzeichnet, dass man eine Verbindung der Formel I, worin die Symbole die folgende Bedeutung haben: $R^0$ ist Wasserstoff; $R^1$ ist Methyl, Chlormethyl oder Trifluormethyl, $R^2$ ist ein lineares Alkyl mit 7-15 C-Atomen oder ein entsprechender Rest mit 1-3 Doppelbindungen; A ist cis - oder trans-Vinylen; $B^1$ ist ein unsubstituiertes Phenylen; $B^2$ ist die Einfachbindung und M hat eine der in Anspruch 2 angegebenen Bedeutungen, oder ein Alkalimetallsalz davon herstellt.

6. Verfahren gemäss Anspruch 1, dadurch gekennzeichnet, dass man eine Verbindung der Formel I, worin die Symbole die folgende Bedeutung haben: $R^0$ ist Wasserstoff; $R^1$ ist Methyl, Chlormethyl oder Trifluormethyl; $R^2$ ist ein lineares $C_{8-12}$-Alkyl; A ist eine trans-Doppelbindung, $B^1$ ist unsubstituiertes m-Phenylen; $B^2$ ist unsubstituiertes Phenylen und M hat eine der im Anspruch 12 unter Teilformel $M^1$ angegebenen Bedeutungen, oder ein Alkalimetallsalz davon herstellt.

7. Verfahren gemäss einem der Ansprüche 1-6, dadurch gekennzeichnet, dass man eine Verbindung der Formel I zur Verwendung als Leukotrien-Antagonist und/oder zur Hemmung von Phospholipasen herstellt.

8. Verfahren zur Herstellung einer pharmazeutischen Zusammensetzung enthaltend als Wirkstoff mindestens eine der gemäss einem der Ansprüche 1-7 herstellbaren Verbindungen der Formel I zusammen mit mindestens einem pharmazeutisch annehmbaren Trägermaterial, dadurch gekennzeichnet, dass man die entsprechenden Bestandteile auf nicht-chemischem Wege verarbeitet und, wenn eine fertige Arzneimittelform erwünscht ist, in Einzeldosen aufteilt oder in geeignete Behälter in abgemessenen Mengen abfüllt.

9. Verwendung der gemäss einem der Ansprüche 1-7 herstellbaren Verbindungen der Formel I zur Herstellung einer pharmazeutischen Zusammensetzung.

10. Verwendung der gemäss einem der Ansprüche 1-7 herstellbaren Verbindungen der Formel I zur Herstellung einer pharmazeutischen Zusammensetzung zur Linderung oder Behebung der krankhaften Zustände oder Symptome in einem Säuger, welche auf die allergogene Einwirkung von Leukotrienen oder eine Entzündung zurückzuführen sind.

## Claims

1. A compound of the formula

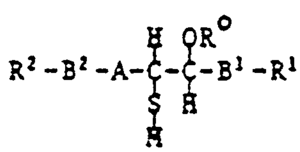

(I)

in which the general symbols have the following meanings:

$R^0$ is hydrogen or $C_{1-7}$-alkanoyl;

$R^1$ is $C_{1-3}$-alkyl which may be substituted by one or more halogen atoms having an atomic number of at most 17;

$R^2$ is an aliphatic radical having from 5 to 15 carbon atoms;

A is a single bond, ethylene or vinylene;

$B^1$ is $C_{1-7}$-alkylene or phenylene;

$B^2$ is a single bond, ethylene or phenylene;

M is an aromatic radical of the partial formula

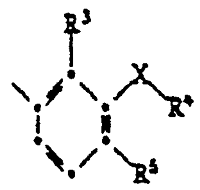

(M)

in which the symbols have the following meanings:
$R^3$ is hydrogen or $C_{1-4}$-alkyl;
X is NH, O, S or, if $R^4$ is hydrogen, a single bond;
one of the symbols $R^4$ and $R^5$ represents hydrogen and the other represents the group $-CO-R^6$, or
$R^4$ and $R^5$ together represent the radical $-CO-C(R^6)=C(R^7)-$ or $-CO-C(R^7)=C(R^6)-$ or
$R^4$ and $R^5$, together with X, represent the radical $-N=C(R^8)-C(R^6)=CH-$, in which
$R^6$ represents $-(CH_2)_b-COOR^3$ (in which b = 0 to 2);
$R^7$ represents hydrogen or $C_{1-4}$-alkyl and
$R^8$ represents hydrogen, methyl, methoxy or halogen, and salts of such compounds having salt-forming properties.

2. A compound of the formula I according to claim 1, in which the symbols $R^1$, $R^2$, A, $B^1$ and $B^2$ have the meanings given in claim 1, $R^0$ represents hydrogen and M represents a phenyl radical in which each of $R^3$ and $R^5$ represents hydrogen, x represents the group -NH- and $R^4$ represents the radical $-CO-CH_2-COOH$, or M represents a radical of the partial formula

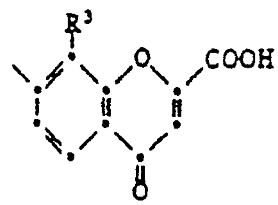

($M^1$)

in which $R^3$ represents propyl or hydrogen, or M represents a radical of the partial formula

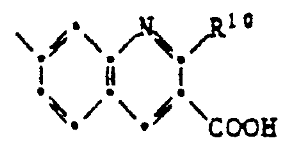

($M^3$)

in which $R^{10}$ is halogen or methoxy, or a physiologically tolerable salt thereof.

3. A compound according to claim 2, in which, in formula I, the symbols have the following meanings: $R^0$ is hydrogen; $R^1$ is methyl, chloromethyl or trifluoromethyl; $R^2$ is a linear alkyl radical having from 7 to 15 carbon atoms or a corresponding radical having from 1 to 3 double bonds; A is cis - or trans - vinylene; $B^1$ is a linear alkylene group having 2 or 3 carbon atoms; $B^2$ is a single bond and M has one of the meanings given in claim 2, or an alkali metal salt thereof.

4. A compound according to claim 2, in which, in formula I, the symbols have the following meanings: $R^0$ is hydrogen; $R^1$ is methyl, chloromethyl or trifluoromethyl; $R^2$ is a linear alkyl radical having from 8 to 12 carbon atoms; A is a single bond or vinylene in the cis -or trans -configuration; $B^1$ is unsubstituted phenylene or a linear alkylene group having 2 or 3 carbon atoms; $B^2$ is unsubstituted phenylene and M has one of the meanings given in claim 2, or an alkali metal salt thereof.

5. A compound according to claim 1, in which, in formula I, the symbols have the following meanings: $R^0$

is hydrogen; $R^1$ is methyl, chloromethyl or trifluoromethyl; $R^2$ is a linear alkyl radical having from 7 to 15 carbon atoms or a corresponding radical having from 1 to 3 double bonds; A is cis - or trans - vinylene; $B^1$ is unsubstituted phenylene; $B^2$ is a single bond and M has one of the meanings given in claim 2, or an alkali metal salt thereof.

6. A compound according to claim 2, in which, in formula I, the symbols have the following meanings: $R^0$ is hydrogen; $R^1$ is methyl, chloromethyl or trifluoromethyl; $R^2$ is a linear $C_{8-12}$-alkyl radical; A is a trans - double bond; $B^1$ is unsubstituted m -phenylene; B2 is unsubstituted phenylene and M has one of the meanings given in claim 2 under partial formula $M^1$, or an alkali metal salt thereof.

7. A compound according to any one of claims 1 to 6 for use as a leucotriene antagonist and/or for the inhibition of phospholipases.

8. Process for the manufacture of compounds of the formula I defined in claim 1, characterised in that an epoxide of the formula

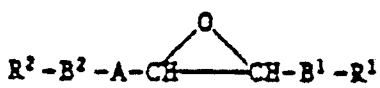

(II)

in which A, $B^1$, $B^2$, $R^1$ and $R^2$ have the meanings given in claim 1 is reacted with a mercapto derivative of the formula

$$H-S-M \qquad (III)$$

in which M has the meanings given in claim 1, and, if necessary or desired, a resulting compound of the formula I in which $R^0$ represents hydrogen is acylated to a corresponding compound in which $R^0$ is $C_{1-7}$-alkanoyl, and/or a compound present in the form of an ester is hydrolysed to the free acid or a salt, and/or a resulting free compound having salt-forming properties is converted into a salt, and/or a compound is freed from a corresponding salt form.

9. Pharmaceutical compositions containing as active ingredient at least one of the compounds according to any one of claims 1 to 7 together with at least one pharmaceutically acceptable carrier.

10. Use of the compounds according to any one of claims 1 to 7 for the manufacture of a pharmaceutical composition that can be used for the alleviation or elimination of pathological conditions or symptoms in a mammal that are attributable to the allergogenic action of leucotrienes or an inflammation.

Claims for the following Contracting States : AT, ES, GR

1. Process for the manufacture of a compound of the formula

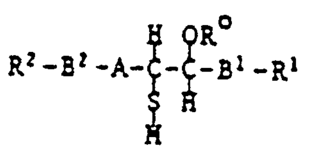

(I)

in which the general symbols have the following meanings:
$R^0$ is hydrogen or $C_{1-7}$-alkanoyl;
$R^1$ is $C_{1-3}$-alkyl which may be substituted by one or more halogen atoms having an atomic number of at most 17;
$R^2$ is an aliphatic radical having from 5 to 15 carbon atoms;
A is a single bond, ethylene or vinylene;

$B^1$ is $C_{1-7}$-alkylene or phenylene;
$B^2$ is a single bond, ethylene or phenylene;
M is an aromatic radical of the partial formula

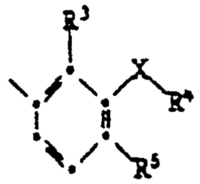

(M)

in which the symbols have the following meanings:
$R^3$ is hydrogen or $C_{1-4}$-alkyl;
X is NH, O, S or, if $R^4$ is hydrogen, a single bond;
one of the symbols $R^4$ and $R^5$ represents hydrogen and the other represents the group -CO-$R^6$, or
$R^4$ and $R^5$ together represent the radical -CO-C($R^6$) = C($R^7$)- or -CO-C($R^7$) = C($R^6$)- or
$R^4$ and $R^5$, together with X, represent the radical -N = C($R^8$)-C($R^6$) = CH-, in which
$R^6$ represents -$(CH_2)_b$-$COOR^3$ (in which b = 0 to 2);
$R^7$ represents hydrogen or $C_{1-4}$-alkyl and
$R^8$ represents hydrogen, methyl, methoxy or halogen, and salts of such compounds having salt-forming properties, characterised in that an epoxide of the formula

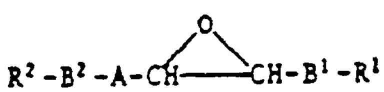

(II)

in which A, $B^1$, $B^2$, $R^1$ and $R^2$ have the meanings given above is reacted with a mercapto derivative of the formula

H-S-M (III)

in which M has the meanings given above, and, if necessary or desired, a resulting compound of the formula I in which $R^0$ represents hydrogen is acylated to a corresponding compound in which $R^0$ is $C_{1-7}$-alkanoyl, and/or a compound present in the form of an ester is hydrolysed to the free acid or a salt, and/or a resulting free compound having salt-forming properties is converted into a salt, and/or a compound is freed from a corresponding salt form.

**2.** Process according to claim 1, characterised in that a compound of the formula I in which the symbols $R^1$, $R^2$, A, $B^1$ and $B^2$ have the meanings given in claim 1, $R^0$ represents hydrogen and M represents a phenyl radical in which each of $R^3$ and $R^5$ represents hydrogen, X represents the group -NH- and $R^4$ represents the radical -CO-$CH_2$-COOH, or M represents a radical of the partial formula

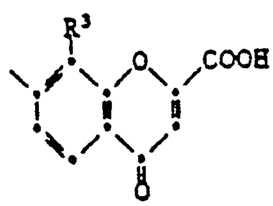

($M^1$)

in which $R^3$ represents propyl or hydrogen, or M represents a radical of the partial formula

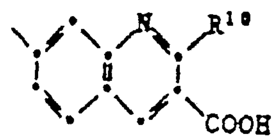

($M^3$)

in which $R^{10}$ is halogen or methoxy, or a physiologically tolerable salt thereof, is manufactured.

3. Process according to claim 2, characterised in that a compound of the formula I in which the symbols have the following meanings: $R^0$ is hydrogen; $R^1$ is chloromethyl or trifluoromethyl; $R^2$ is a linear alkyl radical having from 7 to 15 carbon atoms or a corresponding radical having from 1 to 3 double bonds; A is cis - or trans -vinylene; $B^1$ is a linear alkylene group having 2 or 3 carbon atoms; $B^2$ is a single bond and M has one of the meanings given in claim 2, or an alkali metal salt thereof, is manufactured.

4. Process according to claim 2, characterised in that a compound of the formula I in which the symbols have the following meanings: $R^0$ is hydrogen; $R^1$ is methyl, chloromethyl or trifluoromethyl; $R^2$ is a linear alkyl radical having from 8 to 12 carbon atoms; A is a single bond or vinylene in the cis - or trans -configuration; $B^1$ is unsubstituted phenylene or a linear alkylene group having 2 or 3 carbon atoms; $B^2$ is unsubstituted phenylene and M has one of the meanings given in claim 2, or an alkali metal salt thereof, is manufactured.

5. Process according to claim 1, characterised in that a compound of the formula I in which the symbols have the following meanings: $R^0$ is hydrogen; $R^1$ is methyl, chloromethyl or trifluoromethyl; $R^2$ is a linear alkyl radical having from 7 to 15 carbon atoms or a corresponding radical having from 1 to 3 double bonds; A is cis -or trans -vinylene; $B^1$ is unsubstituted phenylene; $B^2$ is a single bond and M has one of the meanings given in claim 2, or an alkali metal salt thereof, is manufactured.

6. Process according to claim 1, characterised in that a compound of the formula I in which the symbols have the following meanings: $R^0$ is hydrogen; $R^1$ is methyl, chlcromethyl or trifluoromethyl; $R^2$ is a linear $C_{8-12}$-alkyl radical; A is a trans -double bond; $B^1$ is unsubstituted m -phenylene; $B^2$ is unsubstituted phenylene and M has one of the meanings given in claim 12 under partial formula $M^1$, or an alkali metal salt thereof, is manufactured.

7. Process according to any one of claims 1 to 6, characterised in that a compound of the formula I is manufactured for use as a leucotriene antagonist and/or for the inhibition of phospholipases.

8. Process for the manufacture of a pharmaceutical composition containing as active ingredient at least one of the compounds of the formula I, which can be manufactured according to any one of claims 1 to 7, together with at least one pharmaceutically acceptable carrier, characterised in that the appropriate constituents are processed by non-chemical methods and, if a finished medicament form is desired, are divided into individual doses or filled into suitable containers in measured amounts.

9. Use of the compounds of the formula I which can be manufactured according to any one of claims 1 to 7 for the manufacture of a pharmaceutical composition.

10. Use of the compounds of the formula I which can be manufactured according to any one of claims 1 to 7 for the manufacture of a pharmaceutical composition for the alleviation or elimination of pathological conditions or symptoms in a mammal that are attributable to the allergogenic action of leucotrienes or an inflammation.

**Revendications**

1. Un composé de formule

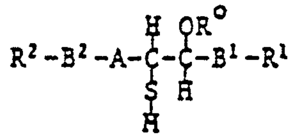

(I)

dans laquelle les symboles généraux ont les significations suivantes :

$R^0$ représente l'hydrogène ou un groupe alcanoyle en C1-C7;

$R^1$ représente un groupe alkyle en C1-C3 qui peut être substitué par un ou plusieurs halogènes de numéro atomique 17 au maximum;

$R^2$ représente un radical aliphatique en C5-C15;

A représente une liaison simple, un groupe éthylène ou vinylène;

$B^1$ représente un groupe alkylène en C1-C7 ou phénylène;

$B^2$ représente une liaison simple, un groupe éthylène ou phénylène;

M représente un radical aromatique répondant à la formule partielle

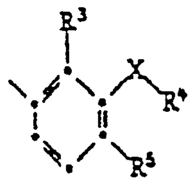

(M)

dans laquelle les symboles ont les significations suivantes :

$R^3$ représente l'hydrogène ou un groupe alkyle en C1-C4;

X représente NH, O, S ou bien encore, lorsque $R^4$ représente l'hydrogène, une liaison simple; l'un des symboles $R^4$ et $R^5$ représente l'hydrogène et l'autre le groupe $-CO-R^6$, ou bien

$R^4$ et $R^5$ forment ensemble le groupe $-CO-C(R^6)=C(R^7)$-ou $-CO-C(R^7)=C(R^6)$- ou bien

$R^4$ et $R^5$ forment avec X le groupe $-N=C(R^8)-C(R^6)=CH$-dans lequel

$R^6$ représente $-(CH_2)_b-COOR^3$ (dans lequel b = 0 à 2),

$R^7$ représente l'hydrogène ou un groupe alkyle en C1-C4 et

$R^8$ représente l'hydrogène, un groupe méthyle, méthoxy ou un halogène, et les sels de ces composés qui sont salifiables.

**2.** Un composé de formule I selon la revendication 1, dans lequel les symboles $R^1$ , $R^2$ , A, $B^1$ et $B^2$ ont les significations indiquées dans la revendication 1, $R^0$ représente l'hydrogène et M un groupe phényle dans lequel $R^3$ et $R^5$ représentent chacun l'hydrogène, X représente le groupe -NH- et $R^4$ le groupe $-CO-CH_2-COOH$, un groupe répondant à la formule partielle

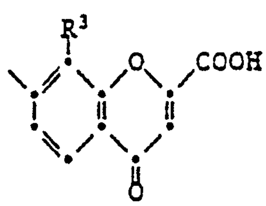

($M^1$),

dans laquelle $R^3$ représente un groupe propyle ou l'hydrogène, ou un groupe répondant à la formule partielle

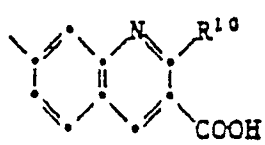

($M^3$)

dans laquelle $R^{10}$ représente un halogène ou un groupe méthoxy, ou un sel d'un tel composé acceptable pour l'usage pharmaceutique.

3. Un composé selon la revendication 2 pour lequel, dans la formule I, les symboles ont les significations suivantes : $R^0$ représente l'hydrogène; $R^1$ représente un groupe méthyle, chlorométhyle ou trifluorométhyle; $R^2$ représente un groupe alkyle linéaire en C7-C15 ou un groupe correspondant contenant une à trois doubles liaisons; A représente un groups cis- ou trans-vinylène; $B^1$ représente un groupe alkylène linéaire en C2-C3; $B^2$ représente une liaison simple et M a l'une des significations indiquées dans la revendication 2, ou un sel de métal alcalin d'un tel composé.

4. Un composé selon la revendication 2, pour lequel, dans la formule I, les symboles ont les significations suivantes : $R^0$ représente l'hydrogène; $R^1$ représente un groupe méthyle, chlorométhyle ou trifluorométhyle; $R^2$ représente un groupe alkyle linéaire en C8-C12; A représente une liaison simple ou un groupe vinylène en configuration cis ou trans, $B^1$ représente un groupe phénylène non substitué ou un groupe alkylène linéaire en C2-C3; $B^2$ représente un groupe phénylène non substitué et M a l'une des significations indiquées dans la revendication 2, ou un sel de métal alcalin d'un tel composé.

5. Un composé selon la revendication 1, pour lequel, dans la formule I, les symboles ont les significations suivantes : $R^0$ représente l'hydrogène; $R^1$ représente un groupe méthyle, chlorométhyle ou trifluorométhyle, $R^2$ représente un groupe alkyle linéaire en C7-C15 ou un groupe correspondant contenant une à trois doubles liaisons; A représente un groupe cis- ou trans-vinylène; $B^1$ représente un groupe phénylène non substitué; $B^2$ représente une liaison simple et M a l'une des significations indiquées dans la revendication 2, ou un sel de métal alcalin d'un tel composé.

6. Un composé selon la revendication 2 pour lequel, dans la formule I, les symboles ont les significations suivantes : $R^0$ représente l'hydrogène; $R^1$ représente un groupe méthyle, chlorométhyle ou trifluorométhyle; $R^2$ représente un groupe alkyle linéaire en C8-C12; A représente une double liaison trans; $B^1$ représente un groupe m-phénylène non substitué; $B^2$ représente une groupe phénylène non substitué et M a l'une des significations indiquées dans la revendication 2 par la formule partielle $M^1$, ou un sel de métal alcalin d'un tel composé.

7. Un composé selon l'une des revendications 1 à 6, pour l'utilisation en tant qu'antagoniste des leucotriènes et/ou pour l'inhibition des phospholipases.

8. Procédé de préparation des composés de formule I définie dans la revendication 1, caractérisé en ce que l'on fait réagir un époxyde de formule

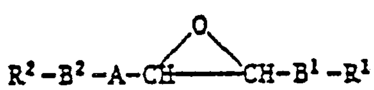

(II)

dans laquelle A, $B^1$, $B^2$, $R^1$ et $R^2$ ont les significations indiquées dans la revendication 1, avec un mercaptan de formule

$$H-S-M \qquad (III)$$

dans laquelle M a les significations indiquées dans la revendication 1 et, lorsque c'est nécessaire ou lorsqu'on le désire, on convertit un composé obtenu, répondant à la formule I dans laquelle $R^0$ représente l'hydrogène, en un composé correspondant pour lequel $R^0$ représente un groupe alcanoyle en C1-C7 par acylation et/ou on saponifie un composé obtenu à l'état d'ester en l'acide libre ou en un sel et/ou on convertit un composé obtenu à l'état libre et salifiable en un sel et/ou on libère un composé à partir d'un sel correspondant.

9. Compositions pharmaceutiques contenant en tant que substance active au moins un des composés selon l'une des revendications 1 à 7 avec au moins un véhicule acceptable pour l'usage pharmaceutique.

**10.** Utilisation des composés selon l'une des revendications 1 à 7 pour la préparation d'une composition pharmaceutique utilisable pour le traitement des états ou symptômes pathologiques d'un mammifère attribués à l'action allergogène des leucotriènes ou à une inflammation.

Revendications pour les Etats contractants suivants : AT, ES, GR

**1.** Procédé de préparation d'un composé de formule

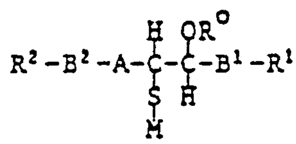

(I)

dans laquelle les symboles généraux ont les significations suivantes :

$R^0$ représente l'hydrogène ou un groupe alcanoyle en C1-C7;
$R^1$ représente un groupe alkyle en C1-C3 qui peut être substitué par un ou plusieurs halogènes de numéro atomique 17 au maximum;
$R^2$ représente un radical aliphatique en C5-C15;
A représente une liaison simple, un groupe éthylène ou vinylène;
$B^1$ représente un groupe alkylène en C1-C7 ou phénylène;
$B^2$ représente une liaison simple, un groupe éthylène ou phénylène;
M représente un radical aromatique répondant à la formule partielle

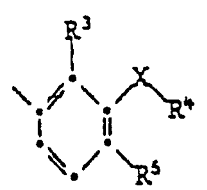

(M)

dans laquelle les symboles ont les significations suivantes :
$R^3$ représente l'hydrogène ou un groupe alkyle en C1-C4;
X représente NH, O, S ou bien encore, lorsque $R^4$ représente l'hydrogène, une liaison simple; l'un des symboles $R^4$ et $R^5$ représente l'hydrogène et l'autre le groupe $-CO-R^6$, ou bien
$R^4$ et $R^5$ forment ensemble un groupe $-CO-C(R^6)=C(R^7)-$ ou $-CO-C(R^7)=C(R^6)-$ ou bien
$R^4$ et $R^5$ forment avec X un groupe $-N=C(R^8)-C(R^6)=CH-$dans lequel
$R^6$ représente $-(CH_2)_bCOOR^3$ (dans lequel b = 0 à 2),
$R^7$ représente l'hydrogène ou un groupe alkyle en C1-C4 et
$R^8$ représente l'hydrogène, un groupe méthyle, méthoxy ou un halogène, et des sels de ces composés qui sont salifiables, caractérisé en ce que l'on fait réagir un époxyde de formule

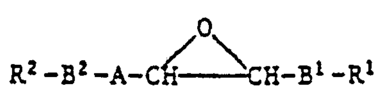

(II)

dans laquelle A, $B^1$, $B^2$, $R^1$ et $R^2$ ont les significations indiquées ci-dessus,
avec un mercaptan de formule

$$H-S-M \qquad (III)$$

dans laquelle M a les significations indiquées ci-dessus, et lorsque c'est nécessaire ou lorsqu'on le désire, on convertit un composé obtenu, répondant à la formule I dans laquelle $R^0$ représente l'hydrogène, en un composé correspondant pour lequel $R^0$ représente un groupe alcanoyle en C1-C7 par acylation, et/ou on saponifie un composé obtenu à l'état d'ester en acide libre ou en un sel

et/ou on convertit un composé obtenu à l'état libre et salifiable en un sel et/ou on libère un composé à partir d'un sel correspondant.

2. Procédé selon la reveneication 1, caractérisé en ce que l'on prépare un composé de formule I dans laquelle les symboles $R^1$, $R^2$, A, $B^1$ et $B^2$ ont les significations indiquées dans la revendication 1, $R^0$ représente l'hydrogène et M un groupe phényle dans lequel $R^3$ et $R^5$ représentent chacun l'hydrogène, X représente le groupe -NH- et $R^4$ représente le groupe $-CO-CH_2-COOH$, un groupe répondant à la formule partielle

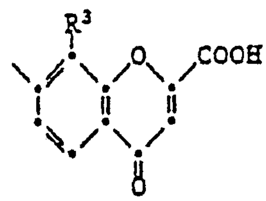

($M^1$),

dans laquelle $R^3$ représente un groupe propyle ou l'hydrogène, ou un groupe répondant à la formule partielle

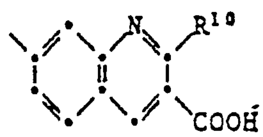

($M^3$)

dans laquelle $R^{10}$ représente un halogène ou un groupe méthoxy, ou un sel d'un tel composé acceptable pour l'usage pharmaceutique.

3. Procédé selon la revendication 2, caractérisé en ce que l'on prépare un composé de formule I dans laquelle les symboles ont les significations suivantes : $R^0$ représente l'hydrogène; $R^1$ représente un groupe chlorométhyle ou trifluorométhyle; $R^2$ représente un groupe alkyle linéaire en C7-C15 ou un groupe correspondant contenant une à trois doubles liaisons; A représente un groupe cis- ou trans-vinylène; $B^1$ représente un groupe alkylène linéaire en C2-C3; $B^2$ représente une liaison simple et M a l'une des significations indiquées dans la revendication 2, ou un sel de métal alcalin d'un tel composé.

4. Procédé selon la revendication 2, caractérisé en ce que l'on prépare un composé de formule I dans laquelle les symboles ont les significations suivantes : $R^0$ représente l'hydrogène; $R^1$ représente un groupe méthyle, chlorométhyle ou trifluorométhyle; $R^2$ représente un groupe alkyle linéaire en C8-C12; A représente une liaison simple ou un groupe vinylène en configuration cis ou trans; $B^1$ représente un groupe phénylène non substitué, un groupe alkylène linéaire en C2-C3, $B^2$ représente un groupe phénylène non substitué et M a l'une des significations indiquées dans la revendication 2, ou un sel de métal alcalin d'un tel composé.

5. Procédé selon la revendication 1, caractérisé en ce que l'on prépare un composé de formule I dans laquelle les symboles ont les significations suivantes : $R^0$ représente l'hydrogène; $R^1$ représente un groupe méthyle, chlorométhyle ou trifluorométhyle, $R^2$ représente un groupe alkyle linéaire en C7-C15 ou un groupe correspondant contenant une à trois doubles liaisons; A représente un groupe cis- ou trans-vinylène; $B^1$ représente un groupe phénylène non substitué; $B^2$ représente une liaison simple et M a l'une des significations indiquées dans la revendication 2, ou un sel de métal alcalin d'un tel composé.

6. Procédé selon la revendication 1, caractérisé en ce que l'on prépare un composé de formule I dans laquelle les symboles ont les significations suivantes : $R^0$ représente l'hydrogène; $R^1$ représente un groupe méthyle, chlorométhyle ou trifluorométhyle; $R^2$ représente un groupe alkyle linéaire en C8-C12; A représente une double liaison trans, $B^1$ représente un groupe m-phénylène non substitué; $B^2$ représente un groupe phénylène non substitué et M a l'une des significations indiquées dans la revendication 12 par la formule partielle $M^1$, ou un sel de métal alcalin d'un tel composé.

7. Procédé selon l'une des revendications 1 à 6, caractérisé en ce que l'on prépare un composé de formule I pour l'utiliser en tant qu'antagoniste des leucotriènes et/ou pour inhiber les phospholipases.

8. Procédé de préparation d'une composition pharmaceutique contenant en tant que substance active au moins un des composés de formule I préparé selon une des revendications 1 à 7 avec au moins un véhicule acceptable pour l'usage pharmaceutique, caractérisé en ce que l'on combine les constituants correspondants par voie non chimique et, lorsqu'on désire une forme d'administration médicamenteuse finie, on partage en doses individuelles ou on introduit en quantités dosées dans des récipients appropriés.

9. Utilisation des composés de formule I préparés selon l'une des revendications 1 à 7 pour la préparation d'une composition pharmaceutique.

10. Utilisation des composés de formule I préparés selon l'une des revendications 1 à 7 pour la préparation d'une composition pharmaceutique prévue pour le traitement des états ou symptômes pathologiques d'un mammifère attribués à l'action allergogène des leucotriènes ou à une inflammation.